# EUROPEAN PATENT APPLICATION

(11) **EP 4 491 729 A1**
(43) Date of publication of application: **15.01.2025**
(21) Application number: 22930986.9
(22) Date of filing: 28.09.2022
(51) Int. Cl.: C12N 15/13, A61K 39/395, A61P 35/00, C07K 16/30, C07K 16/46, C07K 19/00, C12N 5/078, C12N 5/10, C12N 15/12, C12N 15/62, C12N 15/63

(54) **HUMANIZED ANTIBODY THAT BONDS TO EVA1 PROTEIN OR FUNCTIONAL FRAGMENT THEREOF, ANTIBODY-DRUG CONJUGATE AND CHIMERIC ANTIGEN RECEPTOR**

(30) Priority: 09.03.2022 JP 2022036463
(71) Applicant: CURED INC., Yokohama-shi, Kanagawa, 231-0023 (JP); National University Corporation Hokkaido University, Hokkaido 060-0808 (JP); National University Corporation Tokai National Higher Education and Research System, Nagoya-shi, Aichi 464-8601 (JP)
(72) Inventor: MICHISHITA Masahiro, Yokohama-shi, Kanagawa 231-0023 (JP); KONDO Toru, Sapporo-shi, Hokkaido 060-0808 (JP); TERAKURA Seitaro, Nagoya-shi, Aichi 464-8601 (JP); OSAKI Masahide, Nagoya-shi, Aichi 464-8601 (JP); KIYOI Hitoshi, Nagoya-shi, Aichi 464-8601 (JP)
(74) Representative: J A Kemp LLP
(86) International application number: PCT/JP2022/036260
(87) International publication number: WO 2023/171009

(57) **Abstract**

A humanized antibody or a functional fragment thereof binding to a human-derived Eva1 protein, an antibody-drug conjugate comprising the humanized antibody or a functional fragment thereof, or a chimeric antigen receptor comprising an antibody or a functional fragment thereof binding to a human-derived Eva1 protein.

## Description

### [Technical Field]

The present invention relates to a humanized antibody or a functional fragment thereof which binds to a human-derived Eval protein (human Eval). The present invention also relates to an antibody-drug conjugate comprising the humanized antibody or a functional fragment thereof. Moreover, the present invention relates to a chimeric antigen receptor comprising an antibody or a functional fragment thereof which binds to human Eval.

### [Background Art]

Cancers are one of the major death causes in developed countries along with coronary artery diseases, and the proportions of cancers are also steadily increasing year after year. Hence, the urgent development of eradication therapies of cancers has been desired strongly. In the development of curative treatments of cancers, the presence of cancer stem cells has been emphasized recently. Cancer stem cells are present only in small portions of cancer tissues, but repeat self-renewal and further differentiate, and thus are considered to be the source of generation of the majority of cancer cells. In addition, while cancer stem cells have such a high tumorigenic potential, it has been suggested that cancer stem cells have high resistance to chemotherapy and radiotherapy. Hence, even if chemotherapy or radiotherapy can kill most of cancer cells in cancer tissues, cancer stem cells survive, so that relapse, metastasis, and the like of the cancer occur. Accordingly, it is expected that if cancer stem cells can be targeted and also killed, it could lead to the development of a therapeutic method which is also useful for preventing the metastasis, relapse, and the like of the cancers.

In view of such circumstances, the present inventors have successfully identified an Eval protein as a protein expressed at a high level in gliomas and its cancer stem cells. Moreover, the present inventors have revealed that the expression of this Eval protein correlates with the malignancy of gliomas, and that there is a strong correlation between the survival rate of glioma patients and the expression of the Eval gene in gliomas derived from the patients. In addition, the present inventors have also found that suppressing the function of the Eval gene is effective in suppressing the growth potential, tumorigenic potential and tissue infiltration potential of glioma cells as well as the tumor mass-forming potential of glioma stem cells (PTL 1).

In addition, among cancers, the treatment of the gliomas which is based on a surgery with adjuvant therapy of radiotherapy and chemotherapy has not changed much for these several decades. Particularly, no effective therapeutic method has been found yet for glioblastoma multiforme (GBM) which is most malignant among malignant gliomas of central nervous system tissues. Moreover, although temozolomide has been used as a standard therapeutic drug against malignant gliomas, the inventors have confirmed that glioma stem cells are not susceptible to temozolomide even in an amount several times as large as its effective blood concentration. In addition, it is often the case that cancer cells exposed to such a chemotherapeutic agent acquire resistance to the agent, which are not limited to gliomas, and similarly acquire cross resistance to other multiple chemotherapeutic agents. Moreover, chemotherapeutic agents also often cause cytotoxicity to normal cells. To reduce such side effects, the dose or administration of chemotherapeutic agents is limited in many cases.

Under such current circumstances, the use of antibodies as anticancer agents has recently drawn attention, and their importance has been increasingly recognized. For example, in the case of an antibody targeting a cancer-specific antigen, the administered antibody is assumed to be accumulated in the cancer tissues. Hence, the attack on cancer cells through immune systems with an antibody-dependent cellular cytotoxicity (ADCC) activity and a complement-dependent cytotoxicity (CDC) activity can be expected.

In addition, by binding a drug such as a cytotoxic substance or a radionuclide to an antibody (taking a form of a so-called antibody-drug conjugate (ADC)), it becomes possible to efficiently deliver the bound drug to a tumor site. This can reduce the amount of the drug reaching other tissues, and consequently a reduction in side effects can be expected.

In addition, by administering an antibody having an agonistic activity in the case where a cancer-specific antigen has an activity of inducing cell death, or by administering an antibody having a neutralizing activity in the case where a cancer-specific antigen is involved in cell growth and survival, growth termination or shrinkage of a cancer can be expected from the accumulation of a tumor-specific antibody and the activity of the antibody.

Moreover, it is also expected that by administering T-cells expressing a chimeric antigen receptor (CAR) in which variable regions of an antibody are incorporated (CAR-T), the cells are HLA-independently activated to target antigen-positive cancer cells to induce cell death.

From such properties, antibodies are considered to be favorable for application as anticancer agents, and the present inventors also prepared an anti-human Eval mouse antibody having high affinity aiming at creating an antibody preparation targeting the above-mentioned Eva1, and demonstrated its efficacy (PTL 2).

### [Citation List]

### [Patent Literatures]

[PTL 1] International Publication No. WO2012/043747
[PTL 2] International Publication No. WO2017/022668

### [Summary of Invention]

### [Technical Problem]

An object of the present invention is to provide an antibody or a functional fragment thereof which is useful in treatment and the like of cancer, or a conjugate comprising the antibody or a functional fragment thereof.

### [Solution to Problem]

The present inventors attempted to humanize an anti-human Eva1 mouse antibody (the B2E5-48 mouse antibody described in PTL 2) and prepared 16 types of humanized antibodies by combining four types of humanized heavy chain variable regions (H1 to H4) and four types of humanized light chain variable regions (L1 to L4). As a result, the present inventors succeeded in obtaining humanized antibodies (combinations of H2-L2, H2-L4, H3-L4, and the like) which have high binding affinity to the Eva1 protein and also have high production capacity (antibody titer).

In addition, the present inventors prepared a chimeric antigen receptor (CAR) in which the variable regions of the anti-human Eva1 antibody were incorporated, and evaluated its efficacy. As a result, T-cells (CAR-T) which had expressed the CAR exhibited activation (cytokine production) and cell growth in accordance with human Eva1. Moreover, it was revealed that the T-cells exhibited cytotoxic activity against Eva1 protein expressing tumor cells.

In addition, CARs in which variable regions of B2E5-48 mouse antibody were incorporated were evaluated while the spacer length between the antigen-binding region containing the variable regions and the transmembrane region was changed. As a result, it was revealed that in the case where T-cells expressing the CARs were cultured in the absence of IL-2, CAR-T in which the spacer length was 232 amino acids was most excellent in cell growth and cytokine production. On the other hand, in the case where T-cells were cultured in the presence of IL-2, the cell growth of CAR-T having a spacer composed of 15 amino acids was most excellent.

Furthermore, it was also found that in CAR in which the variable regions of the above humanized antibody were incorporated, the combination of L2-H2 was most useful from the viewpoint of activation, cell growth, and the like in accordance with human Eva1. Moreover, regarding CAR-T having an antigen recognition site of L2-H2, various intracellular domains were tested, and differences in reactivity due to these differences were evaluated. As a result, it was revealed that CARs having intracellular domains of 4-1BB and CD79A/CD40 were excellent in cell growth. In addition, the above-described CAR-T (L2-H2 hEva1-CAR-T) was administered to cancer-bearing models, and its antitumor activity and safety were checked.

In addition, an antibody-drug conjugate (ADC) containing the above humanized antibody was prepared, and its efficacy was evaluated. As a result, the antitumor activity of the ADC against various tumors such as glioma, pancreatic cancer, and stomach cancer was observed. On the other hand, no adverse effect (decrease in body weight, shortening of lifetime, or the like) on the living organisms to which the ADC was administered was observed.

The present invention is based on the above study results, and more specifically provides the following aspects.
<1> A humanized antibody or a functional fragment thereof, the humanized antibody comprising:
   a light chain variable region comprising complementarity-determining regions 1 to 3 each comprising an amino acid sequence set forth in SEQ ID NO: 1, an amino acid sequence composed of Val-Thr-Ser, and an amino acid sequence set forth in SEQ ID NO: 3; and
   a heavy chain variable region comprising complementarity-determining regions 1 to 3 each comprising amino acid sequences set forth in SEQ ID NOs: 4 to 6, wherein
   the humanized antibody or a functional fragment thereof binds to a human-derived Eval protein.
<2> The humanized antibody or a functional fragment thereof according to <1>, wherein
   the light chain variable region comprises one amino acid sequence selected from amino acid sequences set forth in SEQ ID NOs: 7 to 10, amino acid sequences having homologies of 80% or more with the amino acid sequences set forth in SEQ ID NOs: 7 to 10, and an amino acid sequence derived from at least one of the amino acid sequences set forth in SEQ ID NOs: 7 to 10 by substitution, deletion, addition, and/or insertion of one or more amino acids, and
   the heavy chain variable region comprises one amino acid sequence selected from amino acid sequences set forth in SEQ ID NOs: 13 to 16, amino acid sequences having homologies of 80% or more with the amino acid sequences set forth in SEQ ID NOs: 13 to 16, and an amino acid sequence derived from at least one of the amino acid sequences set forth in SEQ ID NOs: 13 to 16 by substitution, deletion, addition, and/or insertion of one or more amino acids.
<3> The humanized antibody or a functional fragment thereof according to <1>, wherein
   the light chain variable region comprises one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
   the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids.
<4> An antibody-drug conjugate obtained by binding the antibody or a functional fragment thereof according to any one of <1> to <3> and a drug.
<5> A pharmaceutical composition comprising the antibody-drug conjugate according to <4>.
<6> A chimeric antigen receptor comprising a region binding to a human-derived Eval protein, a transmembrane region, and an intracellular signal region.
<7> The chimeric antigen receptor according to <6>, wherein
   the region binding to the human-derived Eval protein is a region comprising the antibody or a functional fragment thereof according to any one of <1> to <3>.
<8> The chimeric antigen receptor according to <6>, wherein
   the region binding to a human-derived Eval protein comprises a single-chain variable fragment obtained by binding a light chain variable region, a linker, and a heavy chain variable region in order from an amino terminus side,
   the light chain variable region comprises:
      complementarity-determining regions 1 to 3 each comprising an amino acid sequence set forth in SEQ ID NO: 1, an amino acid sequence composed of Val-Thr-Ser, and an amino acid sequence set forth in SEQ ID NO: 3; and
      one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
      the heavy chain variable region comprises:
         complementarity-determining regions 1 to 3 each comprising amino acid sequences set forth in SEQ ID NOs: 4 to 6; and
         one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids.
<9> The chimeric antigen receptor according to any one of <6> to <8>, wherein the region binding to a human-derived Eval protein and the transmembrane region bind to each other via a spacer composed of 10 to 300 amino acids.
<10> The chimeric antigen receptor according to any one of <6> to <9>, wherein the intracellular signal region comprises a CD79A intracellular region and a CD40 intracellular region.
<11> A nucleotide encoding the chimeric antigen receptor according to any one of <6> to <10>.
<12> A vector comprising the nucleotide according to <11>.
<13> A cell expressing the chimeric antigen receptor according to any one of <6> to <10>.
<14> The cell according to <13>, that is an immune cell.
<15> A pharmaceutical composition comprising the cell according to <13> or <14>.

Note that the sequence set forth in each SEQ ID NO is as follows.

**[Table 1]**

| **SEQ ID NO:** | **Name of sequence** |
|---|---|
| **1** | **CDR 1 of Light Chain (Humanized B2E5-48)** |
| **2** | **CDR 2 of Light Chain (Humanized B2E5-48)** |
| **3** | **CDR 3 of Light Chain (Humanized B2E5-48)** |
| **4** | **CDR 1 of Heavy Chain (Humanized B2E5-48)** |
| **5** | **CDR 2 of Heavy Chain (Humanized B2E5-48)** |
| **6** | **CDR 3 of Heavy Chain (Humanized B2E5-48)** |
| **7** | **Variable Region of Light Chain 2 (Humanized B2E5-48)** |
| **8** | **Variable Region of Light Chain 4 (Humanized B2E5-48)** |
| **9** | **Variable Region of Light Chain 1 (Humanized B2E5-48)** |
| **10** | **Variable Region of Light Chain 3 (Humanized B2E5-48)** |
| **11** | **Variable Region of Light Chain 2 (Humanized B2E5-48) cDNA** |
| **12** | **Variable Region of Light Chain 4 (Humanized B2E5-48) cDNA** |
| **13** | **Variable Region of Heavy Chain 2 (Humanized B2E5-48)** |
| **14** | **Variable Region of Heavy Chain 4 (Humanized B2E5-48)** |
| **15** | **Variable Region of Heavy Chain 1 (Humanized B2E5-48)** |
| **16** | **Variable Region of Heavy Chain 3 (Humanized B2E5-48)** |
| **17** | **Variable Region of Heavy Chain 2 (Humanized B2E5-48) cDNA** |
| **18** | **Variable Region of Heavy Chain 3 (Humanized B2E5-48) cDNA** |

**[Table 2]**

| **SEQ ID NO:** | **Name of sequence** |
|---|---|
| **19** | **Signal peptide and Variable Region of Light Chain (B2E5-48) cDNA** |
| **20** | **Signal peptide and Variable Region of Light Chain (B2E5-48)** |
| **21** | **Variable Region of Light Chain (B2E5-48)** |
| **22** | **CDR1 of Light Chain (B2E5-48)** |
| **23** | **CDR2 of Light Chain (B2E5-48)** |
| **24** | **CDR3 of Light Chain (B2E5-48)** |
| **25** | **Signal peptide and Variable Region of Heavy Chain (B2E5-48) cDNA** |
| **26** | **Signal peptide and Variable Region of Heavy Chain (B2E5-48)** |
| **27** | **Variable Region of Heavy Chain (B2E5-48)** |
| **28** | **CDR1 of Heavy Chain (B2E5-48)** |
| **29** | **CDR2 of Heavy Chain (B2E5-48)** |
| **30** | **CDR3 of Heavy Chain (B2E5-48)** |
| **31** | **Signal peptide and Variable Region of Light Chain (C3) cDNA** |
| **32** | **Signal peptide and Variable Region of Light Chain (C3)** |
| **33** | **Variable Region of Light Chain (C3)** |
| **34** | **CDR1 of Light Chain (C3)** |
| **35** | **CDR2 of Light Chain (C3)** |
| **36** | **CDR3 of Light Chain (C3)** |
| **37** | **Signal peptide and Variable Region of Heavy Chain (C3) cDNA** |
| **38** | **Signal peptide and Variable Region of Heavy Chain (C3)** |
| **39** | **Variable Region of Heavy Chain (C3)** |
| **40** | **CDR1 of Heavy Chain (C3)** |
| **41** | **CDR2 of Heavy Chain (C3)** |
| **42** | **CDR3 of Heavy Chain (C3)** |
| **43** | **Variable Region of Light Chain (A5D11-10) cDNA** |
| **44** | **Variable Region of Light Chain (A5D11-10)** |
| **45** | **CDR1 of Light Chain (A5D11-10)** |
| **46** | **CDR2 of Light Chain (A5D11-10)** |
| **47** | **CDR3 of Light Chain (A5D11-10)** |
| **48** | **Variable Region of Heavy Chain (A5D11-10) cDNA** |
| **49** | **Variable Region of Heavy Chain (A5D11-10)** |
| **50** | **CDR1 of Heavy Chain (A5D11-10)** |
| **51** | **CDR2 of Heavy Chain (A5D11-10)** |
| **52** | **CDR3 of Heavy Chain (A5D11-10)** |
| **53** | **hEva1 cDNA** |
| **54** | **hEva1** |
| **55** | **mEva1 cDNA** |
| **56** | **mEva1** |

**[Table 3]**

| **SEQ ID NO:** | **Name of sequence** |
|---|---|
| **57** | **B2E5-48 CAR cDNA** |
| **5B** | **B2E5-48 CAR** |
| **59** | **Hinge region: 15aa** |
| **60** | **Hinge region: 30aa** |
| **61** | **Hinge region: 60aa** |
| **62** | **Hinge region: 125aa** |
| **63** | **Hinge region: 232aa** |
| **64** | **4-1BB IC** |
| **65** | **CD79A IC** |
| **66** | **CD40 IC** |

Note that as shown in Table 1, SEQ ID NO: 2 is intended for an amino acid sequence of CDR2 of Light Chain (Humanized B2E5-48) (an amino acid sequence composed of valine-threonine-serine in order from N-terminus) (see also Table 7). However, since this sequence is a sequence having less than 4 (3) amino acids, this sequence is handled as an "intentionally skipped sequence" in Sequence Listing.

### [Advantageous Effects of Invention]

The present invention makes it possible to treat or prevent cancers and the like by targeting a human Eval.

### [Brief Description of Drawings]

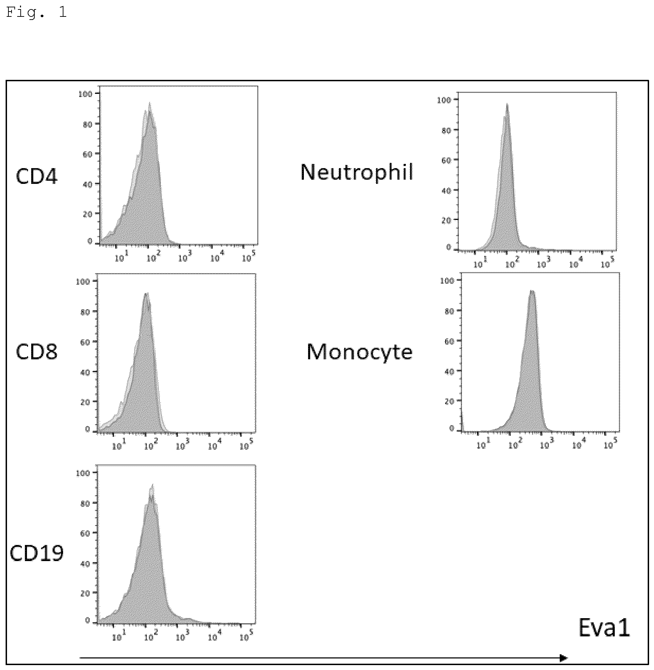

[Fig. 1] Fig. 1 is histograms showing results of analyzing expression of a human Eval in normal human blood cells.

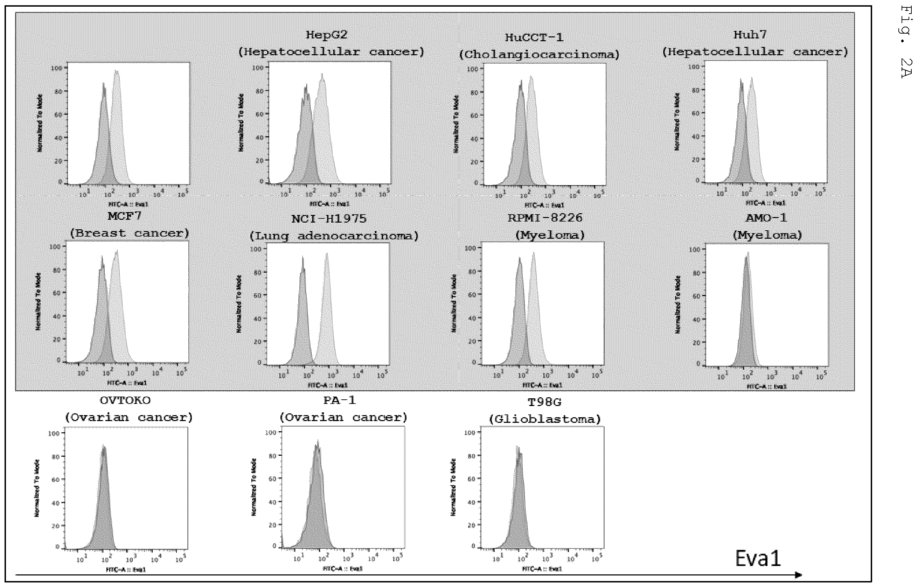

[Fig. 2A] Fig. 2A is histograms showing results of analyzing expression of the human Eval in various types of tumor cells.

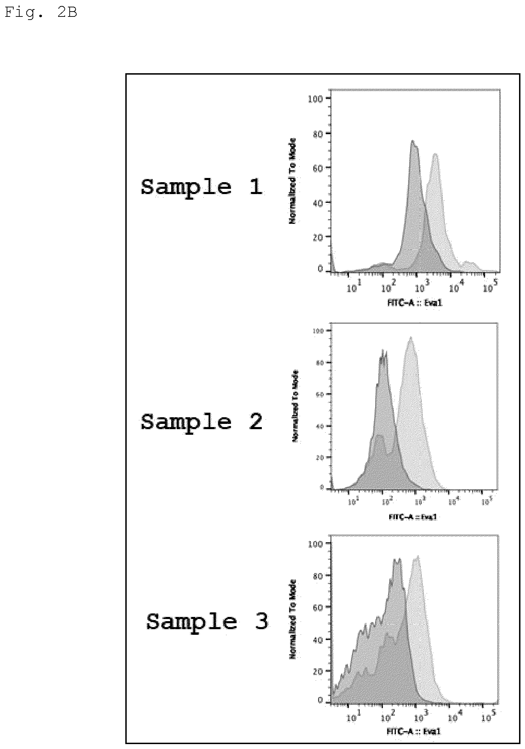

[Fig. 2B] Fig. 2B is histograms showing results of analyzing expression of the human Eval in intrahepatic cholangiocarcinoma cases (Samples 1 to 3).

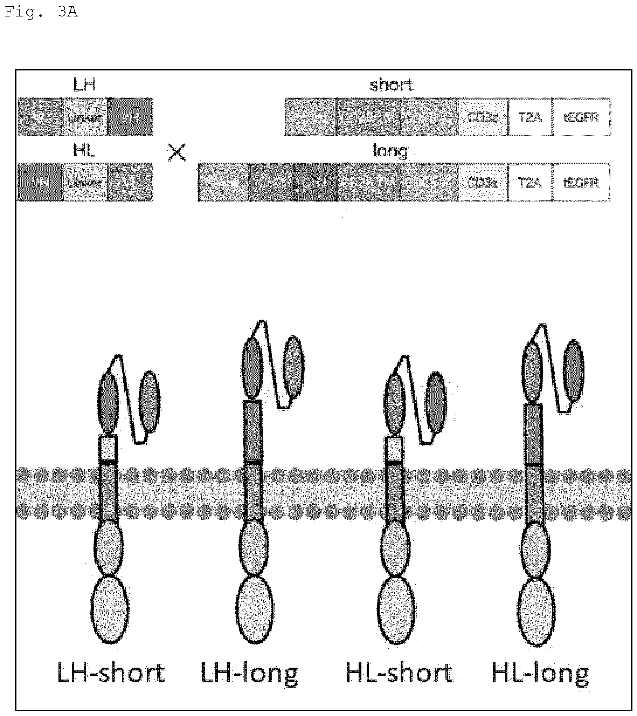

[Fig. 3A] Fig. 3A is a showing an overview of a chimeric antigen receptor (Eva1-CAR) using an anti-Eval antibody sequence.

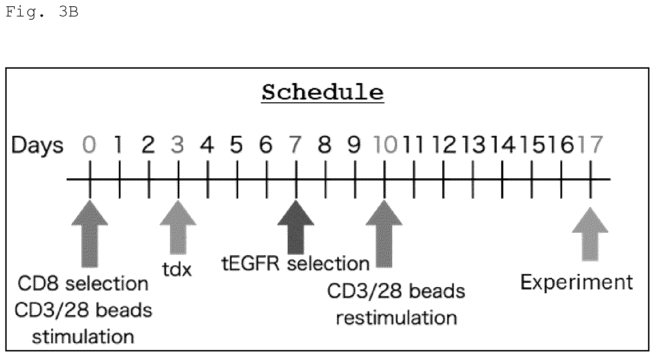

[Fig. 3B] Fig. 3B is a diagram showing an overview of a schedule of preparing Eval-CAR expressing T-cells (Eval-CAR-T) .

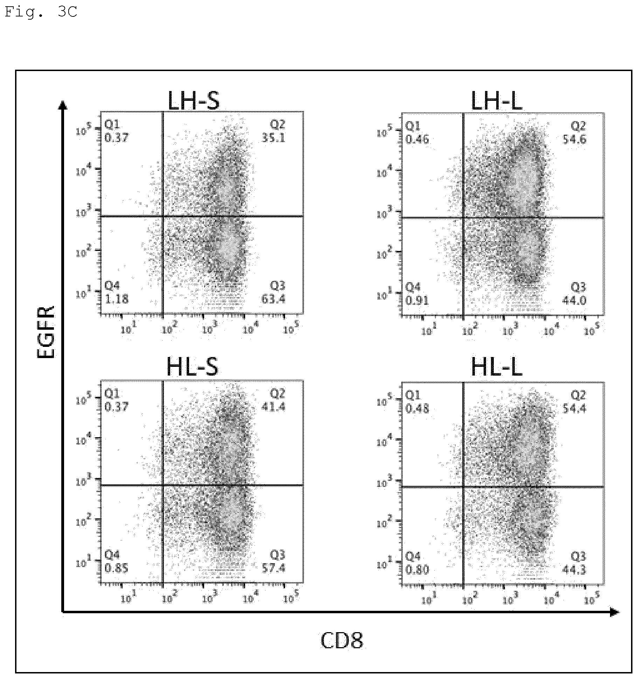

[Fig. 3C] Fig. 3C is dot-plot diagram showing results of analyzing introduction efficiency of the Eval-CAR gene before selection using expression of tEGFR as an index (tEGFR selection).

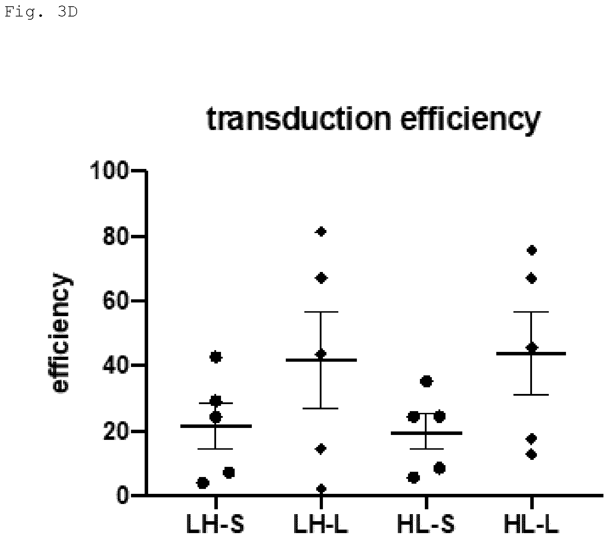

[Fig. 3D] Fig. 3D is dot-plot diagram showing results of analyzing the introduction efficiency of the Eval-CAR gene before the tEGFR selection. In the figure, dots indicate origins (5 healthy subjects) of cells in which the Eval-CAR gene was introduced. Thick lines indicate average values, and whiskers indicate standard error.

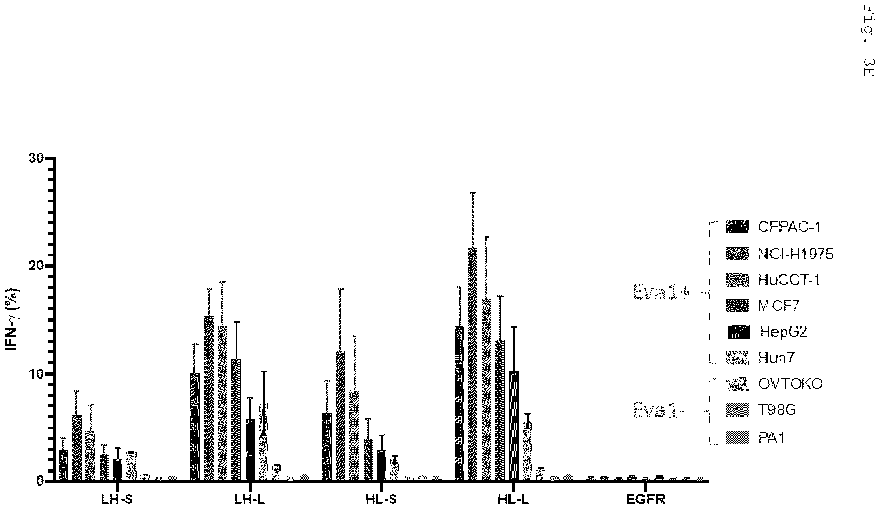

[Fig. 3E] Fig. 3E is a graph showing results of analyzing cytokine (IFN-γ) production of the Eval-CAR-T against various types of tumor cells. In the figure, on the horizontal axis, results on CFPAC-1, NCI-H1975, HuCCT-1, MCF7, HepG2, Huh7, OVTOKO, T98G, and PA1 are shown in order from the left for each introduced Eval-CAR.

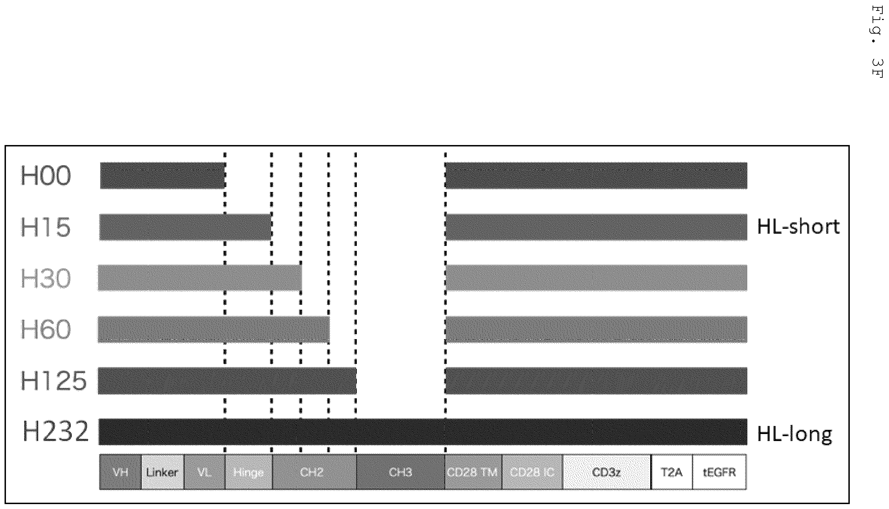

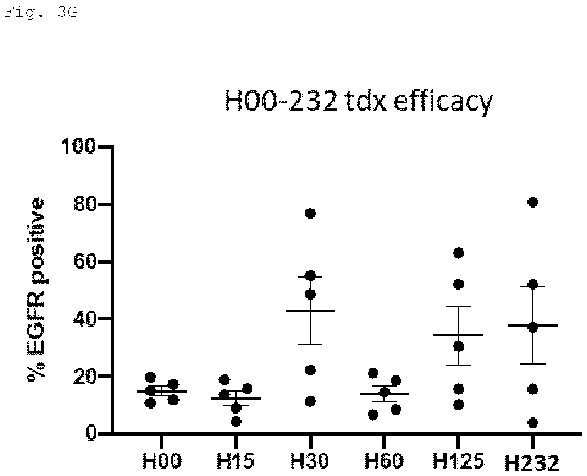

[Fig. 3F] Fig. 3F is a diagram showing an overview of 6 types of spacers having different lengths in Eval-CARs. Note that the notations of "H00" to "H232" shown in Fig. 3F correspond to those in Figs. 3G to 3M.
[Fig. 3G] Fig. 3G is a dot-plot diagram showing results of analyzing introduction efficiency of the Eval-CAR gene before tEGFR selection. In the figure, dots indicate origins (5 healthy subjects) of cells in which the Eval-CAR gene was introduced. Thick lines indicate average values, and whiskers indicate standard error.

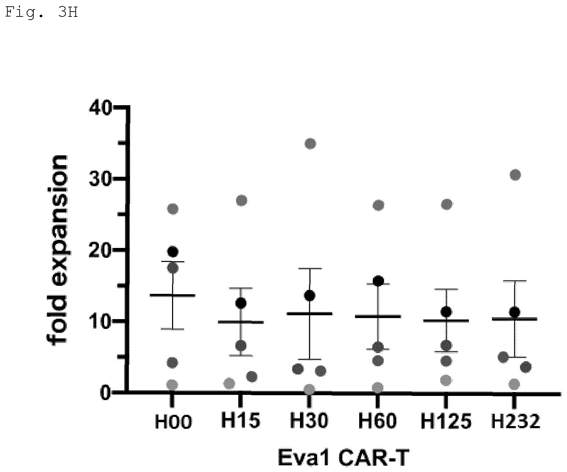

[Fig. 3H] Fig. 3H is a dot-plot diagram showing results of analyzing introduction efficiency of the Eval-CAR gene after the tEGFR selection. In the figure, dots indicate origins (5 healthy subjects) of cells in which the Eval-CAR gene was introduced. Thick lines indicate average values, and whiskers indicate standard error.

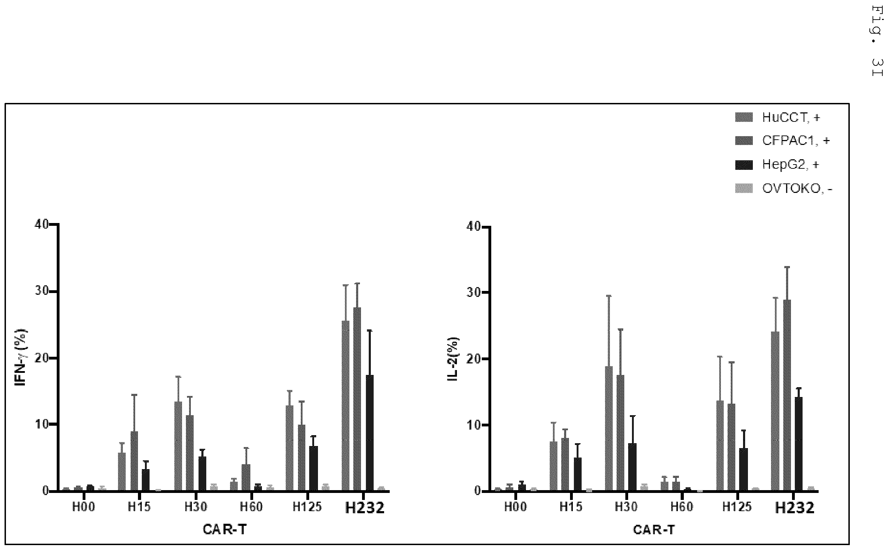

[Fig. 3I] Fig. 3I is graphs showing results of analyzing cytokine production of Eval-CAR-T against various types of tumor cells. In the figure, the graph on the left side shows the results of analyzing IFN-γ production, and the graph on the right side shows the results of analyzing IL-2 production. The results on HuCCT-1, CFPAC-1, HepG2, and OVTOKO are shown in order from the left for each introduced Eval-CAR.

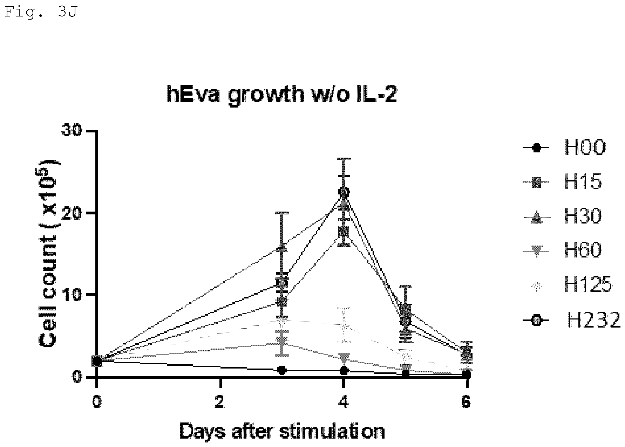

[Fig. 3J] Fig. 3J is a graph showing amplification efficiency of the Eval-CAR-T after antigen stimulation in the absence of IL-2. In the figure, the horizontal axis shows the number of days after the start of the antigen stimulation (co-culture with Eval-positive HuCCT-1). The vertical axis shows the cell count of the Eval-CAR-T.

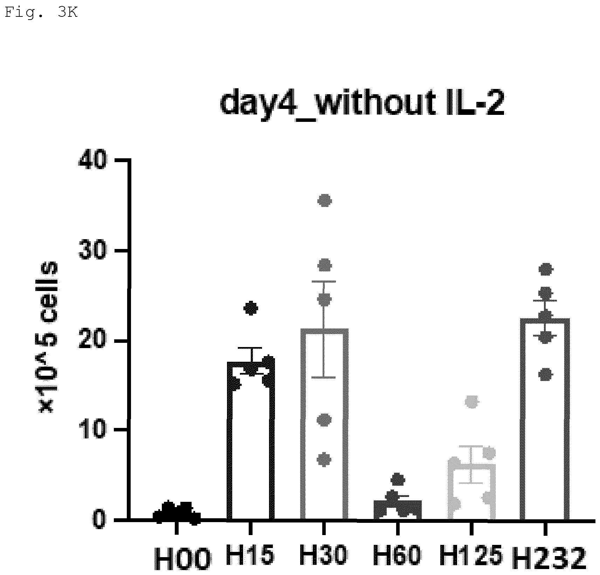

[Fig. 3K] Fig. 3K is a graph showing amplification efficiency of the Eval-CAR-T after antigen stimulation in the absence of IL-2. In the figure, the horizontal axis shows the type of introduced CAR. The vertical axis shows the cell count of the Eval-CAR-T.

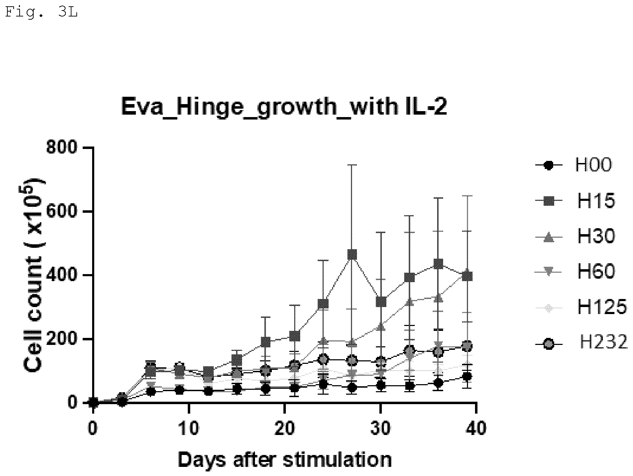

[Fig. 3L] Fig. 3L is a graph showing amplification efficiency of the Eval-CAR-T after antigen stimulation in the presence of IL-2. In the figure, the horizontal axis shows the number of days after the start of the antigen stimulation (co-culture with Eval-positive HuCCT-1). The vertical axis shows the cell count of the Eval-CAR-T.

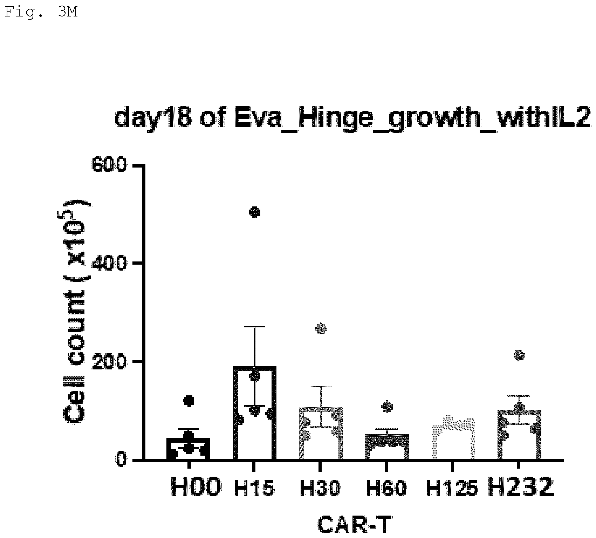

[Fig. 3M] Fig. 3M is a graph showing amplification efficiency of the Eval-CAR-T after antigen stimulation in the presence of IL-2. In the figure, the horizontal axis shows the type of introduce CAR. The vertical axis shows the cell count of the Eval-CAR-T.

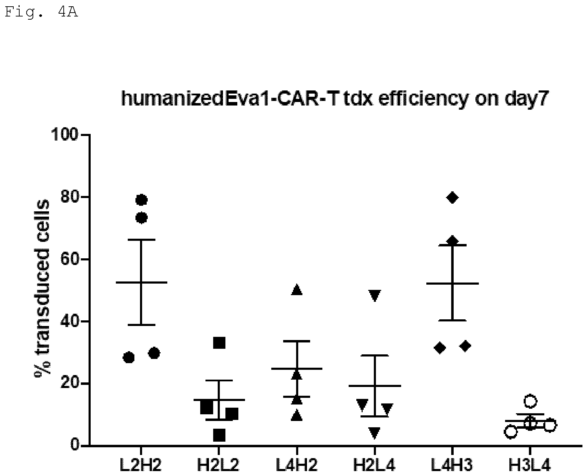

[Fig. 4A] Fig. 4A is a dot-plot diagram showing results of analyzing introduction efficiency of the Eval-CAR (hEva1-CAR) gene using the anti-Eval humanized antibody sequence, before tEGFR selection. In the figure, dots indicate origins (4 healthy subjects) of cells in which the hEva1-CAR gene was introduced. Whiskers indicate standard error, and center lines between the whiskers indicate average values.

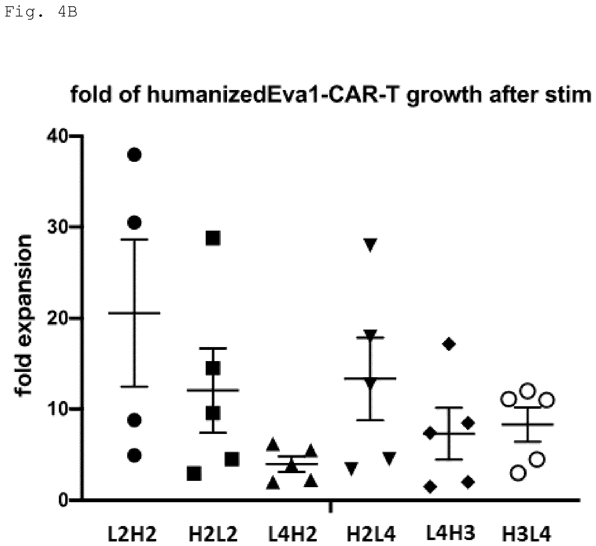

[Fig. 4B] Fig. 4B is a dot-plot diagram showing results of analyzing amplification efficiency of the hEval-CAR-Ts after the tEGFR selection. In the figure, dots indicate origins (4 healthy subjects) of cells in which the hEva1-CAR gene was introduced. Whiskers indicate standard error, and center lines between the whiskers indicate average values.

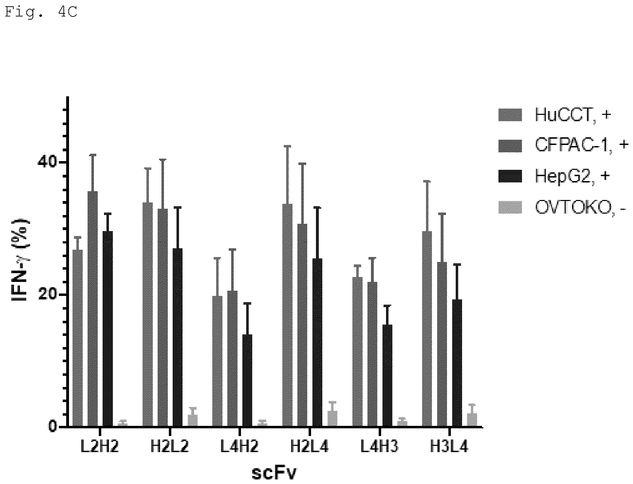

[Fig. 4C] Fig. 4C is a graph showing results of analyzing IFN-γ production of the hEval-CAR-Ts against various types of tumor cells. The results on HuCCT-1, CFPAC-1, HepG2, and OVTOKO are shown in order from the left for each introduced Eval-CAR.

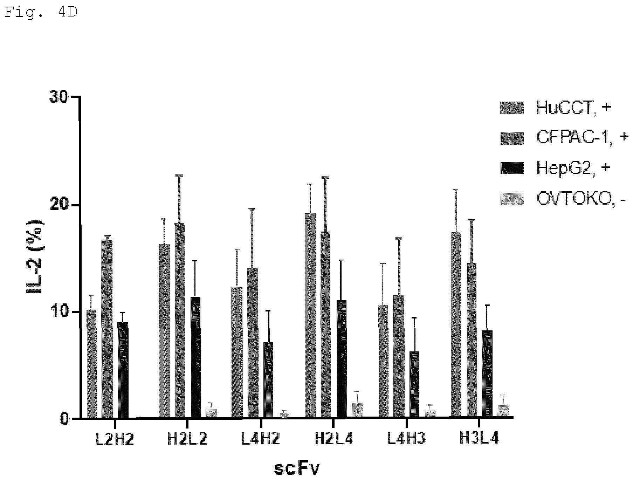

[Fig. 4D] Fig. 4D is a graph showing results of analyzing IL-2 production of the hEval-CAR-Ts against various types of tumor cells. The results on HuCCT-1, CFPAC-1, HepG2, and OVTOKO are shown in order from the left for each introduced Eval-CAR.

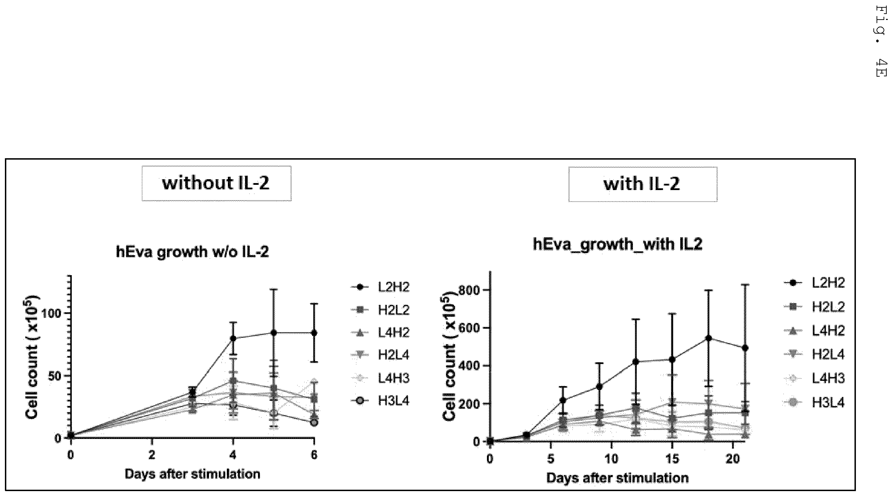

[Fig. 4E] Fig. 4E is graphs showing amplification efficiency of the hEval-CAR-Ts after antigen stimulation. In the figure, the left side shows the results in the absence of IL-2, and the right side shows the results in the presence of IL-2. In each graph, the horizontal axis shows the number of days after the start of the antigen stimulation (co-culture with Eval-positive HuCCT-1). The vertical axis shows the cell count of the hEval-CAR-T.

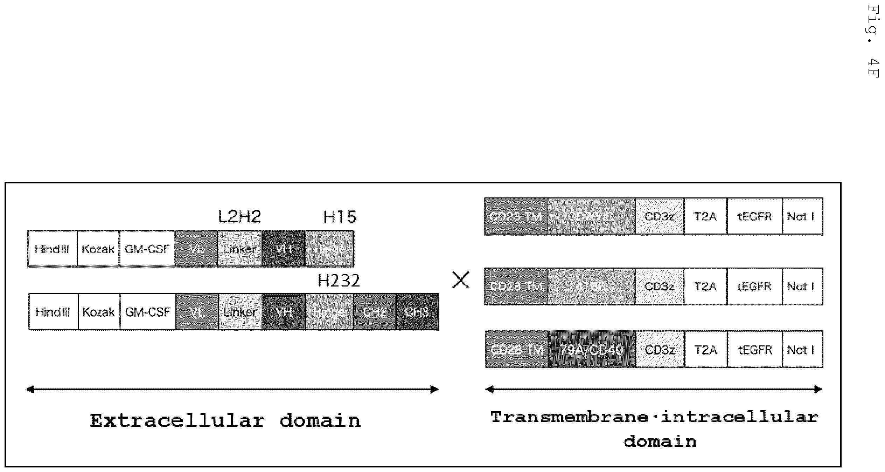

[Fig. 4F] Fig. 4F is a diagram showing an overview of intracellular domains and 6 types of hEval-CAR-Ts having different spacer lengths (antigen recognition site: L2H2).

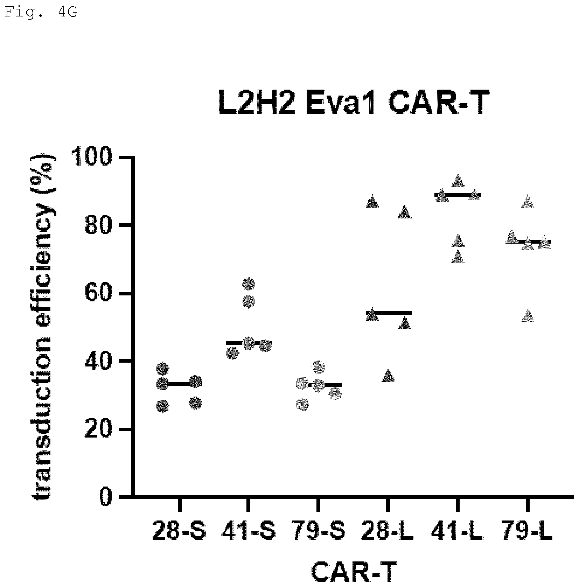

[Fig. 4G] Fig. 4G is a dot-plot diagram showing results of analyzing introduction efficiency of the hEva1-CAR genes before tEGFR selection. In the figure, dots indicate origins (5 healthy subjects) of cells in which the hEva1-CAR gene was introduced. Center lines indicate average values. "28", "41", and "79" on the horizontal axis indicate that the intracellular domains which the CARs had were "CD28IC", "4-1BBIC", and "CD79A/CD40IC", respectively. In addition, "-S" and "-L" indicate that the spacers were "H15" and "H232" shown in Fig. 4F, respectively.

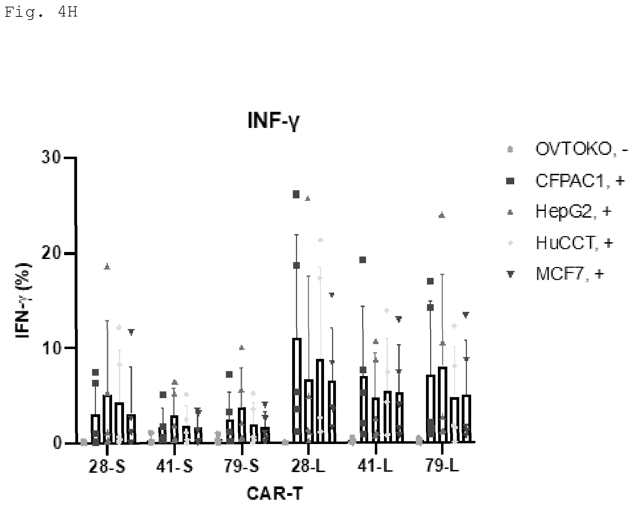

[Fig. 4H] Fig. 4H is a graph showing results of analyzing IFN-γ production of the hEval-CAR-T to various types of tumor cells. The results on OVTOKO, CFPAC-1, HepG2, HuCCT, and MCF7 are shown in order from the left for each introduced Eval-CAR. "28", "41", and "79" on the horizontal axis indicate that the intracellular domains which the CARs had were "CD28IC", "4-1BBIC", and "CD79A/CD40IC", respectively. In addition, "-S" and "-L" indicate that the spacers were "H15" and "H232" shown in Fig. 4F, respectively.

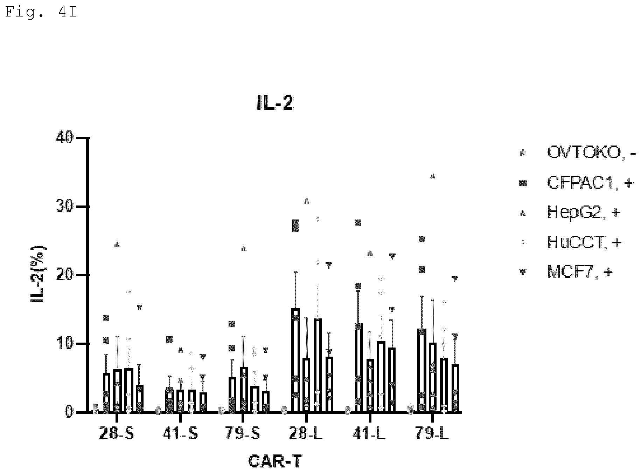

[Fig. 4I] Fig. 4I is a graph showing results of analyzing IL-2 production of the hEval-CAR-T to various types of tumor cells. The results on OVTOKO, CFPAC-1, HepG2, HuCCT, and MCF7 are shown in order from the left for each introduced Eval-CAR. "28", "41", and "79" on the horizontal axis indicate that the intracellular domains which the CARs had were "CD28IC", "4-1BBIC", and "CD79A/CD40IC", respectively. In addition, "-S" and "-L" indicate that the spacers were "H15" and "H232" shown in Fig. 4F, respectively.

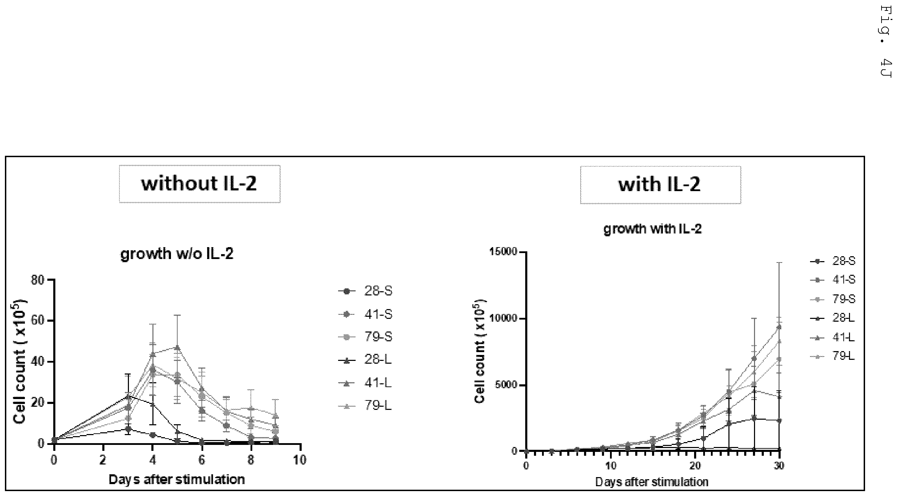

[Fig. 4J] Fig. 4J is graphs showing amplification efficiency of the hEval-CAR-T after antigen stimulation. In the figure, the graph on the left side shows the result in the absence of IL-2. The graph on the right side shows the result in the presence of IL-2. In each graph, the horizontal axis shows the number of days after the start of the antigen stimulation (co-culture with Eval-positive HuCCT-1). The vertical axis shows the cell count of the hEval-CAR-T. In addition, "28", "41", and "79" in the legends indicate that the intracellular domains which the CARs had were "CD28IC", "4-1BBIC", and "CD79A/CD40IC", respectively. In addition, "-S" and "-L" indicate that the spacers were "H15" and "H232" shown in Fig. 4F, respectively.

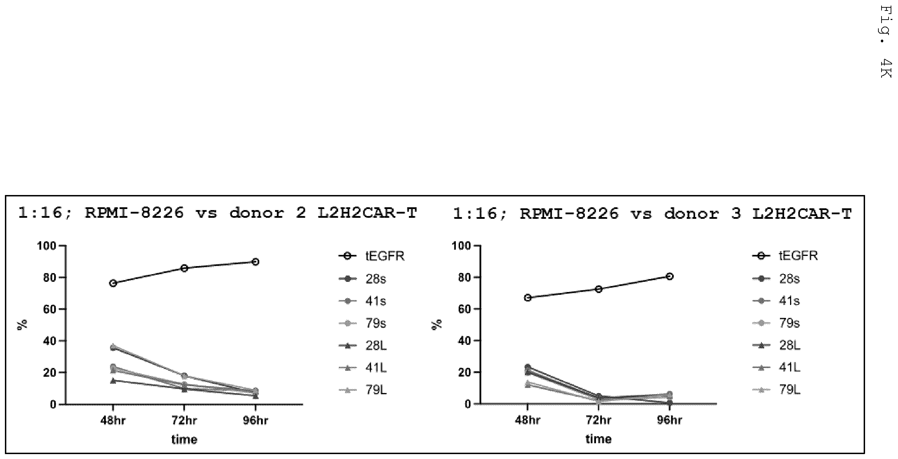

[Fig. 4K] Fig. 4K is graphs showing results of a co-culture test between the hEval-CAR-T and Eval-positive myeloma cells (RPMI-8226). In the figure, the graph on the left side shows the result of hEval-CAR-T prepared from the peripheral blood of donor 2, and the graph on the right side shows the result of hEval-CAR-T prepared from the peripheral blood of donor 3. The vertical axis on each graph shows the percentage of tumor cells accounting for all the cells. The horizontal axis shows a time after the start of the co-culture. In addition, "28", "41", and "79" in the legends indicate that the intracellular domains which the CARs had were "CD28IC", "4-1BBIC", and "CD79A/CD40IC", respectively. In addition, "s" and "L" indicate that the spacers were "H15" and "H232" shown in Fig. 4F, respectively.

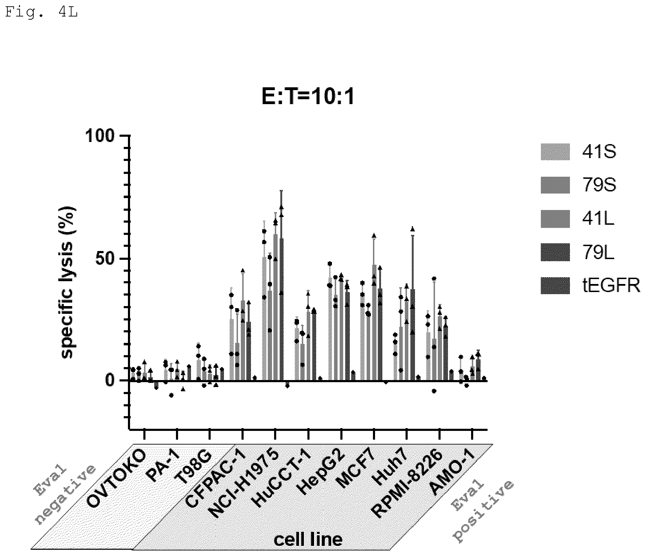

[Fig. 4L] Fig. 4L is a graph showing results of co-culturing the hEval-CAR-T and various types of tumor cell lines labeled with ⁵¹Cr and analyzing cytotoxic activity. In the figure, the vertical axis shows the percentage of injured cells based on the amount of ⁵¹Cr released in a culture supernatant. The horizontal axis shows the various types of tumor cell lines together with whether the expression of Eval was negative or positive. In addition, "41" and "79" in the legends indicate that the intracellular domains which the CARs had were "4-1BBIC" and "CD79A/CD40IC", respectively. In addition, "S" and "L" indicate that the spacers were "H15" and "H232" shown in Fig. 4F, respectively.

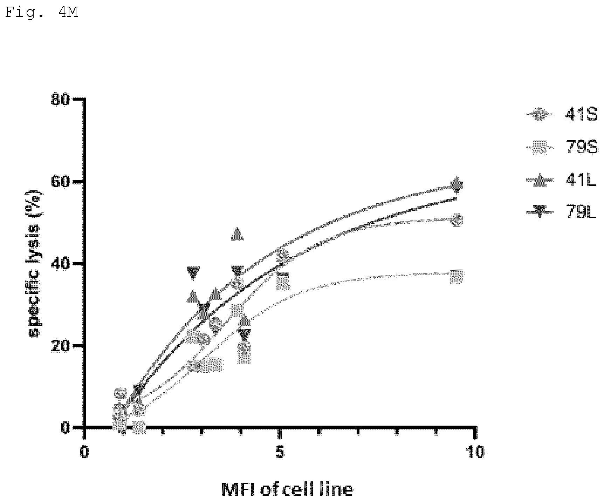

[Fig. 4M] Fig. 4M is a graph showing results of analyzing correlativity between the expression level of the human Eval in various types of tumor cell lines and the cytotoxic activity of the hEval-CAR-T against the various types of tumor cell lines based on the results shown in Fig. 4L. In the figure, the vertical axis shows the percentage of injured cells based on the amount of ⁵¹Cr released in a culture supernatant. The horizontal axis shows a mean fluorescence intensity (MFI) obtained by a flow cytometry analysis using the anti-human Eval antibody in various types of tumor cell lines, which correlates with the Eval expression level. In addition, "41" and "79" in the legends indicate that the intracellular domains which the CARs had were "4-1BBIC" and "CD79A/CD40IC", respectively. In addition, "S" and "L" indicate that the spacers were "H15" and "H232" shown in Fig. 4F, respectively.

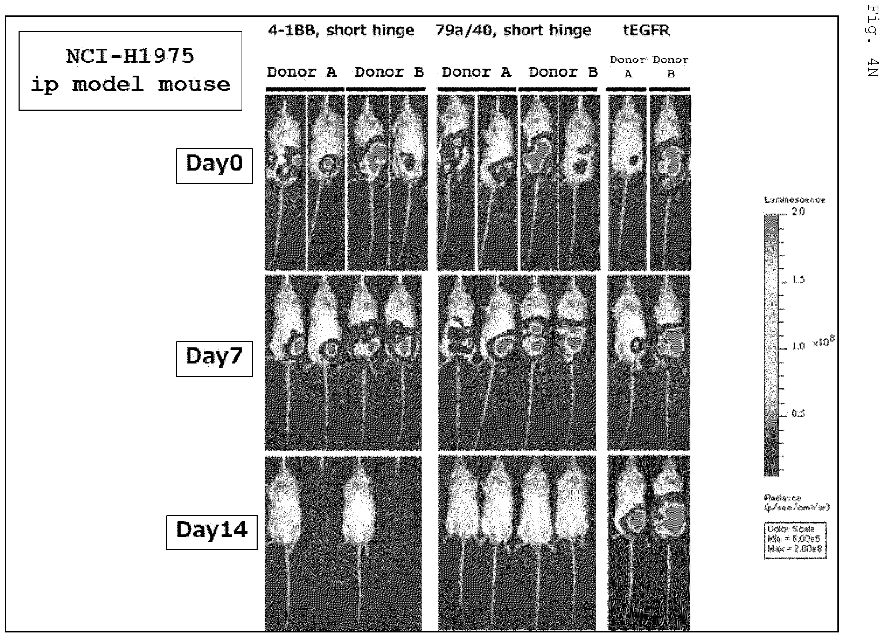

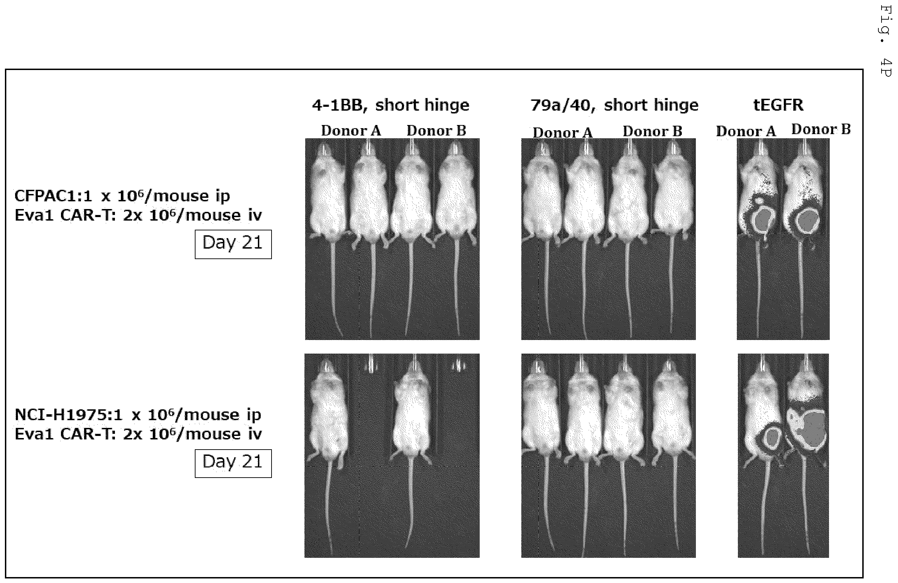

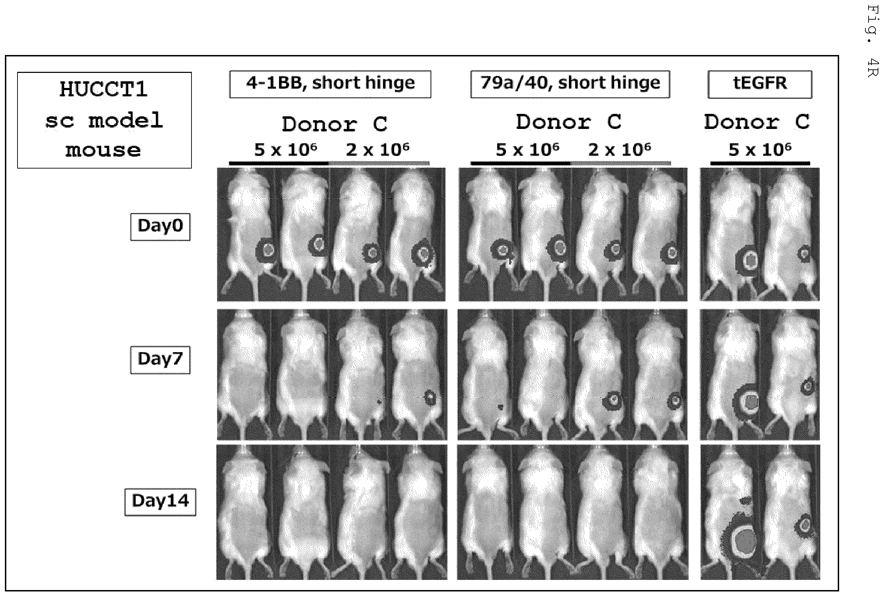

[Fig. 4N] Fig. 4N is a diagram showing results of introducing L2H2 hEval-CAR-T prepared from the peripheral bloods derived from different two donors (donor A, donor B) into mice bearing a human lung adenocarcinoma cell line (H1975) in which a firefly luciferase gene was introduced, and analyzing the antitumor activity of the CAR-T through bioluminescent imaging. As the L2H2 hEval-CAR, those obtained by linking an antigen recognition site (L2H2) and a transmembrane·intracellular domain (domain containing 4-1BB or CD79A/CD40) with a short spacer (notated by "short hinge" in the figure, "H15" shown in Fig. 4F) were used. The analysis was conducted also by preparing an administration example of tEGFR introduction cells (Mock CAR-T-cells in which only tEGFR was transduced) as a control. In addition, "Day" in the figure indicates the number of days after the CAR-T was administered. Note that at "Day 14" of the administration example of the L2H2 hEval-CAR-T that contained 4-1BB in the transmembrane·intracellular domain, two mice are lost. This is because these two mice were dead due to a problem in the experiment procedure. In addition, the emission spectrum is also applied to Figs. 4P and 4R.

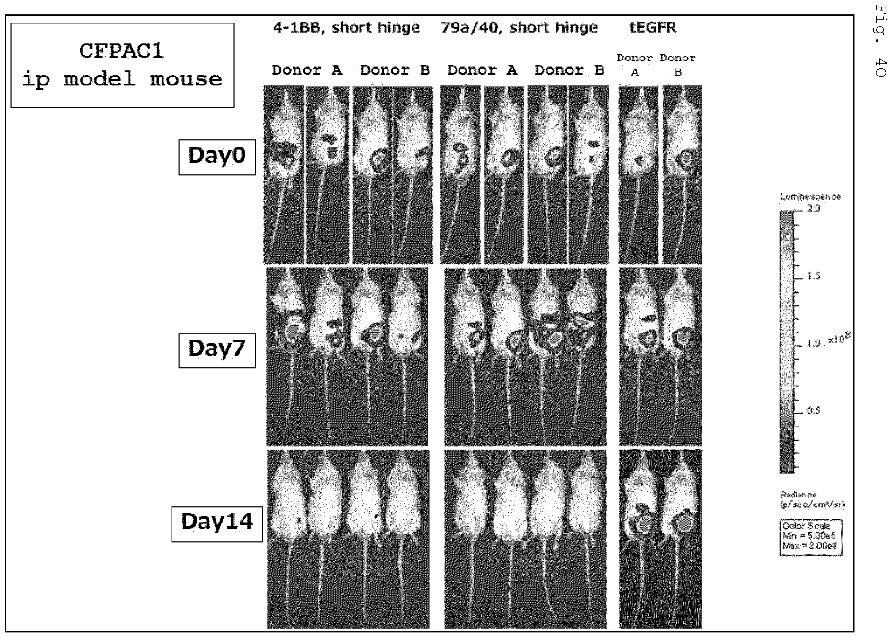

[Fig. 4O] Fig. 4O is a diagram showing results of introducing L2H2 hEval-CAR-T prepared from the peripheral bloods derived from different two donors (donor A, donor B) into mice bearing a human pancreatic cancer cell line (CFPAC1) in which a firefly luciferase gene was introduced, and analyzing the antitumor activity of the CAR-T through bioluminescent imaging. As the L2H2 hEval-CAR, those obtained by linking an antigen recognition site (L2H2) and a transmembrane·intracellular domain (domain containing 4-1BB or CD79A/CD40) with a short hinge were used. The analysis was conducted also by preparing an administration example of tEGFR introduction cells as a control. In addition, "Day" in the figure indicates the number of days after the CAR-T was administered. The emission spectrum is also applied to Figs. 4P and 4R.
[Fig. 4P] Fig. 4P is a diagram showing results of introducing L2H2 hEval-CAR-T prepared from the peripheral bloods derived from different two donors (donor A, donor B) into mice bearing a human pancreatic cancer cell line (CFPAC1) or a human lung adenocarcinoma cell line (H1975) in which a firefly luciferase gene was introduced, and analyzing the antitumor activity of the CAR-T through bioluminescent imaging. As the L2H2 hEval-CAR, those obtained by linking an antigen recognition site (L2H2) and a transmembrane·intracellular domain (domain containing 4-1BB or CD79A/CD40) with a short hinge were used. The analysis was conducted also by preparing an administration example of tEGFR introduction cells as a control. In addition, "Day" in the figure indicates the number of days after the CAR-T was administered. The emission spectrum is also applied to Figs. 4P and 4R.

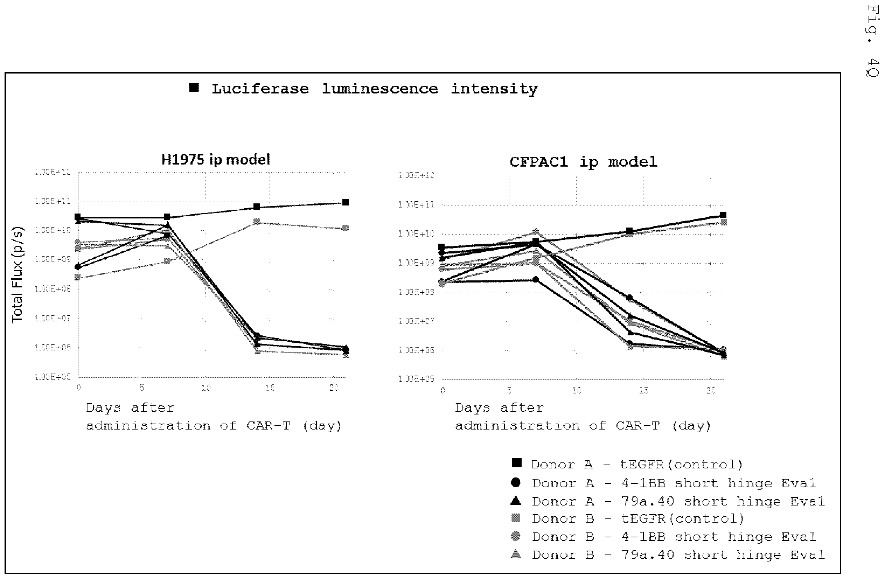

[Fig. 4Q] Fig. 4Q is graphs showing results (emission intensities) of introducing L2H2 hEval-CAR-T prepared from the peripheral bloods derived from different two donors (donor A, donor B) into mice bearing a human pancreatic cancer cell line (CFPAC1) or a human lung adenocarcinoma cell line (H1975) in which a firefly luciferase gene was introduced, and analyzing the antitumor activity of the CAR-T through bioluminescent imaging with time. As the L2H2 hEval-CAR, those obtained by linking an antigen recognition site (L2H2) and a transmembrane·intracellular domain (domain containing 4-1BB or CD79A/CD40) with a short hinge were used. The analysis was conducted also by preparing an administration example of tEGFR introduction cells as a control.
[Fig. 4R] Fig. 4R is a diagram showing results of introducing L2H2 hEval-CAR-T prepared from the peripheral blood derived from a donor C into mice bearing a human cholangiocarcinoma cell line (HuCC T-cell) in which a firefly luciferase gene was introduced, and analyzing the antitumor activity of the CAR-T through bioluminescent imaging. As the L2H2 hEval-CAR, those obtained by linking an antigen recognition site (L2H2) and a transmembrane·intracellular domain (domain containing 4-1BB or CD79A/CD40) with a short hinge were used. The analysis was conducted also by preparing an administration example of tEGFR introduction cells as a control. In addition, "Day" in the figure indicates the number of days after the CAR-T was administered.

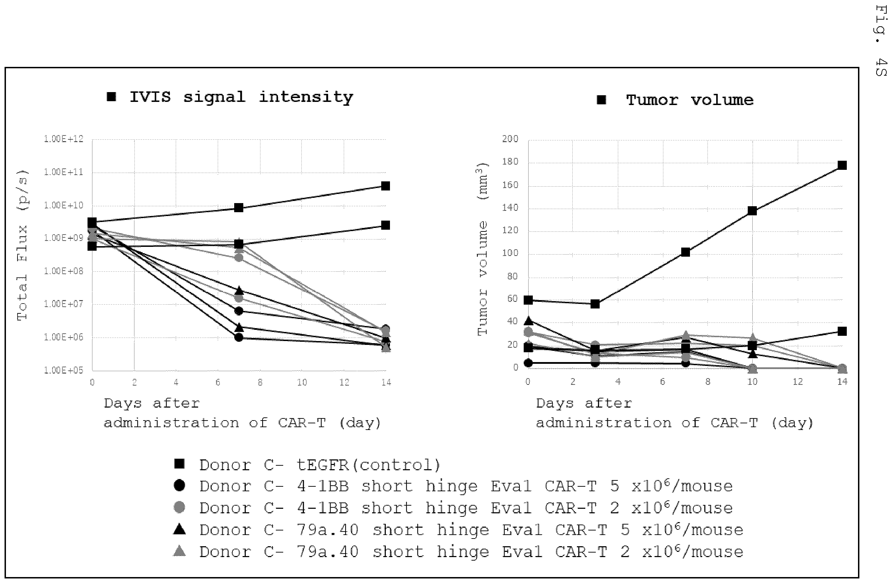

[Fig. 4S] Fig. 4S is graphs showing results of introducing L2H2 hEval-CAR-T prepared from the peripheral blood derived from the donor C into mice bearing a human cholangiocarcinoma cell line (HuCC T-cells) in which a firefly luciferase gene was introduced, and conducting an analysis. As the L2H2 hEval-CAR, those obtained by linking an antigen recognition site (L2H2) and a transmembrane·intracellular domain (domain containing 4-1BB or CD79A/CD40) with a short hinge were used. The analysis was conducted also by preparing an administration example of tEGFR introduction cells as a control. In the figure, the graph on the left side shows the results (emission intensities) of analyzing the antitumor activity of the CAR-T through bioluminescent imaging with time. The graph on the right side shows results of analyzing, with time, tumor volumes in the cancer-bearing mice to which the CAR-T was administered.

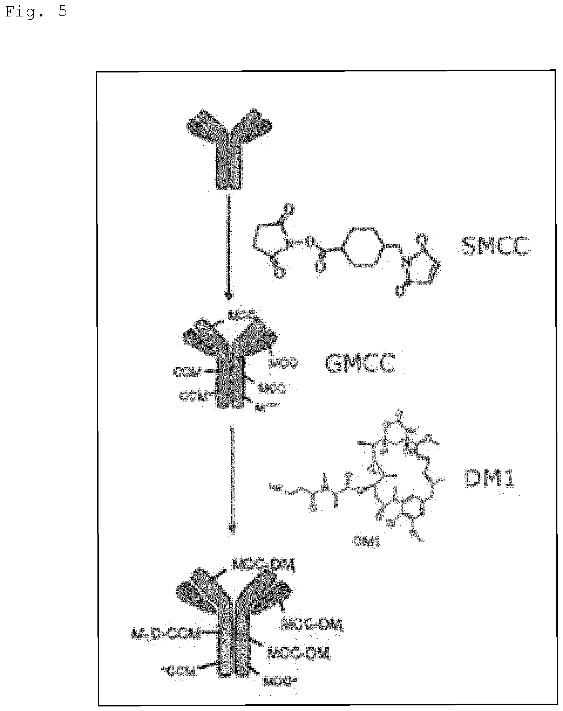

[Fig. 5] Fig. 5 is a diagram showing an overview of a process of producing an antibody-drug conjugate (B2E5-ADC) containing an anti-human Eval humanized antibody.

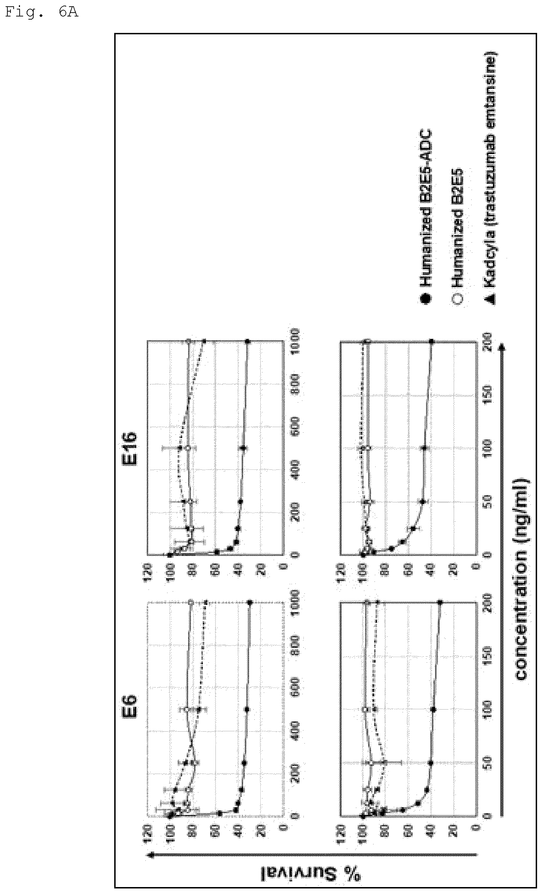

[Fig. 6A] Fig. 6A is graphs showing results of a cytotoxicity assay of the B2E5-ADC against gliomas (hGIC E6, hGIC E16). In each graph, the vertical axis shows the survival rate of tumor cells, and the horizontal axis shows the concentration of ADC or the antibody added to a medium.

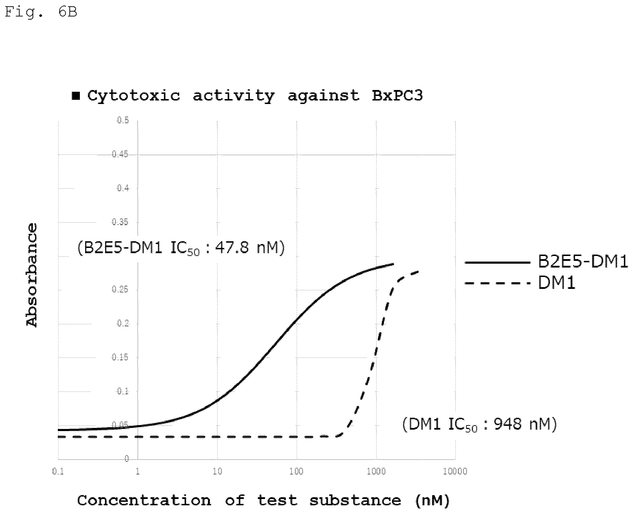

[Fig. 6B] Fig. 6B is graphs showing results of a cytotoxicity assay of the B2E5-ADC against pancreatic cancer (BxPC3). In the figure, the vertical axis in each graph shows an absorbance reflecting the degree of injury in tumor cells. The horizontal axis shows the concentration of ADC or the drug (DM1) added to a medium.

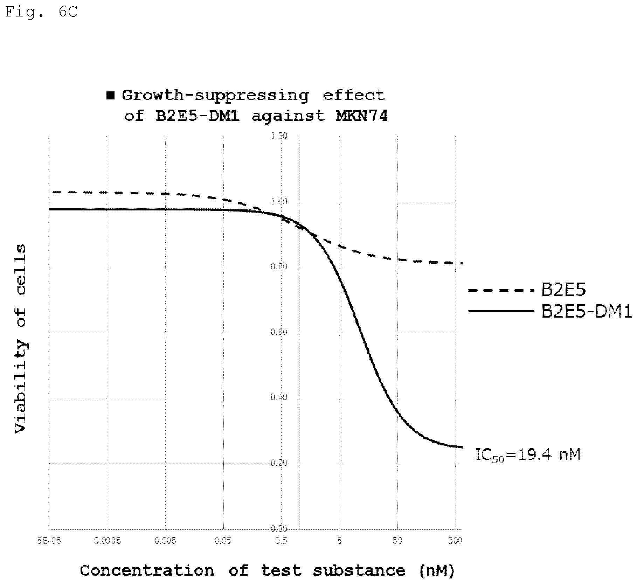

[Fig. 6C] Fig. 6C is graphs showing results of a cytotoxicity assay of the B2E5-ADC against stomach cancer (MKN74). In the figure, the vertical axis in each graph shows the survival rate of tumor cells. The horizontal axis shows the concentration of ADC or the antibody added to a medium.

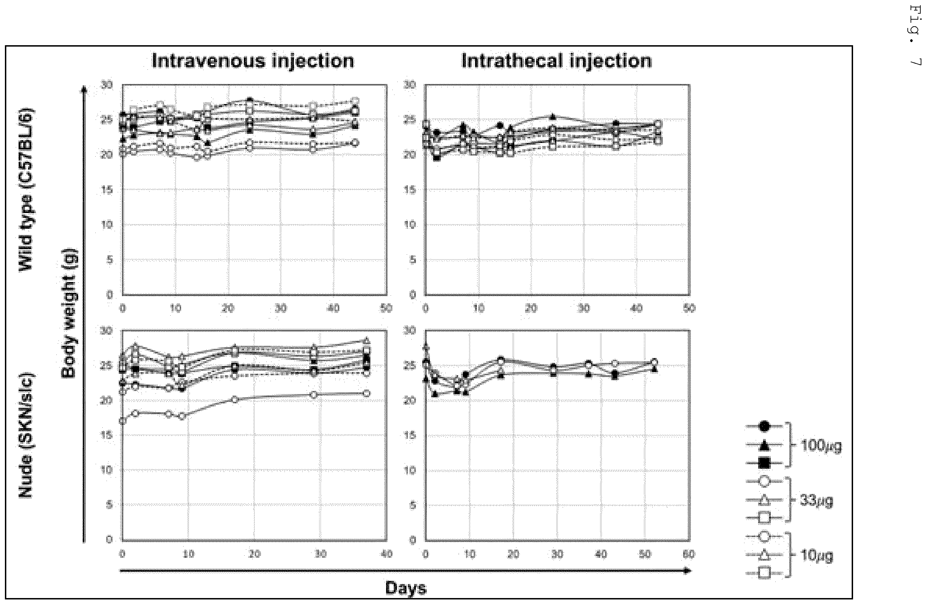

[Fig. 7] Fig. 7 is graphs showing results of a toxicity test of the B2E5-ADC on mice. More specifically, single doses of the B2E5-ADC (100, 33, 10 µg) were administered to wild type (C57BL/6) or nude mice (SKN/slc) through caudal vein (intravenous injection) or lumbar puncture (intrathecal injection), and Fig. 7 is graphs showing change in body weight of these mice with time. In the figure, circle, triangle, and rectangle each represent data of one individual mouse.

### [Description of Embodiments]

### <Anti-Eva1 Antibody>

The present invention relates to an antibody or a functional fragment thereof which binds to a human-derived Eval protein (human Eval).

In the present invention, "Eva (Epithelial V-like antigen) 1" is a single-pass transmembrane-type cell membrane protein which is a molecule also referred to as MPZL2 (Myelin protein Zero-like 2) and is involved in a cytoskeleton system. If derived from human, the Eval is typically a protein having an amino acid sequence set forth in SEQ ID NO: 54 (a protein encoded by a nucleotide sequence set forth in SEQ ID NO: 53) . However, the DNA sequence of a gene is mutated naturally (that is, non-artificially) by its mutation or the like, and the amino acid sequence of a protein encoded by the gene is also modified accordingly. Thus, the "Eval protein" according to the present invention is not limited to the above-described protein having the typical amino acid sequence, and also includes such natural mutants.

The "antibody" in the present invention includes all classes and subclasses of immunoglobulins. The "antibody" includes a polyclonal antibody and a monoclonal antibody. The "polyclonal antibody" is an antibody preparation containing different antibodies against different epitopes. In addition, the "monoclonal antibody" means an antibody obtained from a substantially uniform antibody population. In contrast to a polyclonal antibody, a monoclonal antibody recognizes a single determinant on an antigen. The antibody of the present invention is preferably a monoclonal antibody. The antibody of the present invention is an antibody separated and/or recovered (that is, isolated) from components in a natural environment.

The reactivity of the antibody of the present invention to a human Eval is preferably 10⁻⁶ or less and more preferably 6×10⁻⁷ or less as a KD (dissociation constant) value. The "reactivity" means a binding affinity and/or specificity to a human Eval, which is an antigen. This reactivity can be evaluated by a person skilled in the art through an immunological approach, and for example, can be determined as a KD value by an analysis using the surface plasmon resonance method as shown in Examples described later. In addition, the antibody of the present invention may be an antibody which binds to an Eval protein derived from another animal (for example, a mouse Eval protein (typically, a protein having an amino acid sequence set forth in SEQ ID NO: 56 or a protein encoded by a nucleotide sequence set forth in SEQ ID NO: 55)) besides the human Eval protein.

The antibody of the present invention may have at least one cytotoxic activity selected from an ADCC activity and a CDC activity or may have both of an ADCC activity and a CDC activity.

In the present invention, the "ADCC activity (antibody-dependent cellular cytotoxic activity)" means an activity in which when an antibody binds to a cell-surface antigen on the target cell, then an effector cell (an immune cell such as a NK cell or a monocyte) further binds to the Fc region of the antibody, so that the effector cell is activated, thereby releasing a factor to kill the target cell. On the other hand, the "CDC activity (complement-dependent cytotoxic activity)" means an activity in which when an antibody binds to a target cell, a complement system is activated, and as a result, the target cell is lysed.

In addition, the antibody of the present invention is preferably an antibody having an ADCC activity in an amount of 0.01 ug/mL against target cells expressing the human Eval. Whether or not the antibody of the present invention has such ADCC activity can be measured, for example, by a method described in Examples of PTL 2. More specifically, in a case where Daudi cells expressing the human Eval are used as target cells and an ADCC activity is evaluated in terms of the cell lysis rate, when 0.01 ug/mL of the antibody of the present invention is used, the ADCC activity is a cell lysis rate of preferably 30% or more, more preferably 50% or more, and further preferably 70% or more.

In addition, the antibody of the present invention is an antibody having a CDC activity in an amount of preferably 0.30 µg/mL, and more preferably 0.01 ug/mL against target cells expressing the human Eval. Whether or not the antibody of the present invention has such CDC activity can be measured, for example, by a method described in Examples of PTL 2. More specifically, in a case where Daudi cells expressing the human Eval are used as target cells and a CDC activity is evaluated in terms of the cell lysis rate, when 0.30 ug/mL of the anti-Eval antibody of the present invention is used, the CDC activity is a cell lysis rate of preferably 20% or more, more preferably 40% or more, further preferably 60 or more, and more preferably 80% or more.

The antibody of the present invention may also have an antitumor activity. In the present invention, the "antitumor activity" means at least any one activity of an activity of suppressing the growth of cancer cells, an activity of inducing the death of cancer cells, and an activity of suppressing the metastasis of cancer cells. The antitumor activity can be evaluated, for example, by an analysis using cancer-bearing models (mice in which B16 melanoma cells are inoculated, or the like) as described in Examples of PTL 2. More specifically, after B16 melanoma cells is, for example, intravenously administered to mice, from the following day or several weeks thereafter, an anti-Eval antibody is, for example, intravenously administered every day or every few days. Then, the number of colonies of B16 melanoma cells formed in the lung is counted, so that the in vivo anticancer activity can be evaluated. As negative controls, a control antibody having the same isotype may be administered, or PBS or the like may be administered. Then, the anti-Eval antibody can be determined to have an antitumor activity if the number of colonies formed in the anti-Eval-antibody administration group is smaller than that in the negative-control administration group (given that the number of colonies formed in the negative-control administration group is taken as 100%, the number of colonies formed in the anti-Eval-antibody administration group is preferably 60% or less, more preferably 40% or less, further preferably 20% or less, and more preferably 10% or less ).

The antibody of the present invention only has to have reactivity against the human Eval, and the origin, type, shape, and the like of the antibody are not particularly limited. Specifically, the antibody of the present invention includes an antibody derived from a non-human animal (for example, a mouse antibody, a rabbit antibody, a rat antibody, or a camel antibody), a human-derived antibody, a chimeric antibody, and a humanized antibody.

In the present invention, the "antibody derived from a non-human animal" includes, for example, antibodies against the human Eval having variable regions as follows, as disclosed in PTL 2.
(a) An antibody having a heavy chain variable region containing an amino acid sequence set forth in SEQ ID NO: 27 and a light chain variable region containing an amino acid sequence set forth in SEQ ID NO: 21.
(b) An antibody having a heavy chain variable region containing an amino acid sequence set forth in SEQ ID NO: 39 and a light chain variable region containing an amino acid sequence set forth in SEQ ID NO: 33.
(c) An antibody having a heavy chain variable region containing an amino acid sequence set forth in SEQ ID NO: 49 and a light chain variable region containing an amino acid sequence set forth in SEQ ID NO: 44.

Moreover, the present invention relates to antibodies against a human Eval which has CDRs as follows, for example.
(a) An antibody having CDRs 1 to 3 of a heavy chain determined from the heavy chain variable region containing the amino acid sequence set forth in SEQ ID NO: 27 and CDRs 1 to 3 of a light chain determined from the light chain variable region containing the amino acid sequence set forth in SEQ ID NO: 21.
(b) An antibody having CDRs 1 to 3 of a heavy chain determined from the heavy chain variable region containing the amino acid sequence set forth in SEQ ID NO: 39 and CDRs 1 to 3 of a light chain determined from the light chain variable region containing the amino acid sequence set forth in SEQ ID NO: 33.
(c) An antibody having CDRs 1 to 3 of a heavy chain determined from the heavy chain variable region containing the amino acid sequence set forth in SEQ ID NO: 49 and CDRs 1 to 3 of a light chain determined from the light chain variable region containing the amino acid sequence set forth in SEQ ID NO: 44.

Note that regarding such antibodies, a method for determining a CDR based on an amino acid sequence of a variable region is not particularly limited, but includes, for example, known numbering schemes such as Chothia, IMGT, Kabat, and Aho. More specifically, examples determined by the Chothia numbering method include antibodies against the human Eval which have CDRs as follows.
(a-1) An antibody having amino acid sequences set forth in SEQ ID NOs: 28 to 30 as CDRs 1 to 3 of a heavy chain, respectively, and amino acid sequences set forth in SEQ ID NOs: 22 to 24 as CDRs 1 to 3 of a light chain, respectively. (b) An antibody having amino acid sequences set forth in SEQ ID NOs: 40 to 42 as CDRs 1 to 3 of a heavy chain, respectively, and amino acid sequences set forth in SEQ ID NOs: 34 to 36 as CDRs 1 to 3 of a light chain, respectively. (c) An antibody having amino acid sequences set forth in SEQ ID NOs: 50 to 52 as CDRs 1 to 3 of a heavy chain, respectively, and amino acid sequences set forth in SEQ ID NOs: 45 to 47 as CDRs 1 to 3 of a light chain, respectively.

In addition, examples determined by the IMGT numbering method include antibodies against the human Eval that have CDRs as follows.
(a-2) An antibody having amino acid sequences set forth in SEQ ID NOs: 4 to 6 as CDRs 1 to 3 of a heavy chain, respectively, and an amino acid sequence set forth in SEQ ID NO: 1, an amino acid sequence composed of Val-Thr-Ser, and an amino acid sequence set forth in SEQ ID NO: 3 as CDRs 1 to 3 of a light chain, respectively.

In addition, the antibody of the present invention includes not only antibodies having variable regions and/or CDRs composed of such specific amino acid sequences but also antibodies whose amino acid sequences are modified without impairing desirable activities (reactivities against antigens and the like). Such amino acid sequence mutants can be prepared, for example, by mutagenesis into DNAs encoding the above-mentioned variable regions and the like, or by peptide synthesis. Such modifications include, for example, substitution, deletion, addition, and/or insertion of residues in the amino acid sequence of the antibody. A site where the amino acid sequence of the antibody is modified may be a constant region of a heavy chain or a light chain of the antibody or a variable region (a frame region (FR) and CDR) thereof as long as the resulting antibody has activities equivalent to those before modification. It is conceivable that a modification on an amino acid other than those in CDR has a relatively small influence on the reactivity with an antigen. As of now, there are known methods for screening antibodies whose affinities to antigens have been enhanced by modifying an amino acid of CDR (PNAS, 102: 8466-8471 (2005), Protein Engineering, Design & Selection, 21: 485-493 (2008), International Publication No. WO2002/051870, J. Biol. Chem., 280: 24880-24887 (2005), Protein Engineering, Design & Selection, 21: 345-351 (2008), MAbs. Mar-Apr; 6 (2): 437-45 (2014)). Additionally, currently, an antibody whose affinity to an antigen has been enhanced can also be modeled by utilizing an integrated computing chemical system or the like (for example, Molecular Operating Environment manufactured by CCG in Canada) (see, for example, http://www.rsi.co.jp/kagaku/cs/ccg/products/application/ protein.html). Moreover, as described in Protein Eng Des Sel. 2010 Aug; 23 (8): 643-51, a case has been known where CDR1 in the heavy chain variable region and CDR3 in the light chain variable region are not involved in the affinity to an antigen. In addition, similarly, Molecular Immunology 44: 1075-1084 (2007) has reported that in most antibodies, CDR2 of the light chain variable region is not involved in the affinity to an antigen. As described above, regarding the affinity of an antibody to an antigen, equivalent activity can be exhibited without requiring all of CDR1 to 3 in each heavy chain variable region and light chain variable region. Actually, Biochem Biophys Res Commun. 2003 Jul 18; 307 (1): 198-205, J Mol Biol. 2004 Jul 9; 340(3): 525-42, and J Mol Biol. 2003 Aug 29; 331 (5): 1109-20 have reported cases where having at least one CDR of the original antibody maintained the affinity to an antigen.

In addition, regarding the antibody of the present invention, the number of amino acids to be modified in the variable regions is preferably within 10 amino acids, more preferably within 5 amino acids, and further preferably within 3 amino acids (for example, within 2 amino acids, or 1 amino acid). Moreover, all of such modifications are preferably applied to a region other than CDR, that is, FR from the viewpoint that there is a small influence on the reactivity with an antigen. In addition, the number of amino acids to be modified in CDR is preferably within 5 amino acids, and more preferably within 3 amino acids (for example, within 2 amino acids, or 1 amino acid).

The modification of amino acids is preferably conservative substitution. In the present invention, the "conservative substitution" means substitution with another amino acid residue having a chemically similar side chain. Groups of amino acid residues having chemically similar amino acid side chains are well known in the technical field to which the present invention pertains. For example, amino acids can be grouped into acidic amino acids (aspartic acid and glutamic acid), basic amino acids (lysine·arginine·histidine), neutral amino acids such as amino acids having a hydrocarbon chain (glycine·alanine·valine·leucine·isoleucine·proline), amino acids having a hydroxy group (serine·threonine), amino acids containing sulfur (cysteine·methionine), amino acids having an amide group (asparagine·glutamine), amino acids having an imino group (proline), and amino acids having an aromatic group (phenylalanine·tyrosine·tryptophan).

In addition, the antibody of the present invention also includes an antibody in which an amino acid sequence after modification has a variable region containing an amino acid sequence having a homology of 80% or more at the amino acid sequence level with a variable region composed of the above-mentioned specific amino acid sequence as long as the antibody has activity equivalent to that of the antibody before the modification. Such homology only has to be at least 80%, but is preferably 85% or more, more preferably 90% or more, and further preferably 95% or more (for example, 96% or more, 97% or more, 98% or more, or 99% or more). In addition, the sequence homology can be determined by utilizing the BLASTP (amino acid level) program (Altschul et al. J. Mol. Biol., 215: 403-410, 1990). This program is based on the algorithm BLAST of Karlin and Altschul (Proc. Natl. Acad. Sci. USA, 87: 2264-2268, 1990, Proc. Natl. Acad. Sci. USA, 90: 5873-5877, 1993). In the case of analyzing an amino acid sequence by BLASTP, the parameters are set to, for example, score=50, wordlength=3. In addition, in the case of analyzing an amino acid sequence by using the Gapped BLAST program, the analysis can be conducted as described in Altschul et. al. (Nucleic Acids Res. 25: 3389-3402, 1997). In the case of using the BLAST and Gapped BLAST program, default parameters of each program are used. Specific procedures of these analyzing methods are known.

In addition, the expression "having equivalent activities" means, for example, that the reactivity to an antigen is equivalent to that of a target antibody (for example, B2E5-48 mouse antibody described in PTL 2) (for example, 70% or more, preferably 80% or more, and more preferably 90% or more).

In addition, the modification on the antibody of the present invention may be, for example, a modification of post-translational process of the antibody such as changing the number or positions of sites of glycosylation. This makes it possible to improve the ADCC activity of the antibody, for example. The glycosylation of the antibody is typically N-linked or O-linked glycosylation. The glycosylation of the antibody greatly depends on host cells used for expressing the antibody. The modification of the glycosylation pattern can be conducted by a known method such as introduction or deletion of a specific enzyme involved in carbohydrate production (Japanese Unexamined Patent Application Publication No. 2008-113663, United States Patent No. 5047335, United States Patent No. 5510261, United States Patent No. 5278299, and International Publication No. WO99/54342). Moreover, in the present invention, deamidation may be suppressed by substituting an amino acid subjected to deamidation or an amino acid adjacent to an amino acid subjected to deamidation with another amino acid, for the purpose of increasing the stability of the antibody, or other purposes. In addition, the stability of the antibody can also be increased by substituting a glutamic acid with another amino acid. The present invention also provides an antibody stabilized in this way.

In the present invention, the "chimeric antibody" is an antibody obtained by linking a variable region of an antibody of a certain species and a constant region of an antibody of a species different therefrom. A chimeric antibody can be obtained, for example, by: immunizing a mouse with an antigen; cleaving an antibody variable domain (variable region) which binds to the antigen from the gene of a mouse monoclonal antibody; binding this antibody variable domain to the gene of an antibody constant domain (constant region) derived from a human bone marrow; incorporating this into an expression vector; and introducing the expression vector into a host for the production of a chimeric antibody (for example, Japanese Unexamined Patent Application Publication No. Hei 8-280387, United States Patent No. 4816397, United States Patent No. 4816567, and United States Patent No. 5807715).

As the constant region of a chimeric antibody, those of human-derived antibodies are normally used. For example, Cγ1, Cγ2, Cγ3, Cγ4, Cu, Cδ, Cα1, Cα2, and Cε can be utilized as the constant region of the heavy chain. In addition, Cκ and Cλ can be used as the constant region of the light chain. The amino acid sequences of these constant regions and the base sequences encoding these constant regions are known. In addition, in order to improve the stability of the antibody itself or the stability of the production of the antibody, one or more amino acids in the constant regions of the human-derived antibody may be substituted, deleted, added, and/or inserted.

In the present invention, the "humanized antibody" is an antibody obtained by grafting (CDR grafting) a gene sequence of CDR of an antibody derived from a non-human animal onto a gene of a human-derived antibody, and the preparation method is known such as overlap extension PCR (for example, the Specification of European Patent Application Publication No. 239400, the Specification of European Patent Application Publication No. 125023, International Publication No. WO90/07861, and International Publication No. WO96/02576). A variable region of an antibody is normally composed of three CDRs flanked by four FRs. CDRs are regions substantially determining the binding specificity of an antibody. While the amino acid sequences of CDRs are rich in diversity, the amino acid sequences forming FRs often show high homology even among antibodies having different binding specificities. Hence, in general, it is said that the binding specificity of a certain antibody can be grafted onto another antibody by grafting of CDRs. In addition, from the viewpoint of maintaining the function of CDRs, in grafting of a non-human-derived CDR onto human FRs, human FRs having high homology with the non-human animal-derived FRs are selected. That is, since amino acids in CDR not only recognize an antigen but also coordinate with amino acids of FRs near the CDR and are also involved in maintaining the loop structure of the CDR, it is preferable to utilize human FRs having amino acid sequences having high homologies with amino acid sequences of FRs adjacent to the CDR to be grafted.

Known human FRs having high homologies with non-human animal-derived FRs can be searched for, for example, by utilizing a search system (http://www.bioinf.org.uk/abysis/) specialized for antibodies available on the Internet. A mutation can be introduced into a sequence of a non-human-derived antibody other than CDR to match the sequence of a human FR thus obtained. Alternatively, in the case where a gene (c DNA) encoding an amino acid sequence of a human FR obtained by the search is available, the non-human-derived CDR may be introduced into the sequence. The introduction and the like of mutations can be conducted by using techniques in the art such as nucleic acid synthesis and site-directed mutagenesis.

By qualitatively or quantitatively measuring and evaluating the binding affinity of the humanized antibody thus prepared to an antigen, FRs of a human-derived antibody which allow the CDR to form a favorable antigen-binding site when being ligated via the CDR can be favorably selected. In addition, as necessary, the amino acid residue of FR can be substituted so that CDR of a humanized antibody can form an appropriate antigen-binding site, in accordance with a method described in Sato, K. et al., Cancer Res, 1 993, 53, 851-856 or the like. Moreover, a mutation FR sequence having desired properties can be selected by measuring and evaluating the binding affinity of the mutant antibody with the amino acid substituted to an antigen.

In the present invention, the "humanized antibody" includes, for example, a humanized antibody which has a light chain variable region containing CDRs each containing an amino acid sequence set forth in SEQ ID NO: 1, an amino acid sequence composed of Val-Thr-Ser, and an amino acid sequence set forth in SEQ ID NO: 3 and a heavy chain variable region containing CDR1 to 3 each containing amino acid sequences set forth in SEQ ID NOs: 4 to 6, respectively, and which binds to the human-derived Eval protein.

More specifically, as shown in Examples described later, the humanized antibody includes a humanized antibody wherein
the light chain variable region comprises one amino acid sequence selected from amino acid sequences set forth in SEQ ID NOs: 7 to 10, amino acid sequences having homologies of 80% or more with the amino acid sequences set forth in SEQ ID NOs: 7 to 10, and an amino acid sequence derived from at least one of the amino acid sequences set forth in SEQ ID NOs: 7 to 10 by substitution, deletion, addition, and/or insertion of one or more amino acids, and
the heavy chain variable region comprises one amino acid sequence selected from amino acid sequences set forth in SEQ ID NOs: 13 to 16, amino acid sequences having homologies of 80% or more with the amino acid sequences set forth in SEQ ID NOs: 13 to 16, and an amino acid sequence derived from at least one of the amino acid sequences set forth in SEQ ID NOs: 13 to 16 by substitution, deletion, addition, and/or insertion of one or more amino acids.

Note that although the modification, homology, and the like of the variable regions is as described in the "antibody derived from a non-human animal", in the humanized antibody of the present invention, it is preferable that the sequences of CDRs be not modified, and the sequences of CDR1 to 3 of the light chain variable region and CDR1 to 3 of the heavy chain variable region be specified to the amino acid sequence set forth in SEQ ID NO: 1, the amino acid sequence composed of Val-Thr-Ser, and the amino acid sequence set forth in SEQ ID NO: 3, as well as the amino acid sequences set forth in SEQ ID NOs: 4 to 6, respectively.

In addition, in this humanized antibody, from the viewpoint that the reactivity to an antigen is higher as shown in Examples described later, preferable examples include
(H2-L2) a humanized antibody wherein
the light chain variable region comprises one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids, and
   (H1-L4) a humanized antibody wherein
the light chain variable region comprises one amino acid sequence selected from an amino acid sequence set forth in SEQ ID NO: 8, an amino acid sequence having a homology of 80% or more with the amino acid sequence set forth in SEQ ID NO: 8, and an amino acid sequence derived from at least one of the amino acid sequence set forth in SEQ ID NO: 8 by substitution, deletion, addition, and/or insertion of one or more amino acids, and
the heavy chain variable region comprises one amino acid sequence selected from an amino acid sequence set forth in SEQ ID NO: 15, an amino acid sequence having a homology of 80% or more with the amino acid sequence set forth in SEQ ID NO: 15, and an amino acid sequence derived from at least one of the amino acid sequence set forth in SEQ ID NO: 15 by substitution, deletion, addition, and/or insertion of one or more amino acids, and
   (H2-L3) a humanized antibody wherein
the light chain variable region comprises one amino acid sequence selected from an amino acid sequence set forth in SEQ ID NO: 10, an amino acid sequence having a homology of 80% or more with the amino acid sequence set forth in SEQ ID NO: 10, and an amino acid sequence derived from at least one of the amino acid sequence set forth in SEQ ID NO: 10 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids, and
   (H2-L4) a humanized antibody wherein
the light chain variable region comprises one amino acid sequence selected from an amino acid sequence set forth in SEQ ID NO: 8, an amino acid sequence having a homology of 80% or more with the amino acid sequence set forth in SEQ ID NO: 8, and an amino acid sequence derived from at least one of the amino acid sequence set forth in SEQ ID NO: 8 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of sequences the amino acid sequence set forth in SEQ ID NO: 13 by substitution, deletion, addition, and/or insertion of. one or more amino acids

In addition, in consideration of the degree of production capacity, preferable examples include (H2-L2) a humanized antibody wherein
the light chain variable region comprises one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids,
   (H2-L4) a humanized antibody wherein
the light chain variable region comprises one amino acid sequence selected from an amino acid sequence set forth in SEQ ID NO: 8, an amino acid sequence having a homology of 80% or more with the amino acid sequence set forth in SEQ ID NO: 8, and an amino acid sequence derived from at least one of the amino acid sequence set forth in SEQ ID NO: 8 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids, and
   (H3-L4) a humanized antibody wherein
the light chain variable region comprises one amino acid sequence selected from an amino acid sequence set forth in SEQ ID NO: 8, an amino acid sequence having a homology of 80% or more with the amino acid sequence set forth in SEQ ID NO: 8, and an amino acid sequence derived from at least one of the amino acid sequence set forth in SEQ ID NO: 8 by substitution, deletion, addition, and/or insertion of one or more amino acids, and
the heavy chain variable region comprises one amino acid sequence selected from an amino acid sequence set forth in SEQ ID NO: 16, an amino acid sequence having a homology of 80% or more with the amino acid sequence set forth in SEQ ID NO: 16, and an amino acid sequence derived from at least one of the amino acid sequence set forth in SEQ ID NO: 16 by substitution, deletion, addition, and/or insertion of one or more amino acids.

Moreover, the antibody of the present invention can take an aspect of an antibody which competes, in binding to the human Eval, with an antibody having variable regions or CDRs which contains the above-mentioned specific amino acid sequences or modifications thereof. In other words, the antibody of the present invention can take an aspect of an antibody binding to an epitope to which an antibody having a variable region or CDR which contains the above-mentioned specific amino acid sequences or modifications thereof exhibits reactivity.

In the present invention, the "epitope" means an antigenic determinant present in an antigen, that is, a site on the antigen to which an antigen binding domain in an antibody binds. Thus, the epitope in the present invention may be a polypeptide (linear epitope) having multiple consecutive amino acids in a primary sequence of amino acids, or may be a polypeptide (discontinuous epitope, conformational epitope) of amino acids which are not adjacent to each other in a primary sequence of amino acids but which come closer to each other in a three-dimensional conformation by folding or the like of a peptide or protein. In addition, such an epitope typically has at least 3 (for example, 4) amino acids, and most usually 5 (for example, 6 to 20, 7 to 15, 8 to 10) amino acids.

In addition, in the case where an antibody is obtained, a person skilled in the art can specify a peptide region (epitope) on an antigen to which the antibody exhibits reactivity and prepare various antibodies binding to the peptide region. Moreover, whether two antibodies bind to the same epitope or sterically overlapping epitopes can be determined by the competitive assay method.

In the present invention, the "functional fragment" of the antibody means a part (partial fragment) of the antibody, which exhibits reactivity to the human Eval. Specifically, the functional fragment includes Fab, Fab', F(ab')2, a variable region fragment (Fv), a disulfide bonded Fv, a single-chain variable fragment (single chain Fv, scFv), sc(Fv)2, a diabody, a polyspecific antibody, polymers of these, and the like.

Here, "Fab" means a monovalent antigen-binding fragment of an immunoglobulin, composed of a part of one light chain and a part of one heavy chain. Fab can be obtained by papain digestion of an antibody or by a recombinant method. "Fab‴ is different from Fab in that a small number of residues, including one or more cysteines in an antibody hinge region, are added to the carboxy terminus of a heavy chain CH1 domain. "F(ab') 2" means a bivalent antigen-binding fragment of an immunoglobulin, composed of parts of two light chains and parts of two heavy chains.

The "variable region fragment (Fv)" is the smallest antibody fragment having complete antigen recognition and binding sites. Fv is a dimer in which a heavy chain variable region and a light chain variable region are strongly linked by a non-covalent bond. The "single-chain variable fragment (single chain Fv, scFv)" contains a heavy chain variable region and a light chain variable region of an antibody, and these regions are present in a single polypeptide chain. "sc(Fv)2" is a fragment obtained by binding two heavy chain variable regions and two light chain variable regions with a linker or the like into a single-chain. The "diabody" is a small antibody fragment having two antigen-binding sites, and this fragment contains a heavy chain variable region linked to a light chain variable region in a single polypeptide chain, and each region forms a pair with a complementary region of another chain. The "polyspecific antibody" is a monoclonal antibody having binding specificities to at least two different antigens. For example, a polyspecific antibody can be prepared by co-expressing two immunoglobulin heavy chain/light chain pairs in which two heavy chains have different specificities.

The antibody of the present invention can be prepared by a hybridoma method, or can be prepared by a recombinant DNA method. The hybridoma method is represented by a method of Kohler and Milstein (Kohler & Milstein, Nature, 256: 495 (1975)). An antibody-producing cell used in the cell fusion process in this method is a spleen cell, a lymph node cell, a peripheral blood leucocyte, or the like of an animal (for example, mouse, rat, hamster, rabbit, monkey, or goat) immunized with an antigen (a human Eval, a partial peptide thereof, a protein in which an Fc protein or the like is fused to these, or cells expressing these, or the like). Antibody-producing cells obtained by reacting, in a medium, an antigen with the above-described cells, lymphocytes, or the like isolated in advance from a not-immunized animal can also be used. As the myeloma cells, various known cell lines can be used. The antibody-producing cells and myeloma cells may be originated from different animal species as long as these cells can be fused, but are preferably originated from the same animal species. A hybridoma is produced, for example, by a cell fusion from a spleen cell obtained from a mouse immunized with an antigen and a mouse myeloma cell, and a hybridoma which produces a monoclonal antibody specific to the human Eval can be obtained by the subsequent screening. A monoclonal antibody to the human Eval can be obtained by culturing the hybridoma, or from the ascitic fluid of a mammal administered with the hybridoma.

The recombinant DNA method is a method including cloning a DNA encoding the above-described antibody of the present invention from a hybridoma, B cells, or the like, incorporating the clone into an appropriate vector, introducing this vector into host cells (for example, a mammalian cell line such as HEK cells, E. coli, yeast cells, insect cells, plant cells, or the like), and producing the antibody of the present invention as a recombinant antibody (for example, P. J. Delves, Antibody Production: Essential Techniques, 1997 WILEY, P. Shepherd and C. Dean Monoclonal Antibodies, 2000 OXFORD UNIVERSITY PRESS, Vandamme A. M. et al., Eur. J. Biochem. 192: 767-775 (1990)). For the expression of a DNA encoding the antibody of the present invention, DNAs encoding the heavy chain or the light chain may be separately incorporated into expression vectors to transform host cells, or DNAs encoding the heavy chain and the light chain may be incorporated into a single expression vector to transform host cells (see International Publication No. WO94/11523) . The antibody of the present invention can be obtained in a substantially pure and homogeneous form by culturing the above-described host cells and separating and purifying the antibody from the host cells or a culture liquid. For the separation·purification of the antibody, a normal method used in purification of polypeptides can be used. Once a transgenic animal (cattle, goat, sheep, pig, or the like) incorporating the antibody gene is prepared by using a transgenic animal preparation technique, a large amount of the monoclonal antibody derived from the antibody gene can also be obtained from milk of the transgenic animal.

The present invention can also provide: a DNA encoding the above-described antibody of the present invention, a vector comprising the DNA; a host cell comprising the DNA; and a method for producing an antibody, comprising culturing the host cell and recovering the antibody.

### <Antibody-drug Conjugate>

As shown in Examples described later, the human Eval which is the target of the antibody of the present invention is expressed in various cancers. In addition, the antibody (particularly, the humanized antibody) of the present invention is also useful as ADC. Hence, the present invention can take an aspect of an antibody-drug conjugate (ADC) obtained by binding the antibody (particularly, the humanized antibody) of the present invention or a functional fragment thereof and a drug.

In the ADC of the present invention, the "drug" which can be bound to the antibody is not particularly limited but includes, for example, cytotoxic substances known in the technical field. The "cytotoxic substances" include anticancer agents, for example, alkylating agents such as irinotecan (CPT-11), irinotecan metabolite SN-38 (10-hydroxy-7-ethyl camptothecin), adriamycin, Taxol, 5-fluorouracil, nimustine, ranimustine, and temozolomide, metabolic antagonists such as gemcitabine and hydroxycarbamide, plant alkaloids such as etoposide and vincristine, anticancer antibiotics such as mitomycin and bleomycin, platinum preparations such as cisplatin, molecular target agents such as sorafenib and erlotinib, methotrexate, cytosine arabinoside, 6-thioguanine, 6-mercaptopurine, cyclophosphamide, ifosfamide, busulfan, MMAE (monomethyl auristatin E), DM1 (mertansine), and calicheamicins. The cytotoxic substances also include radioisotopes, for example, ³²P, ¹⁴C, ¹²⁵I, ³H, ¹³¹I, ¹⁸⁶Re, ¹⁸⁸Re, ¹⁰B, ¹¹¹In, and ⁹⁰Y. The cytotoxic substance may be a photosensitive substance which activates cytotoxic activity, more specifically, a substance which turns into a form exhibiting cytotoxicity (cytotoxic activity) by itself by being activated through light irradiation, or may be a substance which generates a cytotoxic substance. Such cytotoxic substances include, for example, chlorines, chlorine e6, porfimer sodium, talaporfin sodium, verteporfin, and precursors and derivatives of these. In addition, the cytotoxic substances include toxic peptides, for example, ribosome-inactivating proteins (RIPs) such as saporin, ricin, and Shiga toxin.

In addition, the antibody and a cytotoxic substance can be bound by a method known in the technical field, which may be either of direct binding and indirect binding. For example, as direct binding, a covalent bond can be utilized. As indirect binding, binding through a linker can be utilized.

In the present invention, it is preferable that the antibody and a cytotoxic substance be bound through a linker. As the two molecules bind through a linker, the antigenicity of the cytotoxic substance can be reduced, and such binding is preferable in administration to a subject. A general technique regarding linkers is described in, for example, Hermanson, G. T. Bioconjugate Techniques, Academic Press, 1996; Harris, J. M. and Zalipsky, S. Eds, Poly (ethylene glycol), Chemistry and Biological Applications, ACS Symposium Series, 1997; Veronese, F. and Harris, J. M. Eds, Peptide and protein PEGylation. Advanced Drug Delivery Review 54 (4), 2002.

In addition, the linker includes not only linear linkers (divalent linkers) but also branched linkers (trivalent or higher-valent linkers). Linear linkers have a cytotoxic substance at one end and an antibody at the other end. Branched linkers normally have a cytotoxic substance at each branch (each chain) and has an antibody at the other end. Such linkers include, for example, peptide linkers such as N-succinimidyl 4-(maleimidomethyl)cyclohexanecarboxylate (SMCC), sulfosuccinimidyl-4-(N-maleimidomethyl)cyclohexane-1-carboxylate (sulfo-SMCC), N-succinimidyl-4-(N-maleimidomethyl)-cyclohexane-1-carboxy-(6-amidocaproate) (LC-SMCC), N-succinimidyl-3-(2-pyridyldithio)butyrate (SPDB), N-succinimidyl-3-(2-pyridyldithio)propionate (SPDP), 4-maleimidobutyric acid N-hydroxysuccinimide ester (GMBS), 3-maleimidocaproic acid N-hydroxysuccinimide ester (EMCS), m-maleimidobenzoyl-N-hydroxysuccinimide ester (MBS), N-(α-maleimidoacetoxy)-succinimide ester (AMAS), succinimidyl-6-(β-maleimidopropionamido)hexanoate (SMPH), N-succinimidyl-4-(p-maleimidophenyl)-butyrate (SMPB), N-(p-maleimidophenyl)isocyanate (PMPI), 6-maleimidocaproyl (MC), maleimidopropanoyl (MP), p-aminobenzyloxycarbonyl (PAB), N-succinimidyl-4-(2-pyridylthio)pentanoate (SPP), N-succinimidyl (4-iodoacetyl)aminobenzoate (SIAB), valine-citrulline (Val-Cit), and alanine-phenylalanine (ala-phe) .

The technique of binding an antibody and a cytotoxic substance through a linker is known in the technical field. For example, binding of an antibody and a linker is a covalent bond or a non-covalent bond (an ionic bond, a hydrophobic bond, or the like), and is preferably a covalent bond. This bond is preferably a bond from which a cytotoxic substance is not readily liberated in the blood when the conjugate of the present invention is administered to a subject. Such bonds include a bond between a maleimide group and a thiol group, a bond obtained by reacting a halo ester and a thiol, an amide bond between a carboxyl group and an amino group, a disulfide bond between a thiol group and a thiol group, a Schiff base from an amino group and an aldehyde group, a thio ester bond between a thiol group and a carboxylic acid, an ester bond between a hydroxyl group and a carboxyl group, a bond based on an amino group and a squaric acid derivative (for example, a dimethyl squarate), a bond between a dienyl aldehyde group and an amino group, and the like. Specific examples of such bonds include a bond between a maleimide group present at one end of a linker and a thiol group contained in a cysteine residue on an antibody, a bond formed by dehydrative substitution between a succinimide group present at one end of a linker and an amino group contained in a lysine residue on an antibody (for example, International Publication No. WO2008/096760), a bond formed by dehydration condensation between an amino group present at one end of a linker and a carboxylic acid contained in an aspartic acid or a glutamic acid on an antibody (for example, WSCDI is used), and the like. For a specific method for bonding an antibody and a linker, see, for example, International Publication No. WO2010/055950.

On the other hand, bonding of an antibody or a linker and a cytotoxic substance is a covalent bond or a non-covalent bond (an ionic bond, a hydrophobic bond, or the like), and is preferably a covalent bond. Particularly, this bond is preferably a bond from which a cytotoxic substance is not readily liberated in the blood when the conjugate of the present invention is administered to a subject. From such viewpoint, although not limited, the bond between the antibody or a linker and a cytotoxic substance is preferably an ester bond, a carbamate bond, a carbonate bond, or a thio carbamate bond, and more preferably an ester bond.

In the ADC of the present invention, the number of bonds of the cytotoxic substance per molecule of the antibody (the number of conjugated drugs) is determined by specifying reaction conditions such as the amounts of the raw materials·reagents to be reacted such that the number becomes a constant number in consideration of efficacy and safety thereof. However, unlike a chemical reaction of low-molecular-weight compounds, a mixture in which different numbers of drugs are bound is normally obtained. The number of drugs bound per molecule of the antibody is specified and described as an average value, that is, an average number of conjugated drugs. Hence, in the Specification of the present application as well, the number of conjugated drugs means an average value unless otherwise noted.

The number of conjugated drugs per molecule of the antibody can be controlled by adjusting the type and concentration of a reducing agent to be used, the concentrations (molar excesses or the like) of the linker and the drug to the antibody, and the temperature and time in conjugation reaction. The "reducing agent" is not particularly limited as long as the reducing agent can reductively cleave disulfide bonds in the antibody and generate conjugation via the above-described thiol group, but includes, for example, dithiothreitol (DTT, DL-DTT), 2-mercaptoethylamine, mercaptoethanol, and TCEP.

In the present invention, the average number of conjugated drugs per antibody is not particularly limited, but is, for example 1 to 10, preferably 3 to 8, and more preferably 3 to 5. Note that the number of conjugated drugs per molecule of the antibody can be obtained by measuring an absorbance at the peak of absorption of the cytotoxic substance (for example, DM1) and absorbance at 280 nm which is the peak of absorption of the antibody, formulating and calculating simultaneous equations with their influences on the respective absorption peaks taken into account to obtain the concentrations of the antibody and the cytotoxic substance, and determining the ratio of numbers of molecules as shown in Examples described below. Alternatively, the number of conjugated drugs per molecule of the antibody can be obtained by using a reagent which colorimetrically determining a thiol group such as DTNB.

In addition, there are also cases where when the ADC of the present invention is left in the atmosphere or recrystallized to absorb water content, the ADC contains adsorbed water or becomes a hydrate, and such a compound and a salt containing water is also encompassed in the present invention.

The ADC of the present invention may contain atomic isotopes of one or more atoms constituting the ADC at a non-natural ratio. The atomic isotopes include, for example, ²H, ³H, ¹²⁵I, and ¹⁴C. In addition, the ADC of the present invention can be detected by a bioimaging technique or the like. Hence, a labeling substance may be bound to the ADC of the present invention. The labeling substance can be selected as appropriate in accordance with the type of the detection method or the like to be used, but includes, for example, radioactive labeling substances, fluorescent labeling substances, paramagnetic labeling substances, superparamagnetic labeling substances, and enzyme labeling substances. In addition, binding of such a labeling substance and the ADC may be direct, or may be indirect. Examples of the indirect binding include bindings utilizing a secondary antibody to which a labeling substance is bound, or a polymer (such as protein A, protein B) to which a labeling substance is bound.

More specifically, as a radioisotope for labeling the ADC, a positron-emitting radionuclide can be utilized. For example, the antibody can be labeled by using a positron-emitting radionuclide such as ⁶⁴Cu, ¹⁸F, ⁵⁵Co, ⁶⁶Ga, ⁶⁸Ga, ⁷⁶Br, ⁸⁹Zr, and ¹²⁴I. For labeling the ADC using these positron-emitting radionuclides, a known method (Nucl Med Biol. 1999; 26 (8) : 943-50., J Nucl Med. 2013; 54 (11): 1869-75, or the like) can be utilized. In addition, after the ADC labeled with a positron-emitting radionuclide is administered to a subject, a radioactive ray emitted from the radionuclide can be measured with a PET (positron emission tomography device) and converted into an image by computer tomography. In this way, the reaching to a target site, accumulation, and the like of the ADC administered to the subject can be followed.

### <Chimeric Antigen Receptor>

As shown in Examples described later, the human Eval, which is the target of the antibody of the present invention, is expressed in various cancers. In addition, the variable regions of the antibody of the present invention are useful in a chimeric antigen receptor (CAR) and T-cells expressing the chimeric antigen receptor (CAR) (so-called CAR-T). Thus, the present invention can also take an aspect of a CAR, comprising a region binding to a human-derived Eval protein, a transmembrane region, and an intracellular signal region.

In the present invention, the "chimeric antigen receptor (CAR)" means a chimeric protein in which a region binding to a human-derived Eval protein, which is an extracellular region, a transmembrane region, and an intracellular signal region are arranged in this order from the N terminus side and directly or indirectly linked.

In the present invention, as the "region (an antigen-binding region) binding to a human-derived Eval protein", for example, the above-mentioned antibody or a functional fragment thereof can be used, but scFv is normally used for the CAR.

As mentioned above, "scFv" has a structure in which a light chain variable region and a heavy chain variable region of a monoclonal antibody (immunoglobulin) are linked by a linker and has a binding capacity with an antigen. The order on the N terminus side in scFv may be a light chain variable region, a linker, and a heavy chain variable region, or may be a heavy chain variable region, a linker, and a light chain variable region, but is preferably the former.

As the "linker" in scFv, a peptide linker can be used, for example. The length of the linker is not particularly limited. For example, a linker in which the number of amino acids is 5 to 25 can be used. The length of the linker is preferably 8 to 25 amino acids, and further preferably 15 to 20 amino acids. Preferable examples of a peptide linker include a peptide linker composed of glycine or glycine and serine (GGS linker, GS linker, GGG linker, and the like). In addition, preferable examples also include linkers composed of 18 amino acids at positions 134 to 151 set forth in SEQ ID NO: 58. Glycine and serine, which are amino acids constituting these linkers, have small sizes, and have advantages that a high-order structure is unlikely to be formed in a linker.

A monoclonal antibody from which scFv is obtained is not particularly limited, but includes, for example, the above-mentioned antibodies derived from a non-human animal, human antibodies, and humanized antibodies. In addition, preferable examples are also as described above, as shown in Examples described later, the above-described combination of (H2-L2) is preferable, and scFv composed of L2, linker, and H2 linked in this order from the N-terminus side is further preferable, from the viewpoint of activation and cell growth, and the like in accordance with a human Eval.

In the present invention, the "transmembrane region" is not particularly limited as long as the "transmembrane region" is a polypeptide which has a function of penetrating a cell membrane and is capable of tethering a CAR to the cell membrane. The protein from which the polypeptide serving as the transmembrane region is originated includes, for example, CD28, CD3ζ, CD3ε, 4-1BB, CD45, CD4, CD5, CD8α, CD8β, CD9, CD16, CD22, CD33, CD37, CD64, CD80, CD86, CD134, CD137, ICOS, CD154, GITR, and the α chain and the β chain of a T-cell receptor.

In the present invention, the "intracellular signal region" is a region which is disposed in a cell when a CAR is disposed on a cell membrane, and is a region capable of transferring a signal necessary for immune cells to exhibit the effector function, that is, transferring a signal (so-called a primary signal) necessary for activation of immune cells when the antigen-binding region binds to an antigen (human Eval). Such intracellular signal regions generally include, in addition to those having only activation signaling domains such as T-cell receptor (TCR) and CD3 conjugates (first-generation CARs), those having co-stimulatory signaling domains (for example, intracellular domains of CD28 or 4-IBB) derived from a co-stimulatory molecule in addition to activation signaling domains (second-generation CARs), and those having multiple co-stimulatory signaling domains (for example, intracellular domains of CD28 and 4-IBB) in addition to activation signaling domains (third-generation CARs). In addition, there are those containing a CD79A intracellular region and a CD40 intracellular region, which the present inventors developed before (see Mol Ther 2021 Sep 1; 29 (9): 2677-2690.).

The "intracellular signal region" only has to be capable of transferring a primary signal as described above, and an activation signaling domain of a protein involved in the signal can be used. It is known that an immunoreceptor tyrosine-based activation motif (ITAM) is involved in the primary signaling. Proteins having ITAM include, for example, CD3ζ, FcRγ, FcRβ, CD3γ, CD3δ, CD3ε, CD5, CD22, CD79a, CD79b, CD66d, DAP10, and DAP12.

In addition, as described above, and also shown in Examples described later, "those containing a CD79A intracellular region and a CD40 intracellular region" can be favorably used as the intracellular signal region according to the present invention. In such a case, the arrangement of the CD79A intracellular domain and the CD40 intracellular domain is not particularly limited, and both an aspect in which the CD79A intracellular region is on the N terminus side and an aspect in which the CD40 intracellular region is on the N terminus side can be employed. The former arrangement is preferable. The CD79A intracellular region and the CD40 intracellular region are preferably directly linked or linked via a linker. The linker is not particularly limited, and any linker can be employed as long as the NFκB signal-activating effect is not significantly impaired. The linker is preferably a linker composed of glycine or glycine and serine (GGS linker, GS linker, GGG linker, or the like). The length of the linker is not particularly limited, but is for example 1 to 20 amino acids, preferably 1 to 10 amino acids, and more preferably 1 to 5 amino acids.

In the present invention, the number of intracellular signal regions contained in the CAR is not limited to one, and the CAR can contain multiple intracellular signal regions (for example, can contain 1 to 5, 1 to 3, or 1 or 2 signaling factors). In this case, the multiple intracellular signal regions contained may be the same or may be different.

The CAR of the present invention may contain another domain in addition to the above-mentioned antigen-binding region, transmembrane region, and intracellular signal region. Examples of the other domain include a co-stimulatory region, a spacer sequence, a signal peptide, and the like but are not limited to these.

It is known that co-stimulatory molecules expressed on T-cells transfer co-stimulatory signals into cells by binding to a ligand specific to each co-stimulatory molecule expressed on antigen-presenting cells to assist activation of the T-cells (secondary signaling). In the present invention, the "co-stimulatory region" means an intracellular domain involved in transfer of co-stimulatory signals of co-stimulatory molecules as described above. Examples of the co-stimulatory molecules include CD28, 4-1BB (CD137), OX40 (CD134), ICOS, CD2, CD4, CD5, CD8α, CD8β, CD154, and the like. The CAR of the present invention may contain co-stimulatory regions of these co-stimulatory molecules.

The number of co-stimulatory regions which the CAR of the present invention can contain is not limited to one, and the CAR may contain multiple co-stimulatory regions. The CAR of the present invention can contain, for example, 1 to 5, 1 to 3, or 1 or 2 co-stimulatory regions. In the case where the CAR of the present invention contains multiple co-stimulatory signaling regions, the regions may be the same or may be different.

In the present invention, the "spacer" means a sequence linking the regions and the like, and is not particularly limited as long as the spacer does not suppress the function of each region. In addition, the number of amino acids of the spacer is not particularly limited, but is normally 1 to 500 amino acids, preferably 5 to 300 amino acids, and further preferably 10 to 100 amino acids.

In particular, in the CAR of the present invention, it is preferable that the antigen-binding region and the transmembrane region be linked directly or via a relatively short spacer. The number of amino acids constituting the spacer between the antigen-binding region and the transmembrane region is for example 1 to 400, and preferably 10 to 300. In addition, as shown in Examples described later, in the case of culturing cells expressing the CAR of the present invention in the absence of IL-2, the number of constituting amino acids is more preferably 150 to 300, and further preferably 200 to 250, from the viewpoint of cell growth and cytokine production. On the other hand, in the case of culturing cells expressing the CAR of the present invention in the presence of IL-2, the number of constituting amino acids is more preferably 10 to 30.

The sequence of the spacer is not particularly limited, but for example, the sequence of the hinge part of IgG or portion thereof, preferably the hinge part of human IgG (for example, subtype IgG1 or IgG4) or portion thereof, the hinge part and a portion of CH2, or a portion of a factor used for the transmembrane domain (for example CD28), or the like can be used as a spacer. Note that it can be expected that the use of the sequence of the hinge part or a portion thereof will form a flexible spacer. Such a spacer specifically includes a spacer composed of an amino acid sequence set forth in SEQ ID NO: 59, 60, 61, 62, or 62 (positions 268 to 499 set forth in SEQ ID NO: 58) .

In the present invention, the "signal peptide" means a peptide for facilitating the secretion of the CAR or instructing localization to a cell membrane, and is also called a leader sequence. A signal peptide can normally be bound to the N terminus of an antigen-binding region directly or indirectly. In addition, optionally, a signal peptide may be cleaved from an antigen-binding region during cell processing and localization of the CAR to a cell membrane. Examples of such a signal peptide include signal peptides such as human GM-CSF receptor, α chain and β chain of a T-cell receptor, CD8α, CD8β, CD3ζ, CD28, CD3ε, CD45, CD4, CD5, CD9, CD16, CD22, CD33, CD37, CD64, CD80, CD86, CD134, CD137, ICOS, CD154, and GITR.

Although examples of the regions and the like in the CAR of the present invention have been described above, a person skilled in the art can search for and obtain specific amino acid sequences regarding these regions and the like as appropriate from known documents and databases such as NCBI (http://www.ncbi.nlm.nih.gov/guide/). Moreover, as shown in the above Table 3 and the like, specific sequences are also disclosed in Sequence Listing of the present application. In addition, the regions and the like according to the present invention can encompass not only such typical amino acid sequences (for example, NCBI reference sequences) but also variants and homologs of these typical amino acid sequences as long as they maintain the functions achieved by the regions. In addition, such variants and homologs normally have high homology with the typical amino acid sequences. The high homology is normally 60% or more, preferably 70% or more, more preferably 80% or more, and further preferably 90% or more (for example, 95% or more, 96% or more, 97% or more, 98% or more, 99% or more).

In addition, the CAR of the present invention may be a molecule composed of one polypeptide or may be a molecule composed of a complex of two or more polypeptides. In addition, the CAR of the present invention may be a molecule composed of a polypeptide or a complex thereof, or may be a polypeptide or a complex thereof linked with another substance (for example, a fluorescent substance, a radioactive substance, an inorganic particles, or the like).

The CAR of the present invention may be chemically modified. The C terminus of the polypeptide constituting the CAR of the present invention may be any of a carboxyl group (-COOH), carboxylate (-COO-), amide (-CONH₂), or an ester (-COOR). Here, as R in the ester, a C1-6 alkyl group such as methyl, ethyl, n-propyl, isopropyl, or n-butyl, for example; a C3-8 cycloalkyl group such as cyclopentyl or cyclohexyl, for example;, a C6-12 aryl group such as phenyl or α-naphthyl, for example; a phenyl-C1-2 alkyl group such as benzyl or phenethyl, for example; a C7-14 aralkyl group such as α-naphthyl-C1-2 alkyl group including α-naphthylmethyl; a pivaloyloxymethyl group, or the like is used. The polypeptide constituting the CAR of the present invention may have an amidated or esterified carboxyl group (or carboxylate) other than the C terminus. As the ester in this case, an ester of the above-described C terminus or the like is used, for example. Moreover, the polypeptide constituting the CAR of the present invention encompasses that in which an amino group of an amino acid residue at the N terminus is protected with a protecting group (for example, a C1-6 acyl group such as a C1-6 alkanoyl including a formyl group, an acetyl group, or the like), that in which a glutamine residue at the N terminus produced by intravital dissection is pyroglutaminated, that in which a substituent (for example, -OH, -SH, an amino group, an imidazole group, an indole group, a guanidino group, or the like) on the side chain of an amino acid within molecules is protected with a proper protecting group (for example, a C1-6 acyl group such as a C1-6 alkanoyl group including a formyl group, an acetyl group, or the like).

In addition, the CAR of the present invention may have another functional protein. Another functional protein is not particularly limited, and may be selected as appropriate in accordance with a function desired to be imparted to the CAR of the present invention. For example, functional proteins used to facilitate the purification and detection of the CAR include Myc-tag, His-tag, HA-tag, FLAG-tag (registered trademark, Sigma-Aldrich), a fluorescent protein tag (GFP and the like), and the like.

The chimeric antigen receptor of the present invention may be in the form of a pharmaceutically acceptable salt with an acid or a base. The salt is not particularly limited as long as the salt is a pharmaceutically acceptable salt, and any of an acid salt and a basic salt can be employed. For example, examples of the acid salts include inorganic acid salts such as hydrochlorides, hydrobromides, sulfates, nitrates, and phosphate; organic acid salts such as acetates, propionates, tartrates, fumarates, maleates, malates, citrates, methanesulfonates, and para toluenesulfonates; amino acid salts such as aspartates and glutamates, and the like. In addition, examples of the basic salts include alkali metal salts such as sodium salts and potassium salts; alkaline earth metal salts such as calcium salts and magnesium salts, and the like.

The chimeric antigen receptor of the present invention may be in the form of a solvate. The solvent is not particularly limited as long as the solvent is pharmaceutically acceptable, and includes, for example, water, ethanol, glycerol, acetic acid, and the like.

Note that there are some reports of experiments, clinical studies, and the like utilizing CARs (for example, Rossig C, et al. Mol Ther 10: 5-18, 2004; Dotti G, et al. Hum Gene Ther 20: 1229-1239, 2009; Ngo MC, et al. Hum Mol Genet 20 (R1): R93-99, 2011; Ahmed N, et al. Mol Ther 17: 1779-1787, 2009; Pule MA, et al. Nat Med 14: 1264-1270, 2008; Louis CU, et al. Blood 118: 6050-6056, 2011; Kochenderfer JN, et al. Blood 116: 4099-4102, 2010; KochenderferJN, et al. Blood 119: 2709-2720, 2012; Porter DL, et al. N Engl J Med365: 725-733, 2011; Kalos M, et al. Sci Transl Med 3: 95ra73, 2011; Brentjens RJ, et al. Blood 118: 4817-4828, 2011; and Brentjens RJ, et al. SciTransl Med 5: 177 ra38, 2013). Hence, the CAR of the present invention can be constructed by referring to these reports. In addition, since specific sequences and the like are also shown in Examples described later, the CAR of the present invention can also be conducted by referring to these.

### <Nucleotide Encoding Chimeric Antigen Receptor>

The present invention provides a polynucleotide encoding the CAR of the present invention. Information of a nucleotide sequence encoding an antigen-binding region such as scFV can also be obtained as follows. A monoclonal antibody recognizing a target antigen is prepared, and an amino acid sequence of the monoclonal antibody is determined by a known method such as the Edman method. Then, the information can be obtained based on the amino acid sequence. Note that regarding the above-mentioned humanized antibodies and the like, specific sequences are also disclosed in Sequence Listing of the present application. In addition, such information can also be obtained by analyzing nucleotide sequences of hybridomas producing the monoclonal antibodies.

In addition, a person skilled in the art can search for and obtain specific nucleotide sequences encoding the regions and the like in the CAR of the present invention including the antigen-binding region as appropriate from known documents and databases such as NCBI (http://www.ncbi.nlm.nih.gov/guide/). Moreover, the nucleotide sequences encoding the above-described regions and the like are not limited to known ones, and any nucleotide sequences encoding the above-described regions and the like can be used. There are multiple codons corresponding to one amino acid due to degeneracy of genetic code. Hence, there are a large number of nucleotide sequences encoding the same amino acid sequence. The nucleotide sequence of the polynucleotide of the present invention may be any of multiple nucleotide sequences generated by degeneracy of genetic code as long as the nucleotide sequence encodes the CAR of the present invention. Moreover, in order to optimize the expression in cells expressing the CAR, a codon selected to encode the amino acid may be modified in the nucleotide sequences encoding the regions and the like in the CAR of the present invention.

Then, a person skilled in the art can prepare polynucleotides encoding the regions and the like by using a known technique such as a chemical synthesis method (a phosphoramidite method or the like) and an amplification method with PCR based on such nucleotide sequence information. Moreover, the polynucleotide encoding the CAR of the present invention can be obtained by linking the polynucleotides encoding the regions and the like obtained in this way directly or via a spacer. Note that the linking of the polynucleotides encoding the regions and the like can be conducted, for example, by using a known method such as overlap extension PCR method as shown in Examples described later.

### <Vector Comprising Polynucleotide Encoding Chimeric Antigen Receptor>

The present invention provides a vector comprising the above-mentioned polynucleotide. The vector of the present invention may be linear or cyclic, and includes, for example, viral vectors, plasmid vectors, episomal vectors, artificial chromosome vectors, and transposon vectors.

The viral vectors include, for example, retrovirus vectors such as lentivirus, Sendai virus vectors, adenovirus vectors, adeno-associated virus vectors, herpes virus vectors, vaccinia virus vectors, poxvirus vectors, polio virus vectors, sindbis virus vectors, rhabdovirus vectors, paramyxovirus vectors, and orthomyxovirus vectors.

The plasmid vectors include, for example, plasmid vectors for animal cells expression such as pcDNA 3.1, pA1-11, pXT1, pRc/CMV, pRc/RSV, and pcDNA I/Neo.

The episomal vectors are vectors capable of extrachromosomal autonomous replication. Specific approach using episomal vectors are known (see Yu et al., Science, 2009, 324, 797-801). Episomal vectors include, for example, vectors containing sequences which are necessary for autonomous replication and are derived from EBV, SV40, and the like as vector elements. Vector elements necessary for autonomous replication specifically include an origin of replication and a gene encoding a protein which binds to the origin of replication to control replication. For example, the vector elements include an origin of replication oriP and EBNA-1 gene in the case of EBV, and an origin of replication ori and SV40LT gene in the case of SV40.

The artificial chromosome vectors include, for example, YAC (Yeast artificial chromosome) vector, BAG (Bacterial artificial chromosome) vector, and PAC (P1-derived artificial chromosome) vector.

Among these vectors, the retrovirus vector is preferable from the viewpoint that genes can be easily and highly efficiently introduced even into cells which are slow to grow and stably expressing strains can be easily established.

In addition to the above-mentioned polynucleotide encoding the CAR, the vector of the present invention may contain expression control sequences such as a promoter, an enhancer, a poly-A addition signal, and a terminator, a nucleotide sequence encoding an origin of replication and a protein which binds to the origin of replication to control replication, a nucleotide encoding another protein, and the like.

By arranging the above-mentioned polynucleotide encoding the CAR and a nucleotide encoding another protein, which will be describe later, operably downstream of a promoter, it becomes possible to efficiently transfer each polynucleotide. Such "promoter" includes, for example, CMV (cytomegalovirus) promoter, SRα promoter, SV40 early promoter, LTR of retrovirus, RSV (Rous sarcoma virus) promoter, HSV-TK (herpes simplex virus thymidine kinase) promoter, EF1α promoter, metallothionein promoter, heat shock promoter, and the like.

The "nucleotide encoding another protein" includes, for example, marker genes such as fluorescent protein genes, reporter genes, and drug-resistant genes, genes encoding molecules involved in T-cell activation such as cytokines, and the like. In addition, it becomes possible to express the other protein polycistronically with the above-mentioned CAR, for example, by using DNA encoding IRES, 2A peptide sequence (for example, 2A peptide (T2A) derived from Thosea asigna), or the like.

Moreover, in the body of a subject (a cancer patient or the like) to which the cells expressing the CAR have been administered, in order to induce apoptosis of the administered CAR expressing cells as appropriate, the vector of the present invention can contain a suicide gene as the "nucleotide encoding another protein". The suicide gene includes, for example, herpes simplex virus thymidine kinase (HSV-TK) and inducible caspase 9 (iCasp9). In addition, drugs that activate the function of such genes include ganciclovir for HSV-TK and AP1903, which is a chemical inducer of dimerization (CID), for iCasp9, and the like (see Cooper LJ. et al., Cytotherapy. 2006; 8 (2): 105-17, Jensen M.C. et al., Biol Blood Marrow Transplant. 2010 Sep; 16 (9): 1245-56, JonesBS., Front Pharmacol. 2014 Nov 27; 5: 254., Minagawa K., Pharmaceuticals (Basel). 2015 May 8; 8 (2): 230-49, and Bole-Richard E., Front Pharmacol. 2015 Aug 25; 6: 174).

### <Cell Expressing Chimeric Antigen Receptor>

The present invention provides a cell expressing the CAR of the present invention. The cell of the present invention can be obtained by introducing the above-mentioned polynucleotide or vector encoding the CAR of the present invention into a cell.

The "cell" into which the polynucleotide or vector of the present invention is introduced is preferably a cell derived from a mammal, and for example, a cell derived from human or a cell derived from a non-human mammal such as a rodent (a mouse, a rat, or the like), cattle, sheep, a horse, a dog, a pig, or a monkey can be used. The type of the cell is not particularly limited, and a cell or the like collected from a body fluid such as blood or bone marrow fluid, a tissue of the spleen, thymus gland, lymph node, or the like, a tumor, cancerous ascites, or the like can be used. A preferable example includes an immune cell as the cell for CAR expression.

The "immune cell" includes CD4 positive CD8 negative T-cells, CD4 negative CD8 positive T-cells, T-cells prepared from iPS cells, αβ-T-cells, γδ-T-cells, Nκ cells, Nκ T-cells, and the like. Various cell populations can be used as long as they contain lymphocytes or precursor cells as described above can be used. PBMCs (peripheral blood mononuclear cells) which are collected from peripheral blood are some of the preferable immune cells. That is, in a preferable one aspect, a gene transfer operation is conducted on PBMCs. PBMCs can be prepared in accordance with or in compliance with a conventional method.

It is preferable to activate cells for expressing the CAR before introduction of the polynucleotide of the present invention. For example, the cells for expressing the CAR can be activated by stimulating the cells with an anti-CD3 antibody and an anti-CD28 antibody. More specifically, the cells can be stimulated with an anti-CD3 antibody and an anti-CD28 antibody by culturing the cells in a culture vessel (for example, a culture plate) coated with an anti-CD3 antibody and an anti-CD28 antibody on the culturing surface. This stimulation can also be conducted by utilizing magnetic beads coated with an anti-CD3 antibody and an anti-CD28 antibody (for example, Dynabeads T-Activator CD3/CD28 provided by VERITAS).

In order to improve the survival rate/growth rate of the cells, it is preferable to use a culture liquid containing a T-cell growth factor in the activation treatment. As the T-cell growth factor, IL-2, IL-15, IL-7, or the like can be used. A T-cell growth factor such as IL-2, IL-15, or IL-7 can be prepared in accordance with a conventional method. Alternatively, a commercial product may also be used. Although the use of a T-cell growth factor of an animal species other than human is not excluded, a T-cell growth factor derived from human (which may be a recombinant) is normally used.

Typically, for its application (administration to a subject), cells after introducing the polynucleotide of the present invention (polynucleotide-introduced lymphocytes of the present invention) are grown. For example, the polynucleotide-introduced lymphocyte of the present invention is cultured (and subcultured as necessary) by using a culture liquid containing a T-cell growth factor. In addition to this culturing, the same treatment (reactivation) as in the case of the cells for expressing the CAR may also be conducted.

Note that a medium containing serum (human serum, fetal bovine serum, or the like) may be used in culturing the cells of the present invention. However, employing a serum-free medium makes it possible to prepare cells having advantages of high safety in clinical application and less difference in the culture efficiency due to differences among serum lots. In the case of using serum, it is preferable to use self-serum, that is, serum collected from the subject who is to be administered with the cells of the present invention.

In addition, the cells of the present invention may be cells in which the function of a protein which suppress excessive immune response or the like (an immune checkpoint substance or the like) is suppressed. Such a protein includes, for example, Programmed Death 1 (PD1), PD-L1, PD-L2, CTLA4, TIM3, CEACAM (CEACAM-1, CEACAM-3, CEACAM-5, or the like), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, and GAL9.

In addition, in order to facilitate allotransplantation, the cells of the present invention may be cells in which the function of an endogenous protein such as TCR or HLA is suppressed.

The method for suppressing a function of a protein includes a knock-out method targeting a gene encoding the protein, a method using dsRNA (double-stranded RNA, for example, siRNA) complementary to a transcription product of the gene, a method using antisense RNA complementary to the transcription product, and a method using RNA having a ribozyme activity of specifically cleaving the transcription product. In addition, a genome editing method which is a method for modifying a target gene by utilizing a site-specific nuclease (for example, a DNA double-strand cleaving enzyme such as zinc finger nuclease (ZFN), transcription activator-like effector nuclease (TALEN), or CRISPR-Cas9) is also favorably used for suppressing a function of the above-described substance.

The method for introducing the polynucleotide or the vector of the present invention into cells includes a lipofection method, a microinjection method, a calcium phosphate method, a DEAE-dextran method, an electroporation method, a particle gun method, and the like. In addition, in the case where the vector of the present invention is a retrovirus vector, it is possible to select an appropriate packaging cell based on a LTR sequence and a packaging signal sequence which the vector has, and prepare retrovirus particles using this packaging cell. The packaging cell includes, for example, PG13, PA317, GP+E-86, GP+envAm-12, and Psi-Crip. In addition, 293 cells and 293 T-cells having transfection efficiency can also be used as packaging cells.

In addition, after the polynucleotide or the vector of the present invention is introduced into cells, the expression of the CAR of the cells can be checked by a known method such as flow cytometry, RT-PCR, northern blotting, western blotting, ELISA, or fluorescent immunostaining.

### <Pharmaceutical Composition>

The present invention also provides a pharmaceutical composition (for example, an anticancer agent) comprising a cell expressing the antibody, ADC or CAR of the present invention (hereinafter, also referred to simply as the "antibody and the like of the present invention") as an active ingredient, as well as a method for treating or preventing a disease related to Eva1 protein (for example, a cancer), comprising a step of administering a therapeutically and prophylactically effective amount of the antibody and the like of the present invention to a subject.

The disease related to an Eva1 protein, which is targeted by the antibody of the present invention only has to be a disease in which the expression of an Eva1 protein is involved in the onset, progress of symptoms, aggravation, or the like of the disease and includes, for example, cancers.

In addition, the "cancer" targeted by the antibody of the present invention only has to be a cancer in which at least a human Eva1 is expressed, and may be a solid tumor or leukemia, lymphoma, or the like as long as the protein is expressed. More specifically, the cancer includes gliomas (tumors arising from neural stem cells, neural precursor cells and neuroglial cells, for example, glioblastoma multiforme (GBM), astrocytoma, medulloblastoma, ependymoma, oligodendroglioma, choroid plexus papilloma, particularly anaplastic astrocytoma, anaplastic oligodendroastrocytoma, and anaplastic oligodendroglioma), pancreatic cancer, stomach cancer, esophageal cancer, colorectal cancer, cholangiocarcinoma, hepatocellular cancer, breast cancer, lung cancer, kidney cancer, urothelial cancer, testicular cancer, prostate cancer, head and neck cancer, thyroid cancer, skin cancer, cervical cancer, endometrial cancer, ovarian cancer, angioma, myosarcoma, myeloma, plasma cell cancer, and melanoma. In addition, such cancers may be primary cancers or metastatic cancers. Moreover, the cancer according to the present invention also includes cancer stem cells.

The "pharmaceutical composition" of the present invention can contain other pharmacologically acceptable components besides the antibody and the like of the present invention. Such other components include, for example, carriers, emulsifiers, humectants, pH buffering agents, media, excipients, disintegrants, buffering agents, tonicity agents, suspensions, solubilizers, soothing agents, stabilizers, preservatives, and antiseptics. More specifically, in the case of a liquid preparation for injection or the like, examples of other pharmacologically acceptable components include aqueous solutions (saline, water for injection, phosphate buffer solution, glucose aqueous solution, glycerol aqueous solution, and the like), aluminum hydroxide, and the like. In addition, in the case of a lyophilized preparation, examples of other pharmacologically acceptable components include sugars (mannitol, lactose, saccharose, and the like), albumin, and the like, but are not limited to these. Moreover, the pharmaceutical composition of the present invention may take an aspect of a kit in which the above-described component can be mixed before administration. In addition, in the case where the pharmaceutical composition of the present invention is used for injection, the pharmaceutical composition of the present invention can take a form in which the pharmaceutical composition of the present invention is introduced in a syringe.

The pharmaceutical composition of the present invention may contain only the antibody or the like of the present invention or may contain the antibody or the like and at least one other anticancer agent as the active ingredients. In addition, the antibody and the like of the present invention can be administered together with another anticancer agent. This can enhance the antitumor effect. another anticancer agent to be used for such purpose may be administered simultaneously, separately, or consecutively with the antibody or the like of the present invention, or these may be administered while the administration interval of each is changed. Such "anticancer agent" includes, for example, immune checkpoint inhibitors (such as anti-PD-1 antibodies), gemcitabine, S-1, erlotinib, 5-FU, temozolomide, leucovorin, irinotecan (CPT-11), oxaliplatin, Abraxane, carboplatin, paclitaxel, pemetrexed, sorafenib, vinblastine, LH-RH analogs (such as leuprorelin and goserelin), estramustine phosphate, estrogen antagonists (such as tamoxifen and raloxifene), aromatase inhibitors (such as anastrozole, letrozole, and exemestane), and drugs described in International Publication No. WO2003/038043, but is not limited as long as the drug has antitumor activity. Moreover, in the case of the pharmaceutical composition comprising the cells of the present invention, the pharmaceutical composition may contain dimethyl sulfoxide (DMSO), serum albumin, or the like for the purpose of protecting the cells, and an antibiotic or the like for the purpose of preventing bacteria from being mixed, as another component. Furthermore, the pharmaceutical composition may contain T-cell activating factors such as cytokines, growth factors, or steroids, vitamins, immunostimulants, or immune checkpoint inhibitors for the purpose of activation, growth, differentiation induction, or the like of the cells, and the cell of the present invention and these other components may be used in combination.

The method for administering the pharmaceutical composition varies depending on the type of a substance to be administered, the aspect of the composition, the age, body weight, gender, health status of the subject, and the like, but the pharmaceutical composition may be administered through administration route of any of parenteral administration (for example, intravenous administration, intraarterial administration, topical administration) and oral administration. The administration method is preferably parenteral administration, and more preferably intravenous administration. In addition, not systemic administration but topical administration may be conducted. Example of topical administration includes direct injection into target tissues, organs, and body parts.

The dose of the pharmaceutical composition can vary depending on the age, body weight, gender, health status of the subject, the degree of progress of symptoms, and a component of the pharmaceutical composition to be administered. In addition, the administration schedule is also adjusted as appropriate depending on these conditions, and the pharmaceutical composition may be administered in a single dose, or may be continuously or periodically administered multiple times; in general, in the case of intravenously administering an antibody, the dose is 0.1 to 1000 mg, and preferably 1 to 100 mg per kg body weight daily for adults. In addition, in the case of administering ADC, the dose is 0.001 to 1000 mg, and preferably 0.01 to 100 mg per kg body weight daily for adults in terms of the amount of the drug contained. The frequency of administration of the antibody or ADC may be, for example, once, or multiple times at intervals of once a day to a year (for example, every week, every 10 to 30 days, every January, every March to June, every half a year, or every year).

In addition, the dose of the cells may be 1×10³ to 1×10¹⁰ (preferably 1×10⁴ to 1×10⁹, and more preferably 1×10⁵ to 1×10⁸) as the number of the cells administered per kg body weight in one administration for adults. The administration interval may be, for example, every week, every 10 to 30 days, every January, every March to June, every year, or the like. In addition, since the cells of the present invention can autonomously grow in the body of an administration target, one administration only may be employed. In addition, the number of the cells of the present invention in the body may be monitored after the administration to determine the administration period in accordance with the result.

Note that in the case where the treatment target is the brain, the presence of the blood-brain barrier (BBB) often becomes problem. However, regarding an individual who is affected with glioblastoma multiforme (GBM) or the like, since BBB, which is present in the normal brain, is not formed in the blood vessels in the brain tumor where angiogenesis has occurred, the antibody can be delivered to the GBM by intravenous injection or the like.

In addition, in the case where BBB is functioning as well, the antibody of the present invention can be administered while the BBB is avoided. For example, there is a method including inserting a cannula or the like by a stereotactic surgery method and directly administering the antibody of the present invention into a glioma or the like through the cannula. In addition, there is a method including implanting a drug delivery system containing the antibody of the present invention into the brain (Gill et al., Nature Med. 9: 589-595 (2003) and the like). Moreover, there is a transfection of a BBB-straddling neuron by using a vector containing a gene encoding the antibody of the present invention (United States Patent Application Publication No. 2003/0083299 and the like).

In addition, besides the above-described method of avoiding BBB, a method for administration by adding or binding the antibody of the present invention to a brain barrier-permeable substance can be utilized. The brain barrier-permeable substance includes, for example, a liposome binding to antibody-binding fragment which binds to a receptor on a vascular endothelium of the BBB (United States Patent Application Publication No. 20020025313 and the like), low-density lipoprotein particles (United States Patent Application Publication No. 20040204354 and the like), apolipoprotein E (United States Patent Application Publication No. 20040131692 and the like), transferrin (United States Patent Application Publication No. 2003/0129186 and the like), and a glycoprotein composed of 29 amino acids derived from rabies virus (see Kumar et al., Nature, July 5, 2007, vol. 448, 39 to 43).

Moreover, it is also possible to deliver the antibody to a glioma or the like through BBB by administering the antibody of the present invention while controlling the activity of a receptor or a channel. Such method includes, for example, increasing the transmissivity of the blood-brain barrier using a glucocorticoid blocker (United States Patent Application Publication No. 2002/0065259, United States Patent Application Publication No. 2003/0162695, United States Patent Application Publication No. 2005/0124533, and the like), activating a potassium channel (United States Patent Application Publication No. 2005/0089473 and the like), inhibiting an ABC drug transporter (United States Patent Application No. 2003/0073713 and the like), and cationating the antibody of the present invention (United States Patent No. 5,004,697 and the like).

Although the administration methods have been described above with diseases in the brain such as gliomas and the like as the subjects above, the method of administering the antibody of the present invention in such diseases is not limited to these. In addition, for other diseases as well, it is possible for a person skilled in the art to appropriately select a known method suitable for such diseases and apply the antibody of the present invention to the affected site like the above-described diseases in the brain.

In the present invention, the "subject" to which the antibody and the like of the present invention is administered includes, for example, humans who are affected with the above-mentioned cancers and the like, humans who have a risk of relapse of the above-mentioned cancers and the like, and humans who would be affected with the above-mentioned cancers and the like. In addition, in the present invention, the "treatment" includes alleviating (ameliorating) characteristic symptoms or involving symptoms of a target disease such as a cancer, preventing or delaying the aggravation of the symptoms, and the like. The "prevention" includes preventing or delaying a disease (disorder) or the onset, expression, or relapse of the symptoms of the disease (disorder), and reducing the risk of the onset, expression, or relapse.

### Examples

Hereinafter, the present invention will be described in further detail based on Examples, but the present invention is not limited to Examples described below.

### <Example 1> Anti-human Eva1 Humanized Antibody

It was attempted to humanize an anti-human Eva1 mouse antibody (the B2E5-48 mouse antibody described in PTL 2) by using materials and methods shown below. Note that the respective amino acid sequences of the light chain variable region (VL) and the heavy chain variable region (VH) of the antibody are as shown in SEQ ID NOs: 21 and 27.

### (Materials and Methods)

### 1.1 Analysis of Variable Regions and Identification of CDRs

CDRs in the variable regions of the B2E5-48 mouse antibody were identified by using IMGT Domain Gap Align tool (http://www.imgt.org/3Dstructure-DB/cgi/DomainGapAlign.cgi) and the sequences of germ cells which most matched were analyzed.

### 1.2 Molecule Modeling

Regarding VL and VH, based on homologies with the crystalline structures of known antibodies, molecule models were constructed by using software in Absolute Antibody. The images were created by using PyMol.

### 1.3 Analysis of Sequences Having Risks

Regarding the sequence of the antibody, specific risks were analyzed based on the motifs of publicly disclosed proteins. The analysis was conducted by using a system constructed with Microsoft Excel (Absolute Antibody Sequence Liability Tool). In this software, risks were evaluated based on the motifs shown in the Table shown below. Note that in the Table shown below, X represents any amino acid other than proline.

**[Table 4]**

| **Risk** | **Liability** | **Motif** |
|---|---|---|
| **High** | **Glycosylation** | **NXS or NXT** |
| | **Free Cysteine** | **C** |
| | **Deamidation** | **NG, NS or QG** |
| | **Isomerization** | **DG, DP or DS** |
| **Medium** | **Deamidation** | **NH** |
| | **Hydrolysis** | **NP** |
| | **Cleavage** | **TS, KK, RK or KR** |
| | **Transglutaminase** | **LLQG** |
| | **Integrin binding** | **RGD or KGD** |
| | **Plastic binding** | **FHENSP or WXXW** |

### 1.4 Gene Synthesis and Cloning

As shown in Table 6 given below, 4 humanized heavy chains and 4 humanized light chains in total were designed. Each was synthesized separately and cloned into human IgG1 heavy chain expression vectors and human κ light chain expression vectors. In the transfection, all possible combinations of humanized sequences were made to prepare 16 types of humanized antibodies in total. In addition to these, a chimeric human IgG1 (obtained by substituting the constant regions of the B2E5-48 mouse antibody with those of the human IgG type 1) was prepared as a control.

More specifically, the DNA sequences encoding the heavy chain variable regions and the light chain variable regions were designed by adding appropriate restriction enzyme recognition sites to the 5' terminus and the 3' terminus so as to be cloned into the cloning vector and expression vector produced by Absolute Antibody. Moreover, for these DNA sequences, the codons were optimized in consideration of expression in human cells. Based on these sequences, chemical synthesis was conducted and the resultants were cloned into appropriate species and types of vectors produced by Absolute Antibody. Note that the preciseness of the sequences was checked by the Sanger Sequencing analyzing the raw data using DNA STAR Lasergene software. After this checking was conducted, plasmid DNA prep of appropriate size was conducted to prepare DNAs with high quality in an amount sufficient for transfection.

### 1.5 Expression and Purification

HEK293T-cells were passaged to the stage most appropriate for transient transfection. Vectors for expression of heavy chains and light chains were introduced into the cells, followed by culturing for 6 days. The cultures were centrifuged at 4000 rpm, and culture supernatants of these were collected and filtered with a filter of 0.22 M. The purification at the first stage was conducted with protein A affinity chromatography, where elution was conducted with a buffer solution of citric acid having pH of 3.0, and neutralization was then conducted with 0.5M Tris having pH of 9.0. The proteins thus eluted were buffer-exchanged into PBS by using a desalting column. The concentrations of antibodies were measured by a UV spectroscopy method and the antibodies were concentrated as necessary. The chimeric human IgG1 was expressed and prepared not only in the HEK293T-cells but also in CHO cells.

### 1.6 Antibody Analytics

The purities of the antibodies were determined by using SDS-PAGE and HPLC. The SEC-HPLC was conducted in a device of Agilent 1100 series by using an appropriate size exclusion chromatography column (SEC). The expression titers of the antibodies were measured by using protein A HPLC.

### 1.7 Surface Plasmon Resonance Analysis

The dissociation constants and association constants of the humanized antibodies and the like were analyzed by using surface plasmon resonance (SPR, product name: BiaCore T200 (manufactured by GE healthcare)).

Specifically, first, human IgG capture antibodies were immobilized on a CM5 chip by an amine coupling method. This immobilization was conducted in both reference cells and active cells. Then, the kinetics analysis on the anti-Eva1 antibody and hEva1 was conducted by a single-cycle method using the following measurement conditions.

### Measurement conditions

- Sample compartment temperature: 10°C
- Running buffer: HBS-EP+buffer, pH 7.4
- Capturing molecule: Human IgG antibody, reference & active cells
- Ligand: Various antibodies 5 nM, active cells only
- Analyte: hEva1-mouse Fc1, 5, 25, 125, 250 nM, reference & active cells
- Regeneration buffer: 3M MgCl₂, reference & active cells
- 3 cycles of Blanks (Analyte zero concentration)

In addition, the cycle conditions were as shown in the following Table.

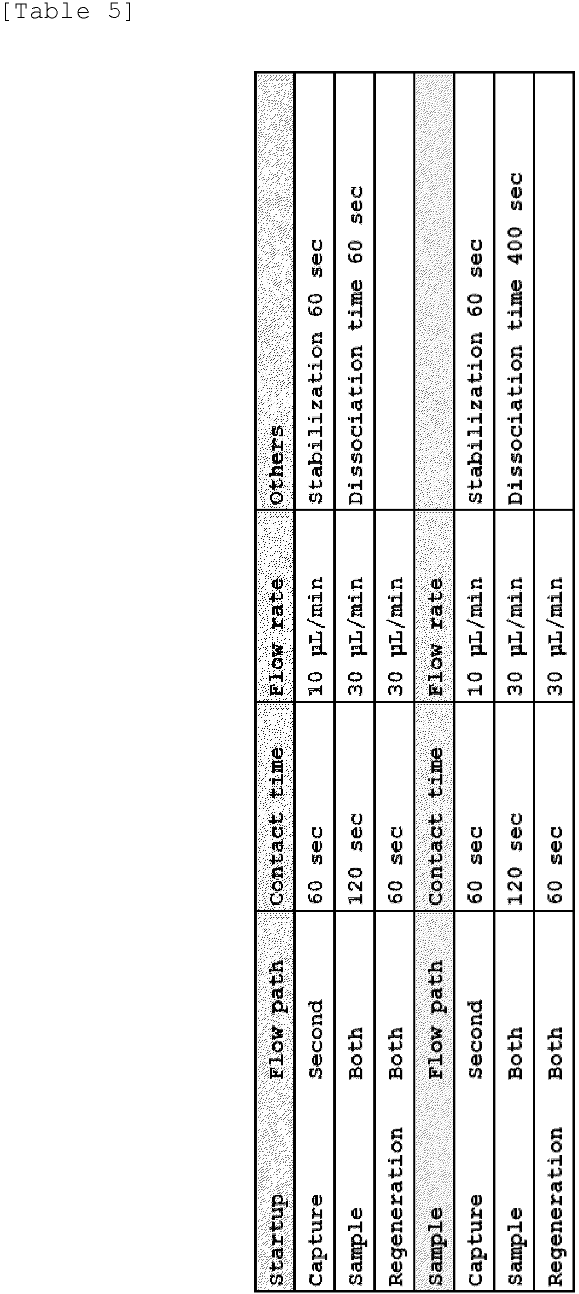

Results obtained by using the above-described materials and methods are shown below.

### (Results)

### 2.1 Sequence Analysis

The sequencing of VH and VL of B2E5-48 mouse antibody was conducted by using an IGMT Gap Align tool and analyzed by comparison with sequences of all known antibody germlines. As a result, although not shown in the figures, these sequences were sequences closest to mice, particularly IGHV1-53 family for VH and IGKV4-68 for VL. In addition, CDR was allocated by using the IMGT definition.

### 2.2 Selection of Germlines

The sequences of VH and VL were analyzed through alignment to the database on human germlines of Absolute Antibody. As a result, germlines selected as frameworks for humanization are shown in Table 6 given below. This Table shows the germlines of the heavy chain and light chain selected as the frameworks for humanization as well as homology (%) of the VH and VL with the original mouse.

**[Table 6]**

| | **Germline ID** | **% identity** |
|---|---|---|
| **Heavy chain** | **IGHV1-46*01** | **67.7** |
| | **IGHV7-4-1*02** | **60.4** |
| **Light chain** | **IGKV3-11*01** | **66.3** |
| | **IGKV1-9*01** | **63.8** |

### 2.4 CDR grafting polymerization

The sequences of the VH and VL were analyzed by using a CDR grafting algorithm and grafted to human germlines in which CDR of B2E5-48 mouse antibody was selected. Although CDR is mainly defined to be involved in binding with antigens, there is a possibility that an amino acid on an outer side of a region called a framework region is directly involved in binding or plays a role of correctly arranging the CDR. In view of this, which amino acids among amino acids of the framework region were left as amino acids derived from the mouse (original) was determined by a structure-guided method. The overview of the sequences generated as a result is shown in Table 7. Note that in this Table, underlined sequence in sequences represents that it is CDR in each variable region.

**[Table 7]**

| **ID** | **Name of sequence Human Germline** | **Sequence** | **SEQ ID NO:** | **Homology with human antibody** |
|---|---|---|---|---|
| | **cAb4053-VH Mouse -** | QVQLQQPGAELVKPGASVKLSCKASGYTFTNYW MHWVKLRPGQGFEWIGEINPTNGGTDYNEKFKR KATLTVDKSSSTAYMQLSSLTSEDSAVYYCTIY TTALDYWGQGTTLTVSS | **27** | **67.70%** |
| **H1** | **cAb4054-VH Humanized IGHV1-46*01** | QVQLVQSGAEVKKPGASVKVSCKASGYTFTNYW MHWVRQAPGQGLEWIGEINPTNGGTDYNEKFQG RVTLTVDKSSSTAYMELSSLRSEDTAVYYCTIY TTALDYWGQGTLVTVSS | **15** | **84.40%** |
| **H2** | **cAb4058-VH Humanized IGHV1-46*01** | QVQLVQSGAEVKKPGASVKVSCKASGYTFTNYW MHWVRQAPGQGLEWMGEINPTNGGTDYNEKFQG RVTMTVDKSTSTAYMELSSLRSEDTAVYYCTIY TTALDYWGQGTLVTVSS | **13** | **87.50%** |
| **H3** | **cAb4062-VH Humanized IGHV7-4-1*02** | QVQLVQSGSELKKPGASVKVSCKASGYTFTNYW MHWVRQAPGQGLEWIGEINPTNGGTDYNEGFTG RFVLSVDKSSSTAYLQISSLKAEDTAVYYCTIY TTALDYWGQGTLVTVSS | **16** | **82.30%** |
| **H4** | **cAb4066-VH Humanized IGHV7-4-1*02** | QVQLVQSGSELKKPGASVKVSCKASGYTFTNYW MHWVRQAPGQGLEWMGEINPTNGGTDYNEGFTG RFVFSVDKSVSTAYLQISSLKAEDTAVYYCTIY TTALDYWGQGTLVTVSS | **14** | **85.40%** |
| | | | | |
| | **cAb4053-VL Mouse -** | QIVLTQSPALMSASPGERVTLTCSASSSVGYMY WYQQKPGSSPKPWIYVTSNLASGVPARFSGSGS GTSYSLTISSMEAEDAATYYCQQWSSNPPTFGA GTKLELK | **21** | **66.30%** |
| **L1** | **cAb4054-VL Humanized IGKV3-11*01** | QIVLTQSPATLSLSPGERATLSCRASSSVGYMY WYQQKPGQAPRPLIYVTSNLASGIPARFSGSGS GTDYTLTISSLEPEDFAVYYCQQWSSNPPTFGQ GTKLEIK | **9** | **84.20%** |
| **L2** | **cA69055-VI. Humanized IGKV3-11*01** | EIVLTQSPATLSLSPGERATLSCRASSSVGYMY WYQQKPGQAPRPLIYVTSNRATGIPARFSGSGS GTDYTLTISSLEPEDFAVYYCQQWSSNPPTFGQ GTKLEIK | **7** | **87.40%** |
| **L3** | **cAb4056-VL Humanized IGKV1-9*01** | QIQLTQSPSFLSASVGDRVTITCRASSSVGYMY WYQQKPGKAPKPLIYVTSNLASGVPSRFSGSGS GTEYTLTISSLQPEDFATYYCQQWSSNPPTFGQ GTKLEIK | **10** | **83.00%** |
| **L4** | **cAb4057-VL Humanized IGKV1-9*01** | DIQLTQSPSFLSASVGDRVTITCRASSSVGYMY WYQQKPGKAPKPLIYVTSNLASGVPSRFSGSGS GTEYTLTISSLQPEDFATYYCQQWSSNPPTFGQ GTKLEIK | **8** | **83.20%** |

### 2.5 Analysis on Sequences Having Risks

In order to confirm that very undesirable risks (problems) on sequences were not introduced into the humanized sequences, the sequence of the original mouse antibody and the humanized sequences were subjected to the Absolute Antibody Sequence Liability Tool.

As a result, glycosylation motif and free cysteine, which are sequences having the highest risks among those shown in Table 4, were not detected in any of the mouse antibody and the humanized antibodies. On the other hand, it was confirmed that deamidation and isomerization motifs were present in these antibodies. However, although having high risks as shown in Table 4, these motifs are normally often observed in antibodies. In addition, although there is a possibility that modifications based on these motifs lead to confusions during production, in many cases, these can be solved depending on a management method. Moreover, it is also possible to remove these motifs by mutagenesis as necessary. In addition, although sequences having medium risks and low risks were detected from the viewpoint of obtaining more complete antibodies, it has hardly been reported that these caused problems in the process of producing antibodies. Therefore, it was determined that the sequences of the mouse antibody and the humanized antibodies did not have particular problems.

### 3. Production and Analysis of Antibodies

As described above, the production of 16 types of humanized antibodies was attempted. As a result, no antibody was detected at all in the supernatants of 6 types (H1-L1, H1-L2, H1-L3, H2-L1, H3-L1, and H4-L1), and other 3 types (H2-L3, H3-L3, and H4-L2) also had significantly low antibody concentrations (Titer (mg/L) described below) in the supernatants. It is considered that normal antibodies were not formed in these and were not sufficiently secreted in the culture supernatants.

**[Table 8]**

| | **Titer (mg/L)** | **ka (M⁻¹s⁻¹)** | **kd (s⁻¹)** | **K_{D} (M)** |
|---|---|---|---|---|
| **Chimeric antibody (CHO)** | **-** | **1.901×10⁴** | **8.447×10⁻³** | **4.449×10⁻⁷** |
| **Chimeric antibody (293T)** | **60** | **1.964×10⁴** | **9.470×10⁻³** | **4.822×10⁻⁷** |
| **H1-L4** | **63** | **1.747×10⁴** | **9.313×10⁻³** | **5.331×10⁻⁷** |
| **H2-L2** | **75** | **1.972×10⁴** | **9.201×10⁻³** | **4.666×10⁻⁷** |
| **H2-L3** | **3** | **2.071×10⁴** | **8.858×10⁻³** | **4.277×10⁻⁷** |
| **H2-L4** | **75** | **1.720×10⁴** | **9.101×10⁻³** | **5.291×10⁻⁷** |
| **H3-L2** | **50** | **1.677×10⁴** | **1.320×10⁻²** | **7.871×10⁻⁷** |
| **H3-L3** | **13** | **1.501×10⁴** | **1.253x10⁻²** | **8.348×10⁻⁷** |
| **H3-L4** | **94** | **1.580×10⁴** | **1.380×10⁻²** | **8.734×10⁻⁷** |
| **H4-L2** | **3** | **1.898×10⁴** | **1.457×10⁻²** | **7.677×10⁻⁷** |
| **H4-L3** | **38** | **1.548×10⁴** | **1.357×10⁻²** | **8.766×10⁻⁷** |
| **H4-L4** | **31** | **1.534×10⁴** | **1.525×10⁻²** | **9.941×10⁻⁷** |

In addition, as a result of analyzing the binding affinity of each antibody by the SPR method, the average dissociation constants (K_{D}) of these were all 10⁻⁷ order as shown the above Table. Particularly, in the humanized antibodies, H2-L2, H1-L4, H2-L3, and H2-L4 were at similar levels to that of the chimeric antibody (produced from HEK293T-cells). In addition, in consideration of the above-described antibody concentrations as well, it was suggested that H2-L2, H2-L4, and H3-L4 were useful in antibody application, which will be described later.

### <Reference Example> Expression of Human Eva1 Protein

### (Analysis on Expression of Eva1 Protein in Normal Human Blood Cells)

In order to analyze the usefulness of the anti-Eva1 antibodies in pharmaceutical agent applications, first for analyzing the usefulness in chimeric antigen receptor (CAR) which will be described later, expression of the Eva1 protein in various blood cells where CAR is expressed was evaluated.

Specifically, the peripheral bloods of healthy subjects were hemolyzed (BD FACS Lysing Solution), followed by staining in CD4, CD8, CD19, neutrophils (gated from the scatter plot), and monocytes (gated from the scatter plot) and at the same time, staining in mouse anti-human Eva1 antibody and secondary antibody, and the resultants were analyzed with flow cytometry.

As a result, as shown in Fig. 1, expression of the Eva1 protein was not observed in all of CD4, CD8 T-lymphocytes, CD19B lymphocytes, neutrophils, and monocytes. Hence, it was suggested that there was a high probability that the blood cells expressing CAR with the Eva1 protein as an antigen do not target these cells.

### (Analysis on Expression of Eva1 protein in Tumors)

Next, the expression of the Eva1 protein in various tumors was evaluated. Specifically, first, various tumor cell lines were peeled from culture flasks and were made into cell suspensions, and then, the Eva1 protein was stained in the same method as described in the above-described (Analysis on Expression of Eva1 Protein in Normal Human Blood Cells).

As a result, as shown in Fig. 2A, although expression was not observed in OVTOKO, PA-1, and T98G, the expression of the Eva1 protein was observed in the other various tumor cell lines.

In addition, expression of the Eva1 protein in cells separated from surgical specimens of the cases of intrahepatic cholangiocarcinoma was also analyzed. Specifically, tumor portions were collected from the surgical specimens of intrahepatic cholangiocarcinoma, and cell suspensions were prepared (MACS Tumor Dissociation Kit, human, Miltenyi Biotec). Moreover, tumor cells were purified by using Tumor cell isolation kit, human, Miltenyi Biotec, the Eva1 protein was stained in the same manner as described above, and the resultants were analyzed with flow cytometry.

As a result, as shown in Fig. 2B, Eva1 was positive in all the three cases of intrahepatic cholangiocarcinoma.

### <Example 2> Chimeric Antigen Receptor Using Mouse Anti-Eva1 Antibody Sequence

### (Preparation of Eva1-CAR Using Anti-human Eva1 Mouse Antibody Sequence)

Next, in order to analyze the usefulness of a chimeric antigen receptor (CAR) targeting the Eva1 protein, such CAR was prepared. Specifically, as shown in Fig. 3A, a single-chain antibody was constructed by using VL and VH of the anti-human Eva1 mouse antibody (the above-described B2E5-48 mouse antibody). Then, VL-linker-VH and VH-linker-VL were prepared, and CAR molecules were constructed by linking a CD28 transmembrane region (CD28TM), a CD28 intracellular domain (CD28IC), and CD3 zeta via a short spacer domain of 15aa (composed of amino acids at positions 268 to 282 set forth in SEQ ID NO: 58 or an amino acid sequence set forth in SEQ ID NO: 59) or a long spacer domain of 232aa (composed of amino acids at positions 268 to 499 set forth in SEQ ID NO: 58 or an amino acid sequence set forth in SEQ ID NO: 63), respectively.

Moreover, EGFR (truncated EGFR, tEGFR) which did not have an intracellular domain was bound behind a T2A sequence. This was transcripted as one mRNA, and after translation, was cleaved with the T2A sequence into CAR and tEGFR molecule, which were distributed in 1:1. tEGFR can be used in selection of a transduction marker and gene-transferred cells.

Specifically, Eva1-CARs (which had the above-described long spacer domain, where the order of the variable regions from the N terminus side was VL, VH) had the configurations as shown in Table 9 given below.

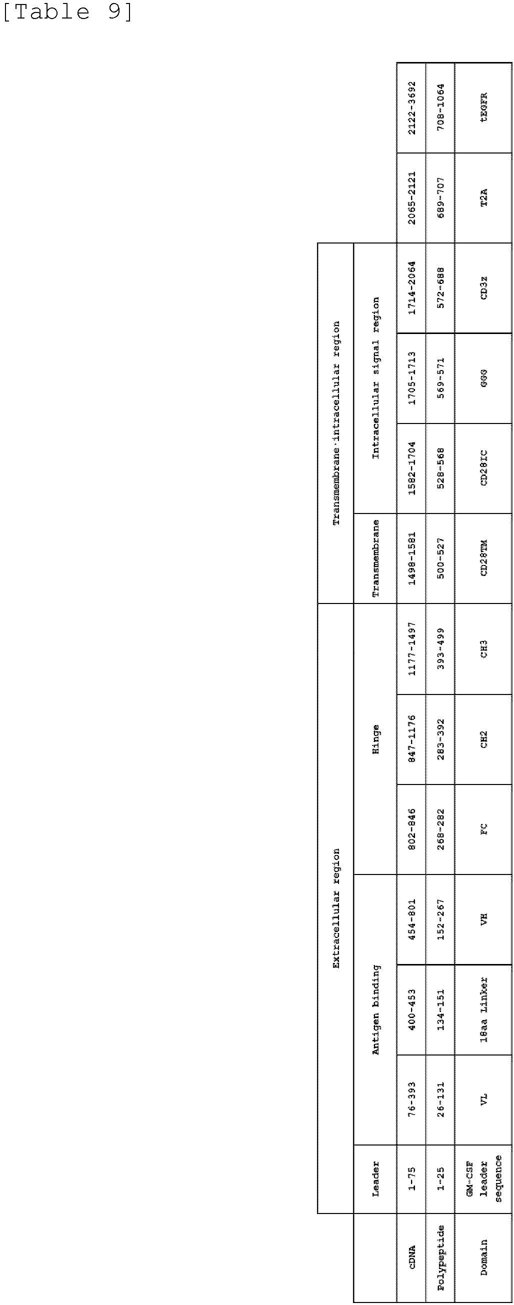

Note that in Table 9, the numbers in the item "c DNA" indicate the positions (bp) in the DNA sequence set forth in SEQ ID NO: 57, and the numbers in the item "Polypeptide" indicate the positions (amino acids) in the sequence set forth in SEQ ID NO: 58.

### (Preparation of Eva1-CAR-T Using Anti-human Eva1 Mouse Antibody Sequence)

Next, as shown in Fig. 3B, the blood was collected from a healthy donor at Day 0, and was purified into CD8 positive cells by using CD8 microbeads (Miltenyi Biotec). Thereafter, T-cells were activated by mixing in a ratio of T cells:beads=1:3 by using CD3/CD28 beads (Invitrogen). At Day 3, transduction was conducted by using a retrovirus which was capable of transducing a sequence encoding the above-described CAR molecule (The construction of the retrovirus was conducted by the same method as that described in Mol Ther 2021 Sep 1; 29 (9): 2677-2690.). Specifically, the retrovirus was loaded on a retronectin coated plate, followed by spinning at 2100 rpm for 120 min. Thereafter, the plate was washed with PBS, and then, stimulated CD8+T-cells were inoculated, followed by spinning at 800 rpm for 2 min. At Day 4, the cells were moved from the retronection coated plate to a normal culture plate. At Day 7, the cells were collected, and a biotinylated EGFR antibody (Erbitax) was added, and thereafter, the tEGFR positive cells were purified by using MACS anti-biotin beads. The purified cells were continuously cultured from Day 7 to Day 10. At Day 10, the cells were counted and stored. A part of the cells was stimulated again at Day 10 by using CD3/CD28 beads, and experiments were conducted at Days 17-20.

Note that the same method was used as the transduction scheme in the following. In addition, 50 U/ml of human·recombinant IL-2 was added at Day 1, 4, 7, 10, 13, 16, (19), and culturing was conducted.

### (Transduction Efficiency of Eval-CAR-T gene Using Anti-human Eva1 Mouse Antibody Sequence)

The transduction efficiencies at Day 7 thus obtained are shown in Figs. 3C and 3D. Fig. 3C shows representative plots (EGFR in the vertical axis shows transduced cells which were obtained by conducting primary staining with biotinylated erbitax and thereafter secondary staining with streptavidin-PE) relating to LH-S (CAR which used VL-VH and had a short spacer), HL-S (CAR which used VH-VL and had a short spacer), LH-L (CAR which used VL-VH and had a short spacer), and HL-L (CAR which used VH-VL and had a long spacer). In addition, Fig. 3D shows results of conducting the same experiments by using cells derived from five healthy donors. As shown in Figs. 3C and 3D, those having long spacers had a high transduction efficiency.

### (Cytokine Production Capacity of Eval-CAR-T Using Anti-human Eva1 Mouse Antibody Sequence)

Next, the cytokine production capacity of the Eval-CAR-T using the anti-human Eva1 mouse antibody sequence was analyzed. Specifically, CAR-T prepared as described above was cultured until day 17, and the cells were co-cultured with various main cell lines in 1:1. After co-culturing for 6 hours, immobilization and intracellular staining (BD cytofix/cytoperm buffer) were conducted to stain the cells with IFN-gamma (IFN-γ). Results thus obtained are shown in Fig. 3E.

As shown in Fig. 3E, IFN-gamma production was not observed in mock T cells in which only EGFR was transduced, but IFN-gamma production was observed in the Eval-CAR-T. In any Eval-CAR-T, IFN-gamma production was not observed for the Eva1 negative cell lines, but IFN-gamma production was observed for the Eva1 positive cell lines, so that the antigen specificity of the Eval-CAR-T was confirmed. More intense IFN-gamma production was observed for those having long spacers than those having short spacers.

### (Optimization of Spacer Length of Eval-CAR-T)

As shown in Fig. 3F, 6 types of mouse antibody-derived Eval-CAR-Ts with spacer regions having different lengths were prepared. The spacer lengths were set to 0aa, 15aa (SEQ ID NO: 59), 30aa (SEQ ID NO: 60), 60aa (SEQ ID NO: 61), 125aa (SEQ ID NO: 62), and 232aa (SEQ ID NO: 63). 15aa was the same as the short version in the previous experiment, and 232aa was the same as the long version in the previous experiment. These were incorporated in plasmids to prepare retroviruses in the same manner, and transduction into CD8 positive cells was conducted in the same manner as the above-described method.

### (transduction efficiencies in Preparation of 6 Types of Eval-CAR-Ts Having Different Spacer Lengths)

The transduction efficiencies (EGFR staining) obtained in this way at Day 7 are shown in Fig. 3G. As a result, it was revealed that three CARs having spacer lengths of 30aa, 125aa, and 232aa had higher transduction efficiencies than the other 3 types (n=5).

### (Amplification Efficiencies of 6 Types of Eval-CAR-Ts Having Different Spacer Lengths (CD3/CD28 Bead Stimulation))

Moreover, CD3/CD28 beads stimulation was subsequently conducted on the above-described Eval-CAR-Ts, and amplification factors obtained when the culturing was continued to Day 17 are shown in Fig. 3H. As a result, there was no difference in amplification efficiency by the CD3/CD28 beads stimulation (n=5).

### (Cytokine Production Capacity of 6 Types of Eval-CAR-Ts Having Different Spacer Lengths)

The experiment was conducted by using the above-described Eval-CAR-Ts at Day 17. Specifically, the Eval-CAR-Ts were co-cultured with tumor cell lines in 1:1, and the cells were immobilized and stained in the same methods as described above after 6 hours, and intracellular IFN-gamma and IL-2 were evaluated with flow cytometry. Results thus obtained are shown in Fig. 3I. The figure shows the percentages of being positive among the CD8 positive cells.

As shown in Fig. 3I, 3 types using spacer lengths of 30aa, 125aa, and 232aa exhibited excellent cytokine productions, and among these, the Eva1 CAR-T-cell having a spacer of 232aa exhibited the most excellent cytokine production capacity.

### (Amplification Efficiencies of 6 Types of Eval-CAR-Ts Having Different Spacer Lengths (Eval Stimulation, without IL-2))

Eval-positive HuCCT-1, was irradiated with a radioactive ray of 100 Gy, and was co-cultured with the CAR-Ts in 1:1, and the amplification efficiencies of these were analyzed. Results thus obtained are shown in Figs. 3J and 3K. In Fig. 3J, amplification efficiencies in time series are shown. In Fig. 3K, the amplification factors at Day 4 were plotted. As shown in Figs. 3J and 3K, using spacer lengths of 15aa, 30aa, and 232aa exhibited higher cell amplifications (n=5, the CAR-Ts were prepared from the peripheral bloods derived from 5 different donors).

### (Amplification Efficiencies of 6 Types of Eval-CAR-Ts Having Different Spacer Lengths (Eval stimulation, with IL-2))

In the same manner as described above, Eval-positive HuCCT-1, was irradiated with a radioactive ray of 100 Gy, and was co-cultured with the CAR-T-cells in 1:1. Then, 50 U/ml of IL-2 was added once every three days, at Day 1, 4, and 7, followed by culturing. Results thus obtained are shown in Figs. 3L and 3M. In Fig. 3L, the respective amplification efficiencies in time series are shown. In Fig. 3M, the amplification factors at Day 18 were plotted. As shown in Figs. 3L and 3M, in the case where spacer lengths of 15aa, 30aa, and 232aa were used, it was observed that the cell amplifications tended to be better (n=5, the CAR-Ts were prepared from the peripheral bloods derived from 5 different donors).

### <Example 3> Chimeric Antigen Receptor Using Anti-human Eva1 Humanized Antibody Sequence

### (Preparation of Humanized Eval-CAR-Ts (hEva1 CAR-Ts) Using Anti-Eval Humanized Antibody Sequences)

Next, 6 types of Eva1 CAR-Ts in total were prepared by using the above-mentioned humanized antibody sequences. Specifically, CARs having a spacer of 232aa were designed for each of the 6 types, L2-linker-H (L2H2), H2-linker-L2 (H2L2), L4-linker-H (L4H2), H2-linker-L4 (H2L4), L4-linker-H3 (L4H3), and H3-linker-L4 (H3L4). Then, DNAs encoding these CARs were incorporated in plasmids to prepare retroviruses and transduction was conducted in the same manner as described above.

### (Transduction Efficiencies in Preparation of hEva1-CAR-Ts Using 6 Types of anti-human Eva1 humanized antibodies)

The transduction efficiencies (EGFR staining) obtained in this way at Day 7 are shown in Fig. 4A. As shown in the figure, it was revealed that in the case where L2H2 and L4H3 were used, higher transduction efficiencies were exhibited than the other 4 types (n=4, the CAR-Ts were prepared from the peripheral bloods derived from 4 different donors).

### (Amplification Efficiencies of hEval-CAR-Ts Using 6 Types of anti-Eval humanized antibodies (CD3/CD28 beads stimulation))

The amplification factors when the culturing of the hEva1 CAR-Ts prepared as described above was subsequently continued to Day 17 under CD3/CD28 beads stimulation are shown in Fig. 4B. As shown in the figure, in the case where L2H2, H2L2, and H2L4 were used, better amplification efficiencies were exhibited than the other 3 types under CD3/CD28 beads stimulation (n=5, the CAR-Ts were prepared from the peripheral bloods derived from 5 different donors).

### (Cytokine Production Capacities of hEval-CAR-Ts Using 6 Types of Anti-Eval Humanized Antibodies)

The experiment was conducted by using hEval-CAR-Ts at Day 17. Specifically, hEval-CAR-Ts were co-cultured with tumor cell lines in 1:1, and the cells were immobilized and stained in the same manner as described above after 6 hours, and intracellular IFN-gamma and IL-2 were evaluated with flow cytometry. Results thus obtained are shown in Figs. 4C and 4D. These figures show the percentages at which the cytokine was positive among the CD8 positive cells. As shown in Figs. 4C and 4D, although there were no significant differences among the hEva1-CAR-T, the CAR-T using L2H2 exhibited the lowest cytokine production against OVTOKO, which is an Eva1 negative cell line.

### (Amplification Efficiencies of hEval-CAR-Ts Using 6 Types of Anti-Eval Humanized Antibodies (Eval Stimulation))

In the same manner as described above, Eval-positive HuCCT-1, was irradiated with a radioactive ray of 100 Gy, and was co-cultured with hEva1 CAR-T-cells in 1:1. Results thus obtained are shown in Fig. 4E. In this figure, the left side shows the results of culturing in the absence of IL-2, and the right side shows the results of culturing while 50 U/ml of IL-2 was added once every three days, at Day 1, 4, and 7. As shown in Fig. 4E, the CAR-T using L2H2 exhibited the most favorable amplification after stimulation in either case (n=5, the CAR-Ts were prepared from the peripheral bloods derived from 5 different donors).

### (Consideration of Intracellular Domains of L2H2 hEval-CAR-Ts)

As described above, regarding the CARs using humanized antibodies, the CAR-T using L2H2 was excellent in antigen specificity and cell growth. In addition, as shown in Example 2, regarding the CARs using mouse-derived antibodies, in the culturing in the absence of IL-2, the CAR-T having a spacer length of 232aa was most excellent in cell growth and cytokine production. On the other hand, in the culturing in the presence of IL-2, the CAR-T having a spacer of 15aa was most excellent in cell growth.

In view of this, next, as shown in Fig. 4F, 6 types of CAR-Ts in which 3 types of intracellular domains (CD28IC, 4-1BBIC, CD79A/CD40IC) were linked via a single-chain variable region (scFv) composed of L2H2 and spacers having two spacer lengths (S: short spacer, L: long spacer) were prepared.

### (Transduction Efficiency in Preparation of 6 Types of hEval-CAR-Ts)

The transduction efficiencies (EGFR staining) at Day 7 in the preparation of the above-described 6 types of CAR-Ts are shown in Fig. 4G. As shown in the figure, in the case of 4-1BB-L, CD79A/CD40-L, the transduction efficiency was high. In addition, in the same manner as described above, it was also reproduced here that the CARs having long spacers (L) had higher transduction efficiencies than those having short spacers (S) (n=5, the CAR-Ts were prepared from the peripheral bloods derived from 5 different donors).

### (Cytokine Production Capacities of 6 types of L2H2 hEval-CAR-Ts)

The experiment was conducted by using the above-described hEval-CAR-Ts at Day 17. Specifically, the hEval-CAR-Ts were co-cultured with tumor cell lines in 1:1, and the cells were immobilized and stained in the same manner as described above after 4 hours, and intracellular IFN-gamma and IL-2 were evaluated with flow cytometry. Results thus obtained are shown in Figs. 4H and 4I. These figures show the percentages at which each cytokine was positive among the CD8 positive cells. As shown in Figs. 4H and 4I, although there were no significant differences among the hEva1-CAR-T, it was also reproduced that the CARs having long spacers had higher cytokine production capacities than those having short spacers (n=5, the CAR-Ts were prepared from the peripheral bloods derived from 5 different donors).

### (Amplification Efficiencies After Stimulation of 6 types of L2H2 Eval-CAR-Ts (Eval stimulation))

In the same manner as described above, Eval-positive HuCCT-1, was irradiated with a radioactive ray of 100 Gy, and was co-cultured with hEval-CAR-Ts in 1:1. Results thus obtained are shown in Fig. 4J. In this figure, the left side shows the results of culturing in the absence of IL-2, and the right side shows the results of culturing while 50 U/ml of IL-2 was added once every three days, at Day 1, 4, and 7. As shown in Fig. 4J, both the short and long 4-1BB and CD79A/CD40 exhibited favorable result to a similar extent in either case (n=5, the CAR-Ts were prepared from the peripheral bloods derived from 5 different donors).

### (Co-culture Test Using 6 Types of L2H2 hEval-CAR-Ts)

L2H2 hEval-CAR-Ts and RPMI-8226 (Myeloma cell line) that was not irradiated with a radioactive ray were co-cultured in E:T ratio=1:16. After the RPMI-8226 was labeled with CellTrace Violet, the culturing was started, so that the RPMI8226 and the CAR-Ts could be distinguished from each other. A part was collected 48 hours, 72 hours, and 96 hours after the start of culturing, and analyzed by using flow cytometry. Results thus obtained are shown in Fig. 4K. This figure shows the percentages for which the tumor cells accounted among all the cells. Note that since the tEGFR is Mock CAR-T-cells in which only tEGFR was transduced, the tumor cells were continuously increasing. No obvious differences were observed among the intracellular domains.

### (Cytotoxicity Crossmatch Using 6 Types of L2H2 hEva1 CAR-Ts (Chromium Release Assay))

The L2H2 hEval-CAR-Ts and various tumor cell lines labeled with ⁵¹Cr were co-cultured for 4 hours, and the percentages of injured cells were examined by measuring the ⁵¹Cr released into the supernatant (chromium release assay). The E:T ratio was set to 10:1, 3:1, and 1:1. Results thus obtained are shown in Figs. 4L and 4M. As shown in Fig. 4L, the significant cytotoxic activity by the hEva1-CAR-T was observed in the cell lines which seemed to be Eva1 positive. Note that no obvious differences were observed among the intracellular domains. In addition, as shown in Fig. 4M, when the Eva1-MFI of each cell line was put on the horizontal axis, correlations with MFI were presented.

As described above, regarding the hEva1-CAR-T in which the antigen recognition site was L2H2, differences in reactivity due to the differences of intracellular domains were evaluated. As a result of checking the cell growth, cytokine release, and cytotoxic activity in vitro, it was found that CARs having intracellular domains of 4-1BB and CD79A/CD40 had excellent cell growth.

### (Evaluation of Antitumor Activities by hEva1-CAR-Ts Using Cancer-bearing models)

The anticancer effects were evaluated by using L2H2 hEva1-CAR-Ts prepared from the peripheral bloods derived from different 2 donors. Specifically, the evaluation was conducted as follows.

7-week old NOG mice (NOD.Cg-PrkdcscidIl2rgtm1Sug/ShiJic) were intraperitoneally inoculated with CFPAC1 cells (human pancreatic cancer cell line) or H1975 (human lung adenocarcinoma cell line) in each of which 1×10⁶ firefly luciferase genes had been introduced. After 2 weeks, by intravenously administering 2×10⁶ tEGFR introduction cells (Mock CAR-T-cells in which only tEGFR was transduced, control) or L2H2 hEva1-CAR-Ts via the caudal vein, the antitumor activities of the CAR-Ts were evaluated. In this evaluation, bioluminescent imaging (BLI) was conducted by using an IVIS spectrum system to evaluate the progress of the tumor. Results thus obtained are shown in Figs. 4N to 4Q. In addition, the body weights were measured in parallel.

In addition, the anticancer effects against cholangiocarcinoma were also evaluated by using L2H2 hEva1-CAR-Ts prepared from the peripheral bloods derived from donors who were different from the above two donors.

Specifically, first, 2×10⁶ firefly luciferase gene-introduced HuCC T-cells (human cholangiocarcinoma cell line) were subctaneously implanted into 7-week old NOG mice. After 2 weeks, after the tumor diameters and the body weights were measured, by intravenously administering 2×10⁶ or 5×10⁶ tEGFR introduction cells (control) or L2H2 hEva1-CAR-Ts via the caudal vein, the antitumor activities of the CAR-Ts were evaluated with BLI as an index in the same manner as described above. The tumor volumes were calculated by measuring the major diameters and the minor diameters of subcutaneous tumors by using a caliper and in accordance with "the major diameter of the tumor×the minor diameter×the minor diameter×1/2". Results thus obtained are shown in Figs. 4R and 4S. In addition, the body weights were measured in parallel.

Note that in the cases of administration into any cancer-bearing models, as the L2H2 hEva1-CAR, those obtained by linking the antigen recognition site (L2H2) and the transmembrane·intracellular domain (domain containing 4-1BB or CD79A/CD40) with the above-described short spacer were used. In addition, the T cells expressing the CAR were prepared by the same method as described above, and the medium was substituted with a cell banker without conducting restimulation using beads at Day 10 in Fig. 3B, and the cells were stored in liquid nitrogen. After the frozen CAR-T was melted and cultured overnight, restimulation was conducted with beads. One week after the beads stimulation, the cells were administered to the cancer-bearing mice as described above.

As is clear from the results shown in Figs. 4N to 4S, the anticancer effect of the L2H2 hEva1-CAR-T was confirmed in all of pancreatic cancer, lung cancer, and cholangiocarcinoma. In addition, although not shown in the figures, there are no abnormal and significant differences in change in body weight of the mice during the administration period, and there were no adverse effects in the administration targets.

### <Example 3> Antibody-drug Conjugate Containing Anti-human Eva1 Humanized Antibody

An antibody-drug conjugate (ADC) was produced by using the above-mentioned humanized B2E5-48 antibody H2-L2 (hereinafter, also referred to as B2E5-H2-L2).

Specifically, first, a vector TPG986 for expressing B2E5-L2 and a vector TPG987 for expressing B2E5-H2 were prepared by using pcDNA 3.4. Each vector was introduced into E. coli JM109, followed by purification using NucleoBond Xtra Midi Kit (MA CH EREY-NAGEL).

Next, B2E5-H2-L2 was transiently expressed in Expi CHO cells by using the vectors and ExpiFectamine CHO Transfection System. Then, the culture supernatant thus obtained was passed through a Protein A column to adsorb the antibody. After eluted with low pH, the antibody was neutralized, and subsequently was purified with Capto adhere. Thereafter, ultrafiltration was conducted. Note that the molecular weight, the sequence, and the binding capacity to Eva1 were checked for the obtained antibody.

Next, as shown in Fig. 5, the B2E5-H2-L2 was reacted with a linker (SMCC) to introduce an MCC group into the antibody. Then, the linker and DM1 were reacted.

Specifically, first, the solvent of the above-prepared B2E5-H2-L2 was buffer-exchanged with PBS6.5/EDTA. Subsequently, a DMSO solution of SMCC (1.84 mg) (0.40 mL; corresponding to about 5.1 equivalent to one molecule of the antibody) was added at room temperature, and the antibody concentration of the reaction solution was adjusted to 20 mg/mL, followed by reaction at room temperature for 2 hours using a tube rotator. This reaction solution was purified, so that an SMCC-derivatized antibody was obtained.

next, to the solution, a DMA (dimethylacetamide) solution of PBS6.5/EDTA (2.56 mL) and N2-Deacetyl-N2-(3-mercapto-1-oxopropyl)-maytansine (4.67 mg; DM1, Journal of Medicinal Chemistry, 2006, 49, 14, 4392) (0.93 mL; corresponding to about 5.8 equivalent to one molecule of the SMCC-derivatized antibody) was added at room temperature to adjust the antibody concentration of the reaction solution to 10 mg/mL, followed by reaction at room temperature for 16.5 hours using a tube rotator and purification, so that a solution containing the target compound was obtained. Subsequently, excess components were removed by purification, and thereafter the resultant was stored at a concentration of 9.0 g/L in a solution of 6% sucrose, 0.02% Tween 20, and 10mM sodium succinate (pH 5.0).

Then, the molecular weight, the sequence, the binding capacity to Eva1, the drug-antibody ratio (DAR), and the like of the ADC were checked. Note that DAR was obtained by measuring absorbance at 252 nm which is the peak of absorption of the DM1 and absorbance at 280 nm which is the peak of absorption of the antibody, and calculating simultaneous equations considering influences of these on the respective absorption peaks to obtain the concentrations of the antibody and the DM1, and determining the ratio in the number of molecules. As a result, the DAR in the above-prepared ADC was 3.8.

### <Example 4> Antitumor Activity of Antibody-drug Conjugate

The antitumor activity of the above-prepared ADC (B2E5-ADC) containing the anti-human Eva1 humanized antibody was evaluated as follows.

### (Cytotoxicity Assay of B2E5-ADC against Glioma)

Human glioma stem cells (hGIC) were inoculated in a 96-well plate in an amount of 1000 cells per well. Next, B2E5-H2-L2, B2E5-ADC, or Kadcyla (trastuzumab emtansine) serially diluted two-fold was added in an amount of 100 µl/well, followed by culturing for 3 days in a CO₂ incubator at 37°C. Then, 5 µl of MTT (5 mg/ml, Wako Pure Chemical) was added to each well, followed by culturing for 2 hours in a CO₂ incubator at 37°C. After the culture liquid was discarded, 90 µl of DMSO was added, and after the cells were dissolved, the absorbance (570 nm) was measured by using a benchmark (Bio-Rad). Results thus obtained are shown in Fig. 6A.

Note that as a positive control (100%), a measured value in a well in the absence of the antibody was used. In addition, as a negative control (0%), a measured value obtained by adding 5 ul of 1% TritonX100 to a well in the absence of the antibody was used.

### (Cytotoxicity Assay of B2E5-ADC against Pancreatic Cancer)

A pancreatic cancer cell line (BxPC3) collected from a culture dish by trypsin treatment was diluted to 5×10⁴ cells/mL, and was inoculated in a 96-well plate in an amount of 100 µL per well, followed by culturing for 24 hours in a CO₂ incubator (37°C, CO₂ concentration 5%). The medium (RPMI-1640, Wako187-02705, 10%FBS) was sucked with an aspirator, and 900 µL of a fresh medium was added to each well. Each of DM1 and B2E5-ADC was diluted with PBS (DM1: 3800 nM to 0.93 nM, B2E5-ADC: 1000 nM to 0.24 nM), and 10 µL of each was added to the BxPC3. After culturing for 48 hours in a CO₂ incubator, LDH released from dead cells was measured by using Cytotoxicity LDH Assay Kit-WST (Dojindo Laboratories). Results thus obtained are shown in Fig. 6B.

### (Cytotoxicity Assay of B2E5-ADC against Stomach Cancer)

To a stomach cancer cell line (MKN74) cultured in a 10-cm cell culture dish, 1 mL of 2.5 g/l-Trypsin/1mmol/l-EDTA Solution, with Phenol Red [Nacalai Tesque 32777-15] was added, and left to stand at 37°C for 5 minutes, and thereafter 3 mL of a medium was added, and a mixture was collected into a 15-mL laboratory centrifuge. Centrifugation was conducted at 1200 rpm for 5 minutes, the medium was sucked with an aspirator, and a new medium was added, followed by resuspension. The number of cells per 1 µL was counted by using Trypan Blue and adjusted to obtain 30000 cells/mL.

The cells were inoculated in a 96-well culture plate in an amount of 100 µL per well (3000 cells/well), followed by culturing for 24 hours in a CO₂ incubator. The medium was sucked with an aspirator, and 90 µL of a fresh medium was added to each well. The B2E5-H2-L2 and the B2E5-ADC were each serially diluted by using PBS (6.5 ug/mL or more), and 10 µL of each was added to each well, followed by culturing for 72 hours in a CO₂ incubator. Then, 10 µL of a cell count reagent [Nacalai Tesque 07553-44] was added to each well, followed by culturing for 3 hours in a CO₂ incubator. Thereafter, the absorbances at 450 nm were measured. Based on results of the measurement, fitting was conducted by using ImageJ to create a graph, and the cytotoxicity of B2E5-ADC against stomach cancer was evaluated. Results thus obtained are shown in Fig. 6C.

### (Toxicity Test on B2E5-ADC Single-Dose Administered Mice)

To 9 to 10-week old C57BL/6 male mice (CLEA Japan) or 10-week old male nude mice (SKN/slc, Japan SLC), single doses of the B2E5-ADC (100, 33, 10 ug) were administered through caudal vein or lumbar puncture (3 individuals were prepared for each administration concentration). The body weights were measured with time. Results thus obtained are shown in Fig. 7.

### (Conclusion of Results of Tests Using B2E5-ADC)

As shown in Fig. 6A, the B2E5-ADC concentration-dependently exhibited cytotoxic activity against hGIC. On the other hand, no cytotoxic activity of the B2E5-H2-L2 and Kadcyla against hGIC was observed. Moreover, as shown in Figs. 6B and 6C, the antitumor activities of the B2E5-ADC were observed not only against the above-describe glioma but also against pancreatic cancer and stomach cancer.

On the other hand, when the toxicity test of the B2E5-ADC on mice was conducted, as shown in Fig. 7, no influence of the B2E5-ADC administration on the mice was observed, and survival for 1 month or more after the administration was confirmed.

### [Industrial Applicability]

As described above, the present invention makes it possible to achieve a high antitumor activity by targeting the human Eva1. Therefore, the present invention is useful as a drug and the like for treating or preventing cancers.

[Sequence Listing] IBPF22-534WO-J(seq)fin.xml

## Claims

1. A humanized antibody or a functional fragment thereof, the humanized antibody comprising:
a light chain variable region comprising complementarity-determining regions 1 to 3 each comprising an amino acid sequence set forth in SEQ ID NO: 1, an amino acid sequence composed of Val-Thr-Ser, and an amino acid sequence set forth in SEQ ID NO: 3; and
a heavy chain variable region comprising complementarity-determining regions 1 to 3 each comprising amino acid sequences set forth in SEQ ID NOs: 4 to 6, wherein the humanized antibody or a functional fragment thereof binds to a human-derived Eva1 protein.

2. The humanized antibody or a functional fragment thereof according to claim 1, wherein
the light chain variable region comprises one amino acid sequence selected from amino acid sequences set forth in SEQ ID NOs: 7 to 10, amino acid sequences having homologies of 80% or more with the amino acid sequences set forth in SEQ ID NOs: 7 to 10, and an amino acid sequence derived from at least one of the amino acid sequences set forth in SEQ ID NOs: 7 to 10 by substitution, deletion, addition, and/or insertion of one or more amino acids, and
the heavy chain variable region comprises one amino acid sequence selected from amino acid sequences set forth in SEQ ID NOs: 13 to 16, amino acid sequences having homologies of 80% or more with the amino acid sequences set forth in SEQ ID NOs: 13 to 16, and an amino acid sequence derived from at least one of the amino acid sequences set forth in SEQ ID NOs: 13 to 16 by substitution, deletion, addition, and/or insertion of one or more amino acids.

3. The humanized antibody or a functional fragment thereof according to claim 1, wherein
the light chain variable region comprises one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
the heavy chain variable region comprises one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids.

4. An antibody-drug conjugate obtained by binding the antibody or a functional fragment thereof according to any one of claims 1 to 3 and a drug.

5. A pharmaceutical composition comprising the antibody-drug conjugate according to claim 4.

6. A chimeric antigen receptor comprising a region binding to a human-derived Eva1 protein, a transmembrane region, and an intracellular signal region.

7. The chimeric antigen receptor according to claim 6, wherein the region binding to a human-derived Eva1 protein is a region comprising the antibody or a functional fragment thereof according to any one of claims 1 to 3.

8. The chimeric antigen receptor according to claim 6, wherein
the region binding to a human-derived Eva1 protein comprises a single-chain variable fragment obtained by binding a light chain variable region, a linker, and a heavy chain variable region in order from an amino terminus side,
the light chain variable region comprises:
complementarity-determining regions 1 to 3 each comprising an amino acid sequence set forth in SEQ ID NO: 1, an amino acid sequence composed of Val-Thr-Ser, and an amino acid sequence set forth in SEQ ID NO: 3; and
one amino acid sequence selected from an amino acid sequence set forth in SEQ ID NO: 7, an amino acid sequence having a homology of 80% or more with the amino acid sequence set forth in SEQ ID NO: 7, and an amino acid sequence derived from at least one of the amino acid sequence set forth in SEQ ID NO: 7 by substitution, deletion, addition, and/or insertion of one or more amino acids, and
the heavy chain variable region comprises:
complementarity-determining regions 1 to 3 each comprising amino acid sequences set forth in SEQ ID NOs: 4 to 6; and
one amino acid sequence selected from an amino acid sequence set forth in SEQ ID NO: 13, an amino acid sequence having a homology of 80% or more with the amino acid sequence set forth in SEQ ID NO: 13, and an amino acid sequence derived from at least one of the amino acid sequence set forth in SEQ ID NO: 13 by substitution, deletion, addition, and/or insertion of one or more amino acids.

9. The chimeric antigen receptor according to any one of claims 6 to 8, wherein the region binding to a human-derived Eva1 protein and the transmembrane region bind to each other via a spacer composed of 10 to 300 amino acids.

10. The chimeric antigen receptor according to any one of claims 6 to 9, wherein the intracellular signal region comprises a CD79A intracellular region and a CD40 intracellular region.

11. A nucleotide encoding the chimeric antigen receptor according to any one of claims 6 to 10.

12. A vector comprising the nucleotide according to claim 11.

13. A cell expressing the chimeric antigen receptor according to any one of claims 6 to 10.

14. The cell according to claim 13, that is an immune cell.

15. A pharmaceutical composition comprising the cell according to claim 13 or 14.
